# EUROPEAN PATENT APPLICATION

(11) **EP 2 263 667 A2**
(43) Date of publication of application: **22.12.2010**
(21) Application number: 10011148.3
(22) Date of filing: 14.06.2006
(51) Int. Cl.: A61K 31/352, A61P 29/02, A61P 1/08

(54) **Compositions comprising crystalline trans-(+/-)-delta-9-tetrahydrocannabinol**

(30) Priority: 16.06.2005 US 691361 P; 12.10.2005 US 726509 P
(62) Divisional of application: 06754384.3
(71) Applicant: EURO-CELTIQUE S.A., 1653 Luxembourg (LU)
(72) Inventor: Kupper, Robert J., East Greenwich RI 02818 (US)
(74) Representative: Maiwald Patentanwalts GmbH

(57) **Abstract**

Pharmaceutical compositions comprising the cannabinoid active pharmaceutical ingredient, crystalline trans-(±)-Δ⁹-tetrahydrocannabinol, and formulations thereof are disclosed. The invention also relates to methods for treating or preventing a condition such as pain comprising administering to a patient in need thereof an effective amount of crystalline trans-(±)-Δ⁹-tetrahydrocannabinol. In specific embodiments, the crystalline trans-(±)-Δ⁹-tetrahydrocannabinol administered according to the methods for treating or preventing a condition such as pain can have a purity of at least about 98% based on the total weight of cannabinoids.

## Description

### 1. Field of the Invention

The present invention is directed to a new cannabinoid active pharmaceutical ingredient comprising crystalline trans-(±)-Δ⁹-tetrahydrocannabinol. The present invention is further directed to pharmaceutical compositions and improved dosage forms that are formed with crystalline trans-(±)-Δ⁹-tetrahydrocannabinol. The present invention is also directed toward methods for treating or preventing a condition such as, *inter alia*, pain, emesis, loss of appetite or weight loss comprising administering a dosage form of the present invention to a patient in need of such treatment or prevention.

### 2. Background of the Invention

In 1997, the National Institutes of Health issued a report assembled by an *ad hoc* group of experts that summarized the available scientific data regarding the therapeutic applications for marijuana ("Workshop on the Medical Utility of Marijuana," http://www.nih.gov/news/medmarijuana/MedicalMarijuana.htm). This report included a recommendation that the NIH should consider supporting research on the potential use of marijuana for the following medical indications: appetite stimulation/cachexia, nausea and vomiting following anticancer therapy, neurological and movement disorders, pain, and glaucoma. In 1999, a second report was published ("Marijuana and Medicine, Assessing the Science Base," Janet E. Joy, Stanley J. Watson, Jr., and John A. Benson, Jr., Editors; Institute of Medicine, 1999, National Academy Press, Washington D.C.
(http://books.nap.edu/catalog/6376.html)), which provided a review of the actual and potential therapeutic uses of cannabinoids. The latter report identified additional conditions for which trans-(-)-Δ⁹-tetrahydrocannabinol could be useful, including somatic pain, chronic pain, neuropathic pain, inflammation, muscle spasticity including that associated with spinal cord injury and multiple sclerosis, movement disorders including dystonia, Parkinson's disease, Huntington's disease, and Tourette's syndrome, migraine headache, epilepsy, and Alzheimer's disease. In addition to identifying such medical applications for marijuana and/or trans-(-)-Δ⁹-tetrahydrocannabinol, that report emphasized the need for rapid-onset, non-smoked, safe, and reliable cannabinoid delivery systems.

Subsequently, there have been numerous reports in the literature that have documented the therapeutic utility of trans-(-)-Δ⁹-tetrahydrocannabinol, which is the active, natural product found in marijuana. U.S. Patent No. 6,713,048 B2 provides a compilation of references, as well as a summary of the data provided in those references concerning the use of Δ⁹-THC for the treatment of AIDS-associated anorexia and cachexia, nausea and emesis due to cancer chemotherapy, pain due to advanced cancer, spasticity related to multiple sclerosis and spinal cord injury, and glaucoma. The term "THC" has been used in the literature to refer to the optically-active, water-insoluble, lipophilic resinous material identified either as Δ⁹-THC or Δ¹-THC, depending on whether the numbering system is based upon that used for pyran or monoterpinioid compounds, respectively (Agurell et al., eds. The Cannabinoids: Chemical, Pharmacologic, and Therapeutic Aspects: New York, Academic Press (1984); Agurell et al., Pharmacol. Rev 38(1):21-43 (1986); Mechoulam ed., Marijuana: Chemistry, Pharmacology, Metabolism and Clinical Effects, New York: Academic Press (1973); Mechoulam, Pharmacol Biochem Behav 40(3):461-464 (1991)).

In particular, (-)-6a,10a-trans-Δ⁹-tetrahydrocannabinol (*i.e*. "trans-(-)-Δ⁹-THC") has been identified as the component primarily responsible for the antiemetic effects associated with cannabis (S.E. Sallen et al., N. Engl. J. Med. 302:135 (1980); A.E. Chang et al., Cancer 47:1746 (1981); and D.S. Poster et al., J. Am. Med. Asso. 245:2047 (1981)). This compound, *i.e*., trans-(-)-Δ⁹-THC, has been reported to be useful as an antiemetic to relieve nausea and vomiting in patients receiving cancer chemotherapy and to stimulate weight gain in patients suffering from symptomatic HIV infection *(see* U.S. Patent No. 6,703,418 B2 to Plasse).

Although both trans-(-)-Δ⁹-THC (*i.e*. the natural product) and its optical isomer trans-(+)-Δ⁹-THC (*i.e*. the trans-(-)- and trans-(+)-enantiomers, respectively, of trans-(±)-Δ⁹-THC), are reported to be useful for treating pain, the trans-(-)-Δ⁹-THC enantiomer has been identified as the more potent of the two enantiomers (*see, e.g.,* G. Jones et al., Biochem. Pharmacol. 23:439 (1974); S.H. Roth, Can. J. Physiol. Pharmacol. 56:968 (1978); B.R. Martin et al., Life Sciences 29:565 (1981); M. Reichman et al., Mol. Pharmacol. 34:823 (1988); and M. Reichman et al., Mol. Pharmacol. 40:547 (1991)). In fact, a more recent publication has asserted that the pharmacological activity ascribed to trans-(+)-Δ⁹-THC in earlier published reports is likely to represent the presence of low levels of trans-(-)-Δ⁹-THC in the materials tested. In fact, when sufficiently purified, trans-(+)-Δ⁹-THC exhibits approximately only 1% of the activity of the trans-(-)-Δ⁹-THC enantiomer (Mechoulam et. al., Pharmacol Biochem Behav 40(3):461-464 (1991)).

Purified trans-(-)-Δ⁹-THC is a thick, viscous, resinous material that has been compared to pine-tree sap and rubber cement. This material is chemically unstable to light, oxygen, and heat. Accordingly, trans-(-)-Δ⁹-THC is extremely difficult to formulate and is not readily adapted for incorporation into standard dosage forms that are typically available for other, solid pharmaceutical compounds.

Synthetic trans-(-)-Δ⁹-THC (*i*.*e*. "dronabinol"), which is currently sold as Marinol^{®} by Unimed Pharmaceuticals, Inc., is available in 2.5, 5, and 10 mg dosage strengths. The trans-(-)-Δ⁹-THC of Marinol^{®} is formulated as a solution in sesame oil, which is distributed into gelatin capsules. This orally-administered form of trans-(-)-Δ⁹-THC is subject to first-pass metabolism in the liver, is absorbed relatively slowly, and exhibits a delayed onset of pharmacological activity of one-half hour to two hours. In contrast, delivery of the active material, trans-(-)-Δ⁹-THC, by inhalation (*e.g*., by smoking) typically results in an onset of pharmacological activity within 10 minutes of administration ("Workshop on the Medical Utility of Marijuana," (http://www.nih.gov/news/medmarijuana/MedicalMarijuana.htm); U.S. Patent No. 6,713,048 B2, *e.g*. Table 2 and the references cited therein). However, delivery of trans-(-)-Δ⁹-THC by smoking is not preferred in view of the inherent dangers of smoking (*e.g*. emphysema and lung cancer), as well as the undefined composition of the plant materials used to prepare marijuana cigarettes (*see e.g.,* "Marijuana and Medicine, Assessing the Science Base," Janet E. Joy, Stanley J. Watson, Jr., and John A. Benson, Jr., Editors; Institute of Medicine, 1999, National Academy Press, Washington D.C.). In view of these issues, the art is fairly replete with attempts to provide improved therapeutically-effective formulations of trans-(-)-Δ⁹-THC.

U.S. Patent No. 6,328,992 describes a transdermal delivery system for cannabinoid compounds in which the active compound is formulated as a mixture comprising a carrier and a permeation enhancer. According to the '992 patent, suitable carriers can include natural rubber, viscoelastic semi-solid materials, hydrogels, thermoplastic polymers, elastomers and thermoplastic elastomers, or an oil selected from the group consisting of mineral oils, vegetable oils, fish oils, animal oils, carbon tetrachloride, ethanolic solutions of resins and pyrahexyl mixtures. Permeation enhancers of the formulations of the '992 patent include nonionic surfactants or solvents, *e.g*. glycerol esters, polyglycerol esters, alkyl fatty acid esters, ethoxylated sorbitan esters, alcohol ethoxylates, lanolin ethoxylates, ethoxylated fatty methyl esters and alkanolamides.

U.S. Patent No. 6,383,513 describes a cannabinoid composition for nasal delivery. The active material of the '513 patent is formulated in a "biphasic delivery system," such as an oil-in-water emulsion. The biphasic delivery system is prepared by combining the drug with an oil and an emulsifier to provide an oil phase, which is then vigorously mixed with an aqueous phase containing a stabilizer. The oil is preferably a vegetable oil such as olive oil, sesame oil, castor oil, cotton-seed oil, or soybean oil, while the emulsifier can be, for example, a polyoxyethylene block copolymer. The '513 patent also describes the formation of "guest-host complexes" comprising a cannabinoid and a solubilizing agent such as cyclodextrin, in which the drug is contained within a cavity in the solubilizing agent. According to the '513 patent, such guest-host complexes can be freeze-dried to provide a powdered material that can be incorporated into the emulsions described, or that can be delivered using an insufflator device.

U.S. Patent No. 6,713,048 describes a Δ⁹-THC solution metered-dose inhaler containing a composition comprising a hydrofluoroalkane propellant and Δ⁹-THC. Suitable propellants include 1,1,1,2-tetrafluoroethane and 1,1,1,2,3,3,3-heptafluoropropane. In addition the formulations of the '048 patent may include an organic solvent, *e.g*. ethanol, to solubilize the Δ⁹-THC. The Δ⁹-THC of the '048 patent is described as the pharmaceutically pure, nonionized resinous drug substance (6aR-trans)-6a,7,8, 10a-tetrahydro-6,6,9-trimethyl-3-pentyl-6H-dibenzo[b,d]-pyran-1-ol.

U.S. Patent No. 6,730,330 B2 describes a pharmaceutical formulation for transmucosal administration, comprising tetrahydrocannabinol, cannabidiol, and a "self-emulsifying" cannabinoid solubilizer such as glycerol monooleate, glycerol monostearate, medium chain triglyceride, polyethoxylated castor oil, polyoxyethylene alkyl ether, polyoxyethylene ether, polyoxyethylene fatty acid ester, polyoxyethylene stearate, or a sorbitan ester.

U.S. Patent No. 6,747,058 describes a composition for inhalation therapy in which Δ⁹-THC is formulated in a semi-aqueous solvent comprising a "judiciously selected" volumetric ratio of alcohol, water and a pharmaceutically acceptable glycol, such as 35:10:55 (v/v) ethanol:water:propylene glycol.

U.S. Published Patent Application No. 2003/0229027 Al describes a formulation in which a cannabinoid compound is incorporated in a sugar glass or a sugar alcohol glass. According to the '027 publication, a natural cannabinoid compound is dissolved in a water-soluble organic solvent while the sugar is dissolved in water. The two solutions are combined to form a mixture, which is then freeze-dried, spray dried, vacuum dried, or dried from a super-critical fluid. Freeze-drying provides a porous cake which, according to the '027 publication, can be processed into a powder that could be used for tableting or for pulmonary administration.

U.S. Published Patent Application No. 2004/0034108 Al describes pharmaceutical formulations comprising a cannabinoid, a solvent, and a co-solvent, which are useful for administration using a pump spray. Useful solvents described in the '108 publication include C₁-C₄ alcohols, including ethanol, which is the preferred solvent. Co-solvents include glycols, *e.g*. propylene glycol, as well as sugar alcohols, carbonate esters and chlorinated hydrocarbons. The '108 publication notes that, in order to obtain the desired particle size appropriate for administration as an aerosol using a pump spray, the viscosity of the formulations disclosed is critical, and accordingly, the working range of solvent:co-solvent is quite narrow.

U.S. Published Patent Application No. 2004/0138293 Al describes a composition comprising a solution or suspension of tetrahydrocannabinol and cannabidiol. Suitable lipophilic solvents or suspension carriers described by the '293 publication include medium- and/or short-chain triglycerides, medium-chain partial glycerides, polyethoxylated fatty alcohols, polyethoxylated fatty acids, polyethoxylated fatty acid triglycerides or partial glycerides, esters of fatty acids with low molecular weight alcohols, partial esters of sorbitan with fatty acids, polyethoxylated partial esters of sorbitan with fatty acids, partial esters of sugars or oligomeric sugars with fatty acids, polyethylene glycols, and mixtures thereof, as well as mixtures of those compounds with fats, oils and/or waxes or glycols or suspensions in mixtures of lecithins and/or oils and/or waxes. In the embodiment described in the '293 application, a mixture of tetrahydrocannabinol and cannabidiol was taken up in a mixture of medium-chain mono- and di-glycerides of C₈-C₁₂ fatty acids and the solution obtained was distributed to soft gelatin capsules.

U.S. Published Patent Application No. 2004/0229939 Al describes a sublingual formulation comprising tetrahydrocannabinol, ethanol and an excipient. According to the '939 publication, those formulations may comprise tetrahydrocannabinol and ethanol, as well as, in certain embodiments, one or more of the following: microcrystalline cellulose, sodium starch glycolate, magnesium stearate, fumed silica, mannitol, sucrose, lactose, sorbitol, lactitol, xylitol, sodium bicarbonate, sodium carbonate, citric acid, tartaric acid, and a water-soluble surfactant. In a particular embodiment, tetrahydrocannabinol was dissolved in ethanol and the resulting solution combined with mannitol to provide a granular mixture. Additional solid excipients were added to the granular mixture, which was then dried to form a powder that could be compressed into tablets.

As indicated in the art, it is difficult to formulate pharmaceutically-acceptable compositions comprising trans-(-)-Δ⁹-THC, in view of the thick, viscous nature of that material and its sensitivity to oxygen, light, and heat. Accordingly, even in those instances noted above, trans-(-)-Δ⁹-THC formulations are generally unstable and frequently exhibit a relatively abbreviated shelf-life and/or must be stored at low temperature (*see e.g.* US2003/0229027 and WO 02/096899).

Accordingly, in view of the therapeutic potential of trans-(-)-Δ⁹-THC and in light of difficulties associated with its formulation, it is apparent that there is a long-felt, unmet need for an improved, stable cannabinoid active pharmaceutical ingredient, which can be used to prepare improved THC dosage forms for administration to patients afflicted with a Condition that can be ameliorated, treated, or prevented with trans-(-)-Δ⁹-THC.

Citation of any reference in Section 2 of this application is not an admission that the reference is prior art to the application.

### 3. Summary of the Invention

The present invention provides a composition comprising crystalline trans-(±)-Δ⁹-tetrahydrocannabinol. In certain embodiments of the composition, the crystalline trans-(±)-Δ⁹-tetrahydrocannabinol comprises trans-(-)-Δ⁹-tetrahydrocannabinol and trans-(+)-Δ⁹-tetrahydrocannabinol. In other embodiments of the composition, the crystalline trans-(±)-Δ⁹-tetrahydrocannabinol consists essentially of trans-(-)-Δ⁹-tetrahydrocannabinol and trans-(+)-Δ⁹-tetrahydrocannabinol. In a particular embodiment, the crystalline trans-(±)-A⁹-tetrahydrocannabinol comprises at least 95% by weight of trans-(±)-Δ⁹-tetrahydrocannabinol based on a total amount of cannabinoids in the composition. In other aspects, the crystalline trans-(±)-Δ⁹-tetrahydrocannabinol comprises at least 98% by weight, at least 99%, at least 99.5% or at least 99.9 % by weight, of trans-(±)-Δ⁹-tetrahydrocannabinol based on the total amount of cannabinoids in the composition.

In further embodiments of the composition, the crystalline trans-(±)-Δ⁹-tetrahydrocannabinol has a molar ratio of trans-(-)-Δ⁹-tetrahydrocannabinol to trans-(+)-Δ⁹-tetrahydrocannabinol within a range of from about 0.8:1.2 to about 1.2:0.8, or from about 0.9:1.1 to about 1.1:0.9, or from about 0.95:1.05 to about 1.05:0.95. In a specific embodiment, the crystalline trans-(±)-Δ⁹-tetrahydrocannabinol has a molar ratio of trans-(-)-Δ⁹-tetrahydrocannabinol to trans-(+)-Δ⁹-tetrahydrocannabinol of about 1:1.

The present invention further provides a pharmaceutical composition comprising crystalline trans-(±)-Δ⁹-tetrahydrocannabinol and a pharmaceutically-acceptable carrier or excipient. In a preferred embodiment, the crystalline trans-(±)-Δ⁹-tetrahydrocannabinol is present in the pharmaceutical composition in a therapeutically effective amount.

The present invention further provides a dosage form comprising a pharmaceutical composition of the present invention formulated into a dosage form useful for administration to a mammal, and particularly a human patient. The dosage form may be adapted for oral administration, transmucosal administration, transdermal administration, intrathecal administration, parenteral administration or administration by inhalation. In a particular aspect of this embodiment, the dosage form is a unit dosage form. In certain embodiments, the dosage form of the present invention comprises an amount of crystalline trans-(±)-Δ⁹-tetrahydrocannabinol within a range of from about 0.05 mg to about 200 mg, or from about 0.1 mg to about 100 mg; or from about 0.5 mg to about 75 mg; or from about 2 mg to about 50 mg; or from about 5 mg to about 25 mg. In specific embodiments, the dosage form of the present invention comprises about 5 mg, about 10 mg, about 20 mg, about 40 mg, about 50 mg, about 60 mg, about 80 mg, about 100 mg or about 200 mg of crystalline trans-(±)-Δ⁹-tetrahydrocannabinol.

The aforementioned compositions and dosage forms are formulated with crystalline trans-(±)-Δ⁹-tetrahydrocannabinol and may, or may not, comprise crystalline trans-(±)-Δ⁹-tetrahydrocannabinol at the time of administration.

The present invention is also directed toward a method for administering trans-(±)-Δ⁹-tetrahydrocannabinol to a patient in need thereof, which comprises admixing an effective amount of crystalline trans-(±)-Δ⁹-tetrahydrocannabinol and a pharmaceutically-acceptable carrier to provide a composition, and administering the composition to the patient. In one aspect of this embodiment, the composition is in the form of a solution, emulsion, gel, or suspension. In another aspect of this embodiment, the pharmaceutically-acceptable carrier is a solvent and the composition is a solution. In a further aspect of this embodiment, the admixing and the administering are carried out by the patient, and, in certain, embodiments, the administering is carried out immediately after admixing the crystalline trans-(±)-Δ⁹-tetrahydrocannabinol and a pharmaceutically-acceptable carrier to provide the composition.

In certain embodiments, the compositions and dosage forms of the present invention comprise or are formulated with crystalline trans-(±)-Δ⁹-tetrahydrocannabinol prepared by a process comprising allowing trans-(-)-Δ⁹-tetrahydrocannabinol and trans-(+)-Δ⁹-tetrahydrocannabinol to crystallize together. In a non-limiting embodiment, the process comprises allowing trans-(-)-Δ⁹-tetrahydrocannabinol and trans-(+)-Δ⁹-tetrahydrocannabinol to crystallize together from a first composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol. The first composition may be obtained by any process known in the art. For example, the first composition can be obtained by: (a) forming a biphasic composition comprising (i) a first organic phase, and (ii) an alcoholic-caustic phase containing the trans-(-)-Δ⁹-tetrahydrocannabinol and the trans-(+)-Δ⁹-tetrahydrocannabinol; (b) separating the trans-(-)-Δ⁹-tetrahydrocannabinol and the trans-(+)-Δ⁹-tetrahydrocannabinol from the alcoholic-caustic phase; and (c) contacting the trans-(-)-Δ⁹-tetrahydrocannabinol and the trans-(+)-Δ⁹-tetrahydrocannabinol from step (b), with a non-polar organic solvent to form the first composition.

Alternatively, the first composition can be obtained by: (a) forming a biphasic composition comprising (i) a first organic phase, and (ii) an alcoholic-caustic phase containing trans-(-)-Δ⁹-tetrahydrocannabinol; (b) separating the trans-(-)-Δ⁹-tetrahydrocannabinol from the alcoholic-caustic phase; and (c) contacting the trans-(-)-Δ⁹-tetrahydrocannabinol from step (b) with trans-(+)-Δ⁹-tetrahydrocannabinol and a non-polar organic solvent to form the first composition.

Alternatively, the first composition can be obtained by: (a) forming a biphasic composition comprising (i) a first organic phase, and (ii) an alcoholic-caustic phase containing trans-(+)-Δ⁹-tetrahydrocannabinol; (b) separating the trans-(+)-Δ⁹-tetrahydrocannabinol from the alcoholic-caustic phase; and (c) contacting the trans-(+)-Δ⁹-tetrahydrocannabinol from step (b) with trans-(-)-Δ⁹-tetrahydrocannabinol and a non-polar organic solvent to form the first composition.

In other embodiments, the compositions and dosage forms of the present invention comprise or are formulated with crystalline trans-(+)-Δ⁹-tetrahydrocannabinol, which crystalline trans-(±)-Δ⁹-tetrahydrocannabinol has been prepared by a process comprising allowing trans-(-)-Δ⁹-tetrahydrocannabinol and trans-(+)-Δ⁹-tetrahydrocannabinol to crystallize together from a first organic composition comprising trans-(-)-Δ⁹-tetrahydrocannabinol, trans-(+)-Δ⁹-tetrahydrocannabinol, and a non-polar organic solvent, to provide crystalline trans-(±)-Δ⁹-tetrahydrocannabinol. The first organic composition may be obtained by any process known in the art. For example, in one embodiment, the first organic composition can be obtained by: (a) forming a first biphasic composition comprising (i) a first organic phase comprising a first water-immiscible organic solvent, and (ii) an alcoholic-caustic phase containing trans-(-)-Δ⁹-tetrahydrocannabinol and trans-(+)-Δ⁹-tetrahydrocannabinol, (b) separating the alcoholic-caustic phase and contacting the separated alcoholic-caustic phase with acid to provide an acid-treated alcoholic phase, (c) contacting the acid-treated alcoholic phase with a second water-immiscible organic solvent to form a second biphasic composition comprising (i) a second organic phase comprising Δ⁹-THC, and (ii) an acid-treated alcoholic phase, (d) contacting the separated second organic phase from step (c) with a non-polar organic solvent to form the first organic composition comprising Δ⁹-THC.

Alternatively, crystalline trans-(±)-Δ⁹-tetrahydrocannabinol can be prepared by a process comprising allowing trans-(-)-Δ⁹-tetrahydrocannabinol and trans-(+)-Δ⁹-tetrahydrocannabinol to crystallize together from a second organic composition comprising trans-(-)-Δ⁹-tetrahydrocannabinol, trans-(+)-Δ⁹-tetrahydrocannabinol, and a non-polar organic solvent, to provide crystalline trans-(±)-Δ⁹-tetrahydrocannabinol. The second organic composition can be obtained by: (a) forming a first biphasic composition comprising (i) a first organic phase comprising a first water-immiscible organic solvent, and (ii) an alcoholic-caustic phase containing trans-(-)-Δ⁹-tetrahydrocannabinol; (b) contacting the separated alcoholic-caustic phase with acid to provide an acid-treated alcoholic phase, (c) contacting the acid-treated alcoholic phase with a second water-immiscible organic solvent to form a second biphasic composition comprising (i) a second organic phase comprising trans-(-)-Δ⁹-tetrahydrocannabinol, and (ii) an acid-treated alcoholic phase, (d) adding trans-(+)-Δ⁹-tetrahydorcannabinol to the separated second organic phase of step (c) preferably in an amount from about 0.75 to about 1.25 molar equivalents per molar equivalent of trans-(-)-Δ⁹-THC, and (e) contacting the second organic phase of step (d) with a non-polar organic solvent to form the second organic composition comprising Δ⁹-THC.

Alternatively, the second organic composition can be obtained by: (a) forming a first biphasic composition comprising (i) a first organic phase comprising a first water-immiscible organic solvent, and (ii) an alcoholic-caustic phase containing trans-(+)-Δ⁹-tetrahydrocannabinol; (b) contacting the separated alcoholic-caustic phase with acid to provide an acid-treated alcoholic phase, (c) contacting the acid-treated alcoholic phase with a second water-immiscible organic solvent to form a second biphasic composition comprising (i) a second organic phase comprising trans-(+)-Δ⁹-tetrahydrocannabinol, and (ii) an acid-treated alcoholic phase, (d) adding trans-(-)-Δ⁹-THC to the separated second organic phase of step (c) preferably in an amount from about 0.75 to about 1.25 molar equivalents per molar equivalent of trans-(+)-Δ⁹-THC, and (e) contacting the second organic phase of step (d) with a non-polar organic solvent to form the second organic composition comprising Δ⁹-THC.

The trans-(±)-Δ⁹-tetrahydrocannabinol prepared as described herein may be re-crystallized one or more times according to the methods disclosed, to provide crystalline trans-(±)-Δ⁹-tetrahydrocannabinol of a desired purity of at least 95%, at least 98%, at least 99%, at least 99.5% or at least 99.9% by weight.

The present invention further provides a method for treating a Condition comprising administering to a mammal in need of such treatment and effective amount of a pharmaceutical composition of the present invention. In various aspects of this embodiment, the Condition is selected from the group consisting of pain, emesis, loss of appetite, and weight loss. In other aspects of this embodiment, the Condition is selected from the group consisting of cachexia, nausea and vomiting (such as that following anticancer therapy), glaucoma, neuralgia, somatic pain, chronic pain, neuropathic pain, inflammation, neurological disorders, muscle spasticity (such as that associated with spinal cord injury and multiple sclerosis), a movement disorder (such as dystonia, Parkinson's disease, Huntington's disease, and Tourette's syndrome), migraine headache, epilepsy, and Alzheimer's disease. In another embodiment, the Condition is atherosclerosis. In a further embodiment, the Condition is neurological trauma or stroke.

In another embodiment, the present invention is directed toward a method for preparing a cannabinoid composition comprising the active pharmaceutical ingredient (API) of the present invention, the method comprising admixing crystalline trans-(±)-Δ⁹-tetrahydrocannabinol with a pharmaceutically-acceptable carrier or excipient. In certain aspects of this embodiment, the crystalline trans-(±)-Δ⁹-tetrahydrocannabinol comprises at least 95%, or at least 98%, or at least 99%, or at least 99.5% by weight oftrans-(±)-Δ⁹-tetrahydrocannabinol based on the total amount of cannabinoids in the composition. In certain embodiments, the composition is formulated as a dosage form, or preferably as a unit dosage form.

In certain aspects of this embodiment, the pharmaceutically-acceptable carrier or excipient is a powder or other solid material. In another specific non-limiting embodiment, the dosage form is in a powder or other dry form. In a further aspect of this embodiment, the pharmaceutically-acceptable excipient is chosen to provide a cannabinoid dosage form that is a suspension, emulsion, gel, or solution. In a specific non-limiting aspect of this embodiment the dosage form is an emulsion, gel, or solution that is prepared at the time the dosage form is to be administered to the patient.

The present invention further provides compositions and methods adapted for pulmonary administration of trans-(±)-Δ⁹-tetrahydrocannabinol to a mammal. The method comprises depositing a stable cannabinoid composition comprising a therapeutically-effective amount of crystalline trans-(±)-Δ⁹-tetrahydrocannabinol into the lungs of a mammal in need thereof. In one embodiment, the cannabinoid composition is deposited into the lungs of the mammal by inhalation. In one aspect of this embodiment, the crystalline trans-(±)-Δ⁹-tetrahydrocannabinol, optionally admixed with a pharmaceutically-acceptable excipient, is in a form selected from the group consisting of powders, granules, microparticles, nanoparticles, and mixtures thereof. In certain aspects of this embodiment, the pharmaceutically-acceptable excipient is also in a form selected from the group consisting of powders, granules, microparticles, nanoparticles, and mixtures thereof. In other aspects of this embodiment, the crystalline trans-(±)-Δ⁹-tetrahydrocannabinol comprises at least 95%, at least 98%, at least 99%, or at least 99.5% by weight of trans-(±)-Δ⁹-tetrahydrocannabinol based on the total amount of cannabinoids in the composition. In a further aspect of this embodiment, the crystalline trans-(±)-Δ⁹-tetrahydrocannabinol is delivered to the lungs of the mammal using a mechanical device suitable for pulmonary administration and capable of depositing the crystalline trans-(±)-Δ⁹-tetrahydrocannabinol into the lungs of the mammal. The mechanical device can be, for example, selected from the group consisting of a powder inhaler, a unit dose inhaler, a metered-dose inhaler, a nebulizer, and a pump spray.

The present invention further provides methods and compositions adapted for oral administration of crystalline trans-(±)-Δ⁹-tetrahydrocannabinol to a mammal.

The oral dosage form of the present invention can be adapted for immediate release using standard pharmaceutical formulation technology. Alternatively, the oral dosage form can be adapted for controlled release. In certain embodiments, the controlled-release formulation comprises a therapeutically-effective amount of crystalline trans-(±)-Δ⁹-tetrahydrocannabinol and a controlled-release material. The controlled-release material can be selected from the group consisting of hydrophobic polymers, hydrophilic polymers, gums, protein-derived materials, waxes, shellacs, and the like as well as mixtures thereof. In certain embodiments, the controlled-release formulation provides sustained release and is suitable, *e.g*., for 8-hour, 12-hour or 24-hour dosing in a human patient. In certain embodiments, the oral, controlled-release dosage form, after administration to a human patient, can provide a C₂₄/C*ₘₐₓ* ratio of from about 0.55 to about 0.85, and a therapeutic effect for at least about 24 hours. In a specific aspect of this embodiment, the C*ₘₐₓ* is a sub-psychotropic-threshold concentration.

In a particular embodiment, the oral, controlled-release dosage form suitable for 24 hour dosing in a human patient comprises a pharmaceutically-acceptable matrix comprising a therapeutically-effective amount of crystalline trans-(±)-Δ⁹-tetrahydrocannabinol and a controlled-release material in which the matrix comprises a plurality of multiparticulate matrices. In various aspects of this embodiment, the multiparticulate matrices are compressed into a tablet, or are disposed in a pharmaceutically-acceptable capsule, or are disposed within a pharmaceutically-acceptable suspension, emulsion, gel, or solution.

The present invention further provides a process for preparing a solid, orally available, controlled-release dosage form, said process comprising the step of incorporating a therapeutically-effective amount of crystalline trans-(±)-Δ⁹-tetrahydrocannabinol into an appropriate controlled-release material. Such a controlled-release material may be selected from the group consisting of hydrophobic polymers, hydrophilic polymers, gums, protein-derived materials, waxes, shellacs, and the like, as well as mixtures thereof, forming a controlled-release matrix formulation. In a particular embodiment, said dosage form after oral administration to a human patient, provides a C₂₄/C*ₘₐₓ* ratio of from about 0.55 to about 0.85, and a therapeutic effect for at least about 24 hours.

The present invention further provides methods and compositions adapted for transmucosal or transdermal administration of crystalline trans-(±)-Δ⁹-tetrahydrocannabinol to a mammal.

The present invention can be understood more fully by reference to the following detailed description and illustrative examples, which exemplify non-limiting embodiments of the invention.

### 4. Brief Description of the Drawings

FIG. 1 is a graphical illustration of the powder X-ray diffraction pattern obtained upon analysis of a sample of crystalline trans-(±)-Δ⁹-THC prepared according to the methods of the invention.
FIG. 2 is a graphical illustration of the HPLC chromatogram obtained upon analysis of a sample of crystalline trans-(±)-Δ⁹-THC prepared according to the methods of the invention.
FIG. 3 is a graphical illustration of the data obtained from a differential scanning calorimetry analysis of crystalline trans-(±)-Δ⁹-THC prepared according to the methods of the invention.
FIG. 4 is a graphical illustration of the data obtained from a thermal gravimetric analysis of a sample of crystalline trans-(±)-Δ⁹-THC prepared according to the methods of the invention.
FIG. 5A-5B depict the Fourier Transform Infrared spectrum obtained upon analysis of a sample of crystalline trans-(±)-Δ⁹-THC prepared according to the methods of the invention. FIG 5A depicts the spectrum between wavenumbers 500 cm⁻¹ and 4000 cm⁻¹ while FIG 5B depicts the spectrum between wavenumbers 600 cm⁻¹ and 1700 cm⁻¹.
FIG. 6A - 6D depict the ¹H NMR spectrum obtained upon analysis of a sample of crystalline trans-(±)-Δ⁹-THC prepared according to the methods of the invention. FIG 6A depicts the ¹H NMR spectrum between 0 and 10 ppm; FIG 6B depicts the ¹H NMR spectrum between 4.6 and 6.4 ppm; FIG 6C depicts the ¹H NMR spectrum between 1.8 and 3.3 ppm; and FIG 6D depicts the ¹H NMR spectrum between 0.8 and 3.3 ppm.
FIG. 7A - 7D depict the ¹³C NMR spectrum obtained upon analysis of a sample of crystalline trans-(±)-Δ⁹-THC prepared according to the methods of the invention FIG 6A depicts the ¹³C NMR spectrum between 0 and 180 ppm; FIG 6B depicts the ¹³C NMR spectrum between 105 and 155 ppm; FIG 6C depicts the ¹³C NMR spectrum between 10 and 50 ppm; and FIG 6D depicts the ¹³C NMR spectrum between 72 and 82 ppm.

### 5. Detailed Description of the Invention

The active cannabinoid pharmaceutical ingredient disclosed herein comprises highly-purified crystalline trans-(±)-Δ⁹-THC. Compared to the pure enantiomer (*i.e*., trans-(-)-Δ⁹-THC), the crystalline trans-(±)-Δ⁹-THC of the present invention is less sensitive to oxygen, light, and heat. Consequently, the compositions and dosage forms of the present invention exhibit substantially-improved stability over known compositions and dosage forms that comprise the purified enantiomer, trans-(-)-Δ⁹-THC. For example, samples of purified, crystalline trans-(±)-Δ⁹-tetrahydrocannabinol according to the present invention held for three days at room temperature in the presence of air and laboratory lighting remained white. Moreover, since the crystalline trans-(±)-Δ⁹-tetrahydrocannabinol is a crystalline solid material, it is amenable to formulation according to methods disclosed in the art in view of this disclosure.

Accordingly, the active cannabinoid pharmaceutical ingredient disclosed herein is readily adaptable to the preparation of improved dosage forms such as those providing immediate release of the active agent, as well as those providing controlled release of the active agent. Moreover, the improved dosage forms of the present invention comprising crystalline trans-(±)-Δ⁹-THC may reduce the patient-to-patient variability in physiological and/or psychotropic responses upon administration of trans-(-)-Δ⁹-THC as reported in the art.

Preparation of crystalline trans-(±)-Δ⁹-THC for use as an active pharmaceutical ingredient (or "API") in the improved dosage form of the present invention can be accomplished, *inter alia*, according to the methods disclosed herein as well as in commonly-owned U.S. provisional application Serial No. 60/630,556, which is hereby incorporated by reference in its entirety. For example, the API of the present invention can be isolated by crystallizing trans-(±)-Δ⁹-THC from a composition comprising trans-(-)-Δ⁹-THC, trans-(+)-Δ⁹-THC, and a non-polar organic solvent according to the methods disclosed herein, as well as those disclosed in U.S. provisional application Serial No. 60/630,556.

Without being limited by theory, the present inventor believes that cannabinoid impurities typically present in Δ⁹-THC compositions are substantially, if not completely, removed when trans-(-)-Δ⁹-THC and trans-(+)-Δ⁹-THC are allowed to form crystalline trans-(±)-Δ⁹-THC. Accordingly, in one embodiment, crystalline trans-(±)-Δ⁹-THC of the present invention comprises at least 95%, at least 98%, at least 99%, at least 99.5%, or at least 99.9% by weight of crystalline trans-(±)-Δ⁹-THC based on the total amount of cannabinoids.

In one approach for the preparation of crystalline trans-(±)-Δ⁹-THC used in the improved dosage forms of the present invention, trans-(-)-Δ⁹-THC and trans-(+)-Δ⁹-THC are prepared separately and then combined together in the presence of a non-polar solvent to provide a composition from which crystalline trans-(±)-Δ⁹-THC can be isolated. Trans-(-)-Δ⁹-THC is a natural product that can be isolated from the plant *Cannabis sativa* according to methods described in the art. In another approach, each of the enantiomers, *i.e*. trans-(-)-Δ⁹-THC and trans-(+)-Δ⁹-THC, is separately prepared by chemical synthesis either according to methods described herein, according to the methods disclosed in U.S. provisional application Serial No. 60/630,556, or according to methods disclosed in the art. Alternatively, preparations of both trans-(-)-Δ⁹-THC and trans-(+)-Δ⁹-THC can be obtained by fractionation of a mixture comprising both enantiomers such as, *e.g*. by the method disclosed in U.S. provisional application Serial No. 60/630,556, or according to methods disclosed below. The enantiomers can then be combined together in the presence of a non-polar solvent to provide a composition from which crystalline trans-(±)-Δ⁹-THC can be isolated.

In yet another approach, trans-(±)-Δ⁹-THC is synthesized as a mixture comprising both trans-(-)-Δ⁹-THC and trans-(+)-Δ⁹-THC, according to methods described herein, or according to the methods disclosed in U.S. provisional application Serial No. 60/630,556, or according to methods disclosed in the art. The mixture comprising both trans-(-)-Δ⁹-THC and trans-(+)-Δ⁹-THC is then contacted with a non-polar solvent to provide a composition from which crystalline trans-(±)-Δ⁹-THC can be isolated.

Crystalline trans-(±)-Δ⁹-THC, which is prepared as disclosed herein or as disclosed in U.S. provisional application Serial No. 60/630,556, is a solid crystalline material that is at least 95% by weight, at least 98% by weight, at least 99% by weight, at least 99.5% or at least 99.9% by weight of trans-(±)-Δ⁹-THC based on the total amount of cannabinoids. This solid material can readily be granulated and micronized to provide particulate materials and powders for use, *e.g*., in metered-dose inhalers, or for transdermal, transmucosal, parenteral or oral administration. In particular, formulations for oral administration, *e.g*. in the form of tablets, pills, or encapsulated particles or suspensions, can be manufactured as immediate-release or controlled-release (*e.g*. sustained-release) formulations.

The oral formulations of the present invention may further comprise one or more adverse agents that can be adapted for release upon tampering of the dosage form so as to, *e.g*. diminish or obviate the pharmacological activity of trans-(-)-Δ⁹-THC and/or trans-(+)-Δ⁹-THC, in order to discourage administration of the formulation by a route other than oral administration. Illustrative examples of such agents include, but are not limited to, the CBl antagonist, SR 141716 A (*see e.g.* Shire et al. (1996) J. Biol. Chem. 271(12): 6941-46) and the CB2 antagonist SR 144528 (*see e.g.* Shire et al. (1998) J. Pharmacol. Exp. Ther. 284(2): 644-50).

### 5.1. Definitions

As used herein, the generic term "Δ⁹-THC" may refer to trans-(-)-Δ⁹-THC; trans-(+)-Δ⁹-THC; trans-(±)-Δ⁹-THC; or any mixture thereof.
Trans-(-)-Δ⁹-THC has the structure of formula **(1a):** 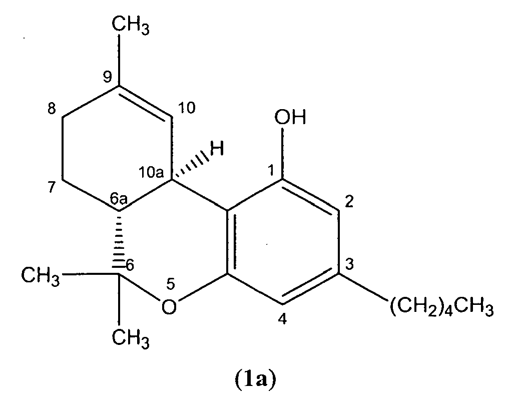
Trans-(+)-Δ⁹-THC has the structure of **(1b):**

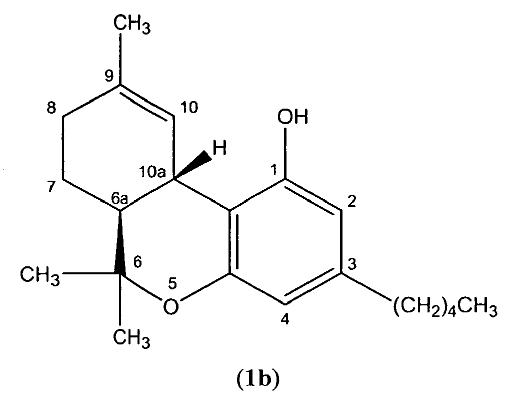

As used herein, the generic term "Δ⁸-THC" may refer to (-)-Δ⁸-THC; (+)-Δ⁸-THC; trans-(±)-Δ⁹-THC; or any mixture thereof.
(-)-Δ⁸-THC has the structure of formula **(2a):** 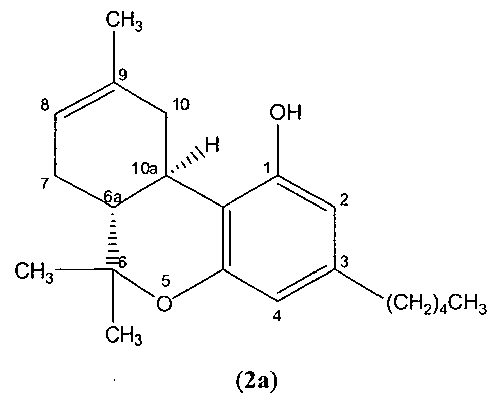
(+)-Δ⁸-THC has the structure of **(2b):**

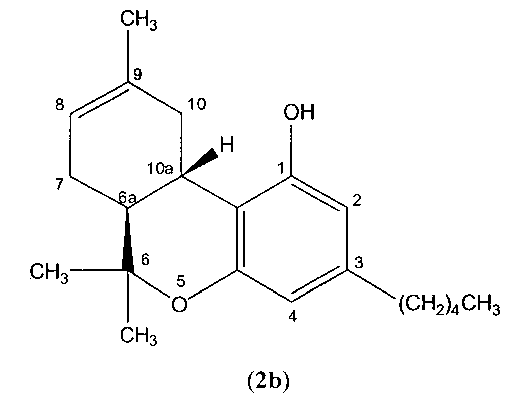

As used herein, the generic term "CBD" may refer to (-)-CBD; (+)-CBD; (±)-CBD; or any mixture thereof.
(-)-CBD has the structure of formula **(3a):** 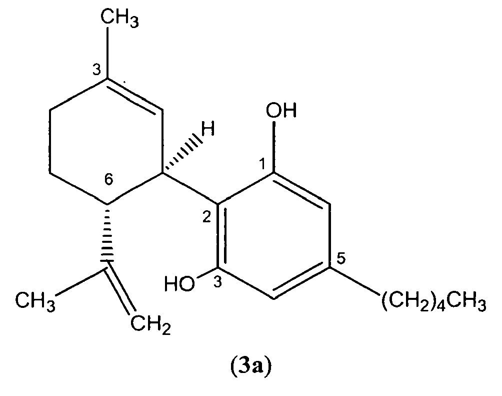
(+)-CBD has the structure of formula **(3b):**

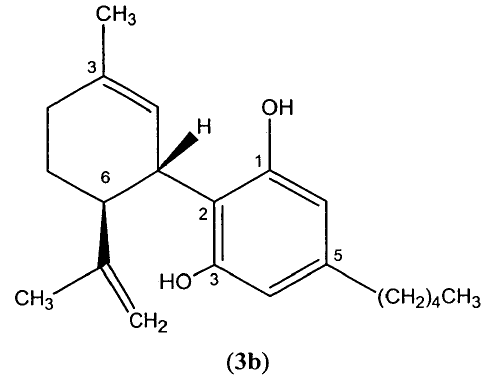

As used herein, the generic term "CBD-bis-1,3-(3,5-dinitrobenzoate)" may refer to (-)-CBD-bis(3,5-dinitrobenzoate); (+)-CBD-bis(3,5-dinitrobenzoate); (±)-CBD-bis(3,5-dinitrobenzoate); or any mixture thereof.
(-)-CBD-bis(3,5-dinitrobenzoate) has the structure of formula **(4a):** 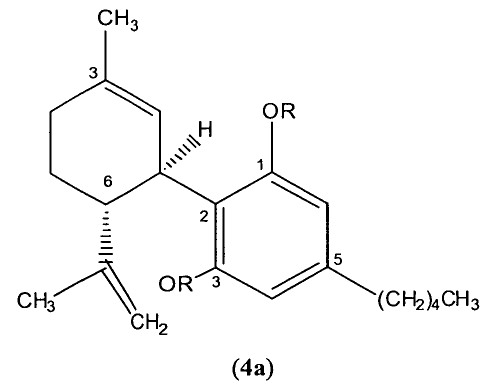 where R is -C(O)(3,5-C₆H₃(NO₂)₂).
(+)-CBD-bis(3,5-dinitrobenzoate) has the structure of formula **(4b):** 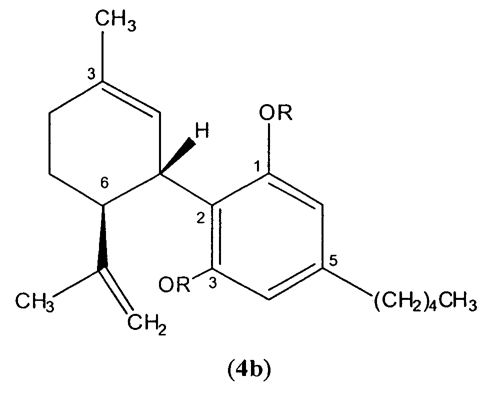 where R is -C(O)(3,5-C₆H₃(NO₂)₂).

As used herein, the generic term "trans-Δ⁹-THC carboxylic acid" may refer to trans-(-)-Δ⁹-THC carboxylic acid; trans-(+)-Δ⁹-THC carboxylic acid; trans-(±)-Δ⁹-THC carboxylic acid; or any mixture thereof. trans-(-)-Δ⁹-THC carboxylic acid has the structure of formula **(5a):** 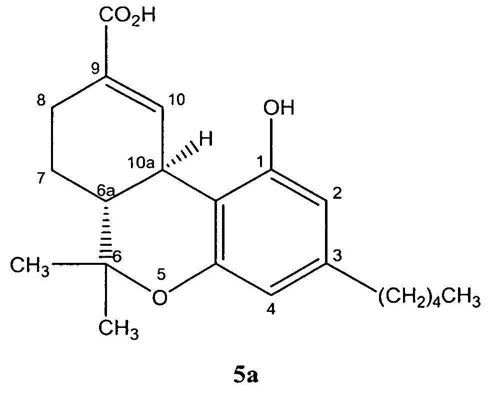 trans-(+)-Δ⁹-THC carboxylic acid has the structure of formula **(5b)**:

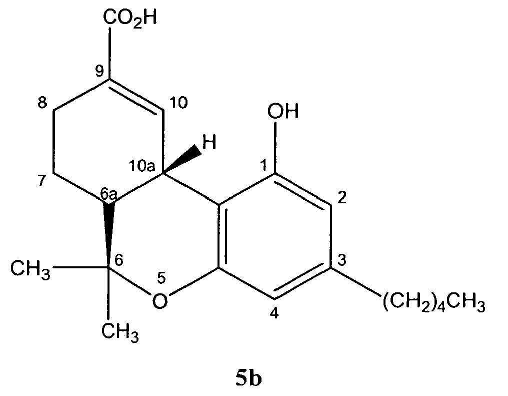

The term "halide" refers to fluoride, chloride, bromide or iodide.
The term "-halo" means -F, -Cl, -Br or -I.
The term "-(C₁-C₄)alkyl" means a saturated straight-chain or branched hydrocarbon having from 1 to 4 carbon atoms. Representative saturated straight chain (C₁-C₄)alkyls are -methyl, -ethyl, -n-propyl, and -n-butyl. Representative saturated branched -(C₁-C₄)alkyls are -isopropyl, -sec-butyl, -isobutyl, and -tert butyl.
The phrase "anhydrous organic solvent," unless otherwise defined herein, means an organic solvent having an amount of water that is less than about 0.01 % by weight of the total amount of water and organic solvent.

The term "cannabinoids" refers to Δ⁹-THC including trans-Δ⁹-THC and cis-Δ⁹-THC; structural isomers of Δ⁹-THC having a molecular formula C₂₁H₃₀O₂, including Δ⁸-THC, (-)-Δ⁸-iso-THC, and (+)-Δ⁸-iso-THC; cannabinol and structural isomers of cannabinol having a molecular formula of C₂₁H₂₈O₂; Δ⁹-THC-carboxylic acid; Δ⁹-THC precursors including CBD, abn-CBD, (+)-abn-CBD, olivetol, (+)-p-mentha-2,8-dien-1-ol and (-)-p-mentha-2,8-dien-1-ol; salts thereof; and derivatives thereof including acids, ethers, esters, amines, and the like.

Unless otherwise specified herein, the phrase "cannabinoid impurities" means cannabinoids other than trans-(-)-Δ⁹-THC, irans-(+)-Δ⁹-THC or (±)-Δ⁹-THC. Unless otherwise specified herein, the generic term "Δ⁹-THC-carboxylic acid" means (-)-Δ⁹-THC-carboxylic acid, (+)-Δ⁹-THC-carboxylic acid, or (±)-Δ⁹-THC-carboxylic acid.

As used herein, the phrase "crystalline trans-(±)-Δ⁹-THC" means a solid, crystalline form of Δ⁹-THC comprising trans-(-)-Δ⁹-THC and trans-(+)-Δ⁹-THC and having an amount of trans-(-)-Δ⁹-THC and trans-(+)-Δ⁹-THC that is at least 95%, at least 98%, at least 99%, at least 99.5% or at least 99.9% by weight based on the total weight of cannabinoids. Crystallinity of the crystalline trans-(±)-Δ⁹-THC of the present invention can be demonstrated, for example, by the presence of any signal(s) determined by powder X-ray diffraction. In one illustrative, non-limiting embodiment, powder X-ray diffraction of crystalline trans-(±)-Δ⁹-THC according to the present invention will provide diffraction data partially or fully equivalent to that presented in Table 1 and FIG 1.

In one embodiment, the crystalline trans-(+)-Δ⁹-THC of the present invention comprises a racemic mixture of trans-(-)-Δ⁹-THC and trans-(+)-Δ⁹-THC. In certain embodiments of the present invention, crystalline trans-(+)-Δ⁹-THC comprises about equimolar amounts of trans-(-)-Δ⁹-THC and trans-(+)-Δ**⁹**-THC. In other embodiments of the present invention, crystalline trans-(±)-Δ⁹-THC comprises, by weight, from about 40% trans-(-)-Δ⁹-THC to about 60% trans-(-)-Δ⁹-THC and from about 60% trans-(+)-Δ⁹-THC to about 40% trans-(+)-Δ⁹-THC; or from about 45% trans-(-)-Δ⁹-THC to about 55% trans-(-)-Δ⁹-THC and from about 55% trans-(+)-Δ⁹-THC to about 45% trans-(+)-Δ⁹-THC; or from about 48% trans-(-)-Δ⁹-THC to about 52% trans-(-)-Δ⁹-THC and from about 52% trans-(+)-Δ⁹-THC to about 48% trans-(+)-Δ⁹-THC; or from about 49% trans-(-)-Δ⁹-THC to about 51 % trans-(-)-Δ⁹-THC and from about 51 % trans-(+)-Δ⁹-THC to about 49% trans-(+)-Δ⁹-THC.

The crystalline trans-(±)-Δ⁹-THC of the present invention may be a polymorphic material, *i.e*. it may exist in more than one crystalline form identified, for example, by a particular "space group" or "crystal class." As used herein, the phrase "crystalline trans-(±)-Δ⁹-THC" is intended to encompass all such polymorphic crystalline forms and is not intended to be limited to any one crystalline form.

As used herein, the phrase "active pharmaceutical ingredient" or "API" means any substance or mixture of substances intended to be used in the manufacture of a drug (medicinal) product and that, when used in the production of a drug, becomes an active ingredient of the drug product. Such substances are intended to furnish pharmacological activity or other direct effect in the diagnosis, cure, mitigation, treatment, or prevention of disease or to affect the structure and function of the body.

The phrase "pharmaceutically acceptable salt," as used herein, refers to a salt prepared from an API having an acidic functional group such as a phenolic group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N,N,-di-lower alkyl-N-(hydroxy lower alkyl)-amines, such as N, N,-dimethyl-N-(2-hydroxyethyl)amine, or tri-(2-hydroxyethyl)amine; N-methyl-D-glucamine; and amino acids such as arginine, lysine, and the like.

The pharmaceutical compositions of the present invention comprise crystalline trans-(±)-Δ⁹-tetrahydrocannabinol and a pharmaceutically-acceptable carrier or excipient. As used herein, the term "carrier" or "excipient" refers to a substance other than the active pharmaceutical ingredient (or "API") included in a composition or dosage form of the present invention. Carriers or excipients, when present, may, without limitation, be selected from one or more of the group consisting of: binders, fillers, compression aids, disintegrants lubricants, glidants, sweetners, coloring agents, flavors, preservative, suspension agents, dispersing agents, film formers, and coatings and any combinations thereof.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" refers to a carbon atom to which four different groups are attached.

The terms "enantiomer," "enantiomeric," and the like, refer to a molecule that is non-superimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers, and which is optically inactive.

As used herein, the terms "patient" and "subject" may be used interchangeably, and mean an animal, particularly a mammal, including, but not limited to a cow, horse, sheep, pig, cat, dog, mouse, rat, rabbit, guinea pig, *etc.,* more preferably a primate, and most preferably a human.

The term "sustained-release" is defined for purposes of the present invention as the release of API from a dosage form at such a rate that blood (*e.g*., plasma) concentrations are maintained within the therapeutic range over a period of time of about 8 hours, about 12 hours, or about 24 hours. In a preferred embodiment, plasma concentrations of trans-(-)-Δ⁹-THC are maintained at a sub-psychotropic level.

As used herein, the term "Cₘₐₓ" denotes the maximum plasma concentration of the API obtained during a dosing interval.

As used herein, the term "C₂₄" denotes the plasma concentration of the API at 24 hours after administration.

As used herein, the term "C₂₄/Cₘₐₓ ratio" refers to the ratio of the plasma concentration of the API at 24 hours after administration to the maximum plasma concentration of the API attained within the dosing interval.

As used herein, the term "adverse agent" means an agent that (a) reduces or eliminates one or more pharmacological effects of a therapeutic agent, such as a euphoric or toxic effect, or (b) causes an undesired physiological reaction, such as emesis. In one embodiment, an oral dosage form of the present invention comprises a first composition and a second composition, wherein the first composition comprises crystalline trans-(±)-Δ⁹-THC as API, and the second composition comprises an adverse agent. In certain embodiments, the adverse agent is coated with a layer that is substantially insoluble in the gastrointestinal tract. When such an oral dosage form is orally administered to a patient as intended (*i.e*., in an untampered form), only the API of the first composition is substantially released in the gastrointestinal tract of the patient, and the adverse agent is not substantially released. However, if the oral dosage form is tampered with so that the coating on the second composition is breached, then the adverse agent will also be substantially released upon administration, thereby reducing the euphoric effect of the first composition or causing an undesired physiological reaction.

### 5.2 Methods for Isolating trans-(-)-Δ⁹-THC

Trans-(-)-Δ⁹-THC can be extracted and purified from *Cannabis sativa* plant material, as well as hashish, according to methods disclosed in the art (*see e.g.* WO 03/064407 A2; Turk et al. (1971) J. Pharm. Pharmac. 23: 190-195; Y. Gaoni et al., J. Am. Chem. Soc. 93:217 (1971); and U.S. Patent No. 6,365,416 B1 to Elsohly et al.). For example, according to one published method, macerated or powdered plant material can be extracted with a non-polar solvent such as hexane, and the resulting extract chromatographed on a silica gel column. Selected fractions can be pooled and subjected to fractional distillation under vacuum to provide approximately 90% pure THC. Additional purification involving either a second fractional distillation or HPLC purification can provide substantially pure THC *(see* U.S. Patent No. 6,365,416 B1). In another approach (R.F. Turk et al., J. Pharm. Pharmac. 23:190-195 (1971)), trans-(-)-Δ⁹-THC can be isolated from marijuana plant tissue, but the product can contain an undetermined amount of carboxylic precursors of THC. In both instances, the plant extracts may contain trans-(-)-Δ⁹-THC as well as impurities such as cannabinoid isomers from which the desired compound must be separated.

Trans-(-)-Δ⁹-THC can be chemically synthesized according to methods disclosed in the art. For example, U.S. Patent No. 3,560,528 to Petrizilka describes the reaction of a *cis*/*trans* mixture of (+)-*p*-mentha-2,8-dien-1-ol with olivetol in the presence of an acid catalyst such as *p*-toluenesulfonic acid monohydrate ("PTSA-H₂O") or trifluoroacetic acid as a dehydrating agent in refluxing benzene to provide (-)-Δ⁸-THC, which can be converted to trans-(-)-Δ⁹-THC by addition of HCl followed by dehydrochlorination (*see* Y. Mechoulam et al., J. Am. Chem. Soc. 89:4553 (1967); and R. Mechoulam et al., J. Am. Chem. Soc. 94:6159 (1972)).

U.S. Patent No. 4,025,516 to Razdan et al. describes the reaction of a mixture of *cis*/*trans*-(+)-*p*-mentha-2,8-dien-1-ol with olivetol in an inert organic solvent in the presence of an excess of a non-alkaline dehydrating agent and an acid catalyst to form trans-(-)-Δ⁹-THC. This patent also describes the reaction of (-)-cannabidiol ("(-)-CBD" or "(-)-abnormal-CBD" ("(-)-abn-CBD")) with a Lewis acid such as boron trifluoride diethylether ("BF_{3·}Et₂O") in an inert solvent under anhydrous conditions to form trans-(-)-Δ⁹-THC (*also see* WO 03/070506).

R. K. Razdan et al., J. Am. Chem. Soc. 96:5860 (1974) describes the reaction of a *cis*/*trans* mixture of (+)-*p*-mentha-2,8-dien-1-ol with olivetol in the presence of 1% BF₃·Et₂O, methylene chloride and anhydrous magnesium sulfate to form trans-(-)-Δ⁹-THC.

U.S. Patent No. 4,381,399 to Olsen et al. describes a method for separating trans-(-)-Δ⁹-THC from a crude synthetic mixture, the method comprising esterifying the crude mixture, isolating the resultant trans-(-)-Δ⁹-THC ester, hydrolyzing the ester, and distilling trans-(-)-Δ⁹-THC at reduced pressure. Additional methods for the synthesis of Δ⁹-THC are disclosed in U.S. Patent No. 5,227,537 to Stoss et al.; in Razdan et al. (1975) Experientia 31: 16; and in International PCT Publication WO 02/096899 Al.

In addition to those methods disclosed in the art, the present invention and the disclosure of U.S. provisional application Serial No. 60/630,556 provide methods for making compositions comprising at least 98%, at least 99%, at least 99.5% or at least 99.9% by weight of trans-(-)-Δ⁹-THC based on the total amount of cannabinoids. More specifically, described herein is a method for fractionating trans-(+)-Δ⁹-THC, *e*.*g*. crystalline trans-(+)-Δ⁹-THC, on a chiral stationary phase to provide trans-(-)-Δ⁹-THC. Without being limited by theory, the present inventor believes that cannabinoid impurities typically present in Δ⁹-THC compositions are substantially or completely removed when trans-(-)-Δ⁹-THC and trans-(+)-Δ⁹-THC are allowed to form crystalline trans-(±)-Δ⁹-THC. Thus, the subsequent resolution of trans-(+)-Δ⁹-THC obtained from crystalline trans-(±)-Δ⁹-THC with an eluting solvent on a chiral stationary phase provides a composition comprising at least 98% by weight of trans-(-)-Δ⁹-THC based on the total amount of cannabinoids in the composition. Thus, in this embodiment, trans-(-)-Δ⁹-THC, which can be used in the Crystallizing Step can be reused or "recycled" from a previous resolution of trans-(+)-Δ⁹-THC on a chiral stationary phase, *e.g*. as described in Section 4.6, below.

### 5.3 Methods for Isolating trans-(+)-Δ⁹-THC

Trans-(+)-Δ⁹-THC, which is not known to occur in nature, can be made by known synthetic methods including, but not limited to, reaction of (+)-Δ⁸-THC with HCl followed by dehydrochlorination (*see* R. Mechoulam et al., J. Am. Chem. Soc. 94:6159 (1972)). Alternatively, trans-(+)-Δ⁹-THC can be synthesized according to other methods disclosed in the art (U.S. Patent No. 3,560,528 to Petrizilka; Y. Mechoulam et al., J. Am. Chem. Soc. 89:4553 (1967); U.S. Patent No. 4,025,516 to Razdan et al.; R. K. Razdan et al., J. Am. Chem. Soc. 96:5860 (1974)), provided enantiomerically pure (-)-*p*-mentha-2,8-dien-1-ol is used as the reagent reacted with olivetol to provide the intermediate (*i.e*., cannabidiol), which ultimately can be converted to trans-(+)-Δ⁹-THC. Trans-(+)-Δ⁹-THC can be obtained by methods described in Section 6, below.

In addition to methods disclosed in the art, the present invention and the disclosure of U.S. provisional application Serial No. 60/630,556 provide methods for making compositions comprising at least 98%, at least 99%, at least 99.5% or at least 99.9%by weight of trans-(+)-Δ⁹-THC based on the total amount of cannabinoids in the composition. More specifically, described herein is a method for fractionating trans-(±)-Δ⁹-THC, *e.g*. crystalline trans-(±)-Δ⁹-THC, on a chiral stationary phase to provide a trans-(+)-Δ⁹-THC composition. Without being limited by theory, the present inventor believes that cannabinoid impurities typically present in Δ⁹-THC compositions are substantially or completely removed when trans-(-)-Δ⁹-THC and trans-(+)-Δ⁹-THC are allowed to form crystalline trans-(±)-Δ⁹-THC. Thus, the subsequent resolution of trans-(±)-Δ⁹-THC obtained from crystalline trans-(±)-Δ⁹-THC with an eluting solvent on a chiral stationary phase provides a composition comprising at least 98%, at least 99%, at least 99.5% or at least 99.9% by weight of trans-(+)-Δ⁹-THC based on the total amount of cannabinoids in the composition. Thus, in this embodiment, trans-(+)-Δ⁹-THC, which can be used in the Crystallizing Step can be reused or "recycled" from a previous resolution of trans-(+)-Δ⁹-THC on a chiral stationary phase, *e.g*. as described in Section 4.6, below.

Another chromatographic approach to the isolation of enantiomeric forms of Δ⁹-THC is described in S. L. Levin et al., J. Chromatogr. A 654:53-64 (1993), which describes a method for resolving trans-(-)-Δ⁹-THC and trans-(+)-Δ⁹-THC from a composition comprising equimolar amounts of the trans-(-)- and trans-(+)-enantiomer. This chromatographic separation can be carried out on a column comprising an amylose tris(3,5-dimethylphenylcarbamate) stationary phase immobilized on silica gel.

### 5.4 Methods for Isolating trans-(±)-Δ⁹-THC Mixtures

A mixture of enantiomers comprising both trans-(-)-Δ⁹-THC and trans-(+)-Δ⁹-THC can be obtained by direct chemical synthesis. When such a synthetic method is used, the ratio of trans-(-)-Δ⁹-THC to trans-(+)-Δ⁹-THC can vary depending on the optical purity of the reagents and the choice of synthetic process. Thus, trans-(-)-Δ⁹-THC and trans-(+)-Δ⁹-THC can be obtained in about equimolar amounts by a synthetic route using racemic reagents. Non-limiting methods for preparing a mixture of trans-(-)-Δ⁹-THC and trans-(+)-Δ⁹-THC by a direct synthetic route include reaction of citral and olivetol in the presence of a Lewis acid *(see* R. Mechoulam et al., J. Am. Chem. Soc. 94:6159 (1972)), or hydrolysis of (±)-1-*m*-nitrobenzenesulfoanate-6a,10a-trans-Δ⁹-THC with NaOH in aqueous methanol (K.E. Fahrenholtz et al., J. Am. Chem. Soc. 89:5934-5941 (1967)). More specifically, K.E. Fahrenholtz et al., J. Am. Chem. Soc. 89:5934-5941 (1967) describes the synthesis of a mixture of *dl*-Δ⁹-tetrahydrocannabinol and *dl*-Δ⁸-tetrahydrocannabinol (in a 74:26 ratio) upon reaction of 9-chloro-6a,7,8,9,10,10a-hexahydro-6,6,9-trimethyl-3-pentyl-6H-benzo[c]chromen-1-ol with sodium hydride. This reference also describes the hydrolysis of (±)-1-/M-nitrobenzenesulfoanate-6a,10a-trans-Δ⁹-tetrahydrocannabinol with NaOH in aqueous methanol to provide trans-(±)-Δ⁹-THC, which could subsequently be crystallized from hexane as light tan crystals. In another approach, described in E.G. Taylor et al., J. Am. Chem. Soc. 88:367 (1966), citral can be reacted with olivetol in acidified ethanol to form trans-(±)-Δ-THC in approximately 35% yield. Alternatively, trans-(+)-Δ⁹-THC can be obtained by methods described in Section 6, below.

### 5.5 Methods for Isolating Crystalline trans-(±)-Δ⁹-THC

Crystalline trans-(±)-Δ⁹-THC useful in the present invention can be obtained by any known or later-developed method. For example, a non-limiting method for obtaining crystalline trans-(±)-Δ⁹-THC includes crystallization from a first composition comprising trans-(-)-Δ⁹-THC, trans-(+)-Δ⁹-THC, and a non-polar organic solvent to provide crystalline trans-(±)-Δ⁹-THC, as described below.

Compositions comprising trans-(-)-Δ⁹-THC, trans-(+)-Δ⁹-THC, and trans-(±)-Δ⁹-THC useful for purifying crystalline trans-(±)-Δ⁹-THC can be obtained by methods described in Sections 4.2, 4.3. and 4.4, above, respectively, as well as in Section 6, below. In addition to those methods, in another embodiment, the trans-(-)-Δ⁹-THC and trans-(+)-Δ⁹-THC, which may be used in the Crystallizing Step (described below) can be obtained from derivatives of trans-(-)-Δ⁹-THC and trans-(+)-Δ⁹-THC. For example, an admixture of trans-(-)-Δ⁹-THC and trans-(+)-Δ⁹-THC can be reacted with a phenol protecting group such as m-nitrobenzenesulfonate and crystallized to provide 2-*m*-nitrobenzenesulfonate-(±)-Δ⁹-THC *(see* U.S. Patent No. 3,507,885 to Fahrenholtz; and K.E. Fahrenholtz et al., J. Am. Chem. Soc. 89:5934-5491 (1967)). The 2-*m*-nitrobenzenesulfonate-(±)-Δ⁹-THC can then be deprotected, and the resultant composition comprising trans-(-)-Δ⁹-THC and trans-(+)-Δ⁹-THC can be crystallized from a composition comprising the trans-(-)-Δ⁹-THC, the trans-(+)-Δ⁹-THC, and a non-polar organic solvent to provide crystalline trans-(+)-Δ⁹-THC.

In another embodiment, certain impurities in the trans-(+)-Δ⁹-THC, trans-(-)-Δ-THC, and/or trans-(±)-Δ⁹-THC can be removed according to the "Δ⁹-THC Purification Method" disclosed below, prior to the use of those Δ⁹-THC materials in the Crystallization Step. This Δ⁹-THC Purification Method includes a "Caustic Contacting Step," in which the trans-(+)-Δ⁹-THC, the trans-(-)-Δ⁹-THC, and/or the trans-(±)-Δ⁹-THC composition to be purified is contacted with base. This first step yields an alcoholic-caustic phase that, in the second step of the Δ⁹-THC Purification Method, is contacted with acid to provide an acid-treated alcoholic phase, in which, the present inventor believes, trans-(+)-Δ⁹-THC and trans-(-)-Δ⁹-THC are not soluble.

Crystalline trans-(±)-Δ⁹-THC can also be obtained by allowing trans-(-)-Δ⁹-THC and trans-(+)-Δ⁹-THC to crystallize from a composition comprising trans-(-)-Δ⁹-THC, trans-(+)-Δ⁹-THC and a non-polar organic solvent (the "Crystallizing Step") to provide crystalline trans-(±)-Δ⁹-THC and a liquid phase. Compositions comprising trans-(-)-Δ⁹-THC, trans-(+)-Δ⁹-THC and a non-polar organic solvent useful for the Crystallizing Step can be obtained by any known or later-developed method. For example, crystalline trans-(+)-Δ⁹-THC can be obtained by contacting a suitable amount of trans-(-)-Δ⁹-THC and trans-(+)-Δ⁹-THC with a non-polar organic solvent. The order and rate of addition of the trans-(-)-Δ⁹-THC, the trans-(+)-Δ⁹-THC and the non-polar organic solvent are not critical and can be carried out sequentially or substantially simultaneously. As an example, trans-(-)-Δ⁹-THC, optionally in the presence of a non-polar organic solvent, and trans-(+)-Δ⁹-THC, optionally in the presence of a non-polar organic solvent, can be added to a non-polar organic solvent. Likewise, trans-(+)-Δ⁹-THC in the presence of a non-polar organic solvent and trans-(-)-Δ⁹-THC in the presence of a non-polar organic solvent can be admixed.

The ratio of trans-(-)-Δ⁹-THC and trans-(+)-Δ⁹-THC used in the Crystallizing Step can vary within certain limits. In one embodiment, the trans-(-)-Δ⁹-THC is present in an amount from about 0.75 to about 1.25 molar equivalents per molar equivalent of trans-(+)-Δ⁹-THC. In another embodiment, the trans-(-)-Δ⁹-THC is present in an amount from about 0.9 to about 1.1 molar equivalents per molar equivalent of trans-(+)-Δ⁹-THC. In another embodiment, the trans-(-)-Δ⁹-THC is present in an amount from about 0.95 to about 1.05 molar equivalents per molar equivalent of trans-(+)-Δ⁹-THC. In another embodiment, the trans-(-)-Δ⁹-THC is present in an amount of about 1 molar equivalent per molar equivalent of trans-(+)-Δ⁹-THC. Non-limiting examples of non-polar organic solvents that are useful in the Crystallizing Step include aliphatic (C₄-C₁₀)hydrocarbons such as a straight-chain aliphatic hydrocarbon, a branched aliphatic hydrocarbon or a cyclic aliphatic hydrocarbon, such as a butane, a pentane, a hexane, a heptane, an octane, a nonane, or a decane, or any mixture thereof.

In one embodiment, the non-polar organic solvent used in the Crystallizing Step is a straight-chain or branched-chain heptane. In another embodiment, the non-polar organic solvent used in the Crystallizing Step is a pentane, hexane, heptane, octane or iso-octane. In a specific embodiment, the non-polar organic solvent used in the Crystallizing Step is n-heptane.

The amount of the non-polar organic solvent that can be used in the Crystallizing Step can vary and will depend, in part, on the amount and type of cannabinoid impurities and temperature. Typically, the non-polar organic solvent can be present in an amount sufficient to provide a mixture having a Δ⁹-THC concentration from about 1% to about 95%, preferably from about 20% to about 75%, more preferably from about 40% to about 60% by weight based on the total amount of Δ⁹-THC and the non-polar organic solvent.

The Crystallizing Step can be carried out for a time and at a temperature sufficient to provide trans-(±)-Δ⁹-THC crystals. A time sufficient to crystallize trans-(±)-Δ⁹-THC can be from about 1 hour to about 200 hours; or from about 5 hours to about 150 hours; or from about 25 hours to about 100 hours; or from about 30 hours to about 75 hours.

Typically, a temperature range sufficient to provide crystalline trans-(±)-Δ⁹-THC can be from about -78°C to about 100°C; from about -50°C to about 25°C; from about -30°C to about 0°C; or from about -25°C to about -15°C.

In certain embodiments, the Crystallizing Step can be carried out at two or more different temperatures. In one embodiment, the composition comprising trans-(-)-Δ⁹-THC, trans-(+)-Δ⁹-THC and a non-polar organic solvent can be prepared at a first temperature, *e.g*., 20°C or higher. Without being limited by theory, the present inventor believes that forming the composition at a temperature of 20°C or higher can increase the solubility of the trans-(-)-Δ⁹-THC and the trans-(+)-Δ⁹-THC in the non-polar organic solvent. The temperature of the admixture can then be decreased to a second temperature, *e*.*g*., 0°C or lower. Without being limited by theory, the present inventor believes that holding the admixture at a temperature of 0°C or lower can decrease the solubility of trans-(±)-Δ⁹-THC and promote crystallization. Optionally, the temperature of the admixture can be further decreased to *e.g*., to -15°C to -20°C, to enhance the trans-(±)-Δ⁹-THC crystallization process.

In one embodiment, trans-(-)-Δ⁹-THC and trans-(+)-Δ⁹-THC are dissolved in a non-polar organic solvent; the resultant solution is cooled to about -15°C; and the resultant crystalline trans-(±)-Δ⁹-THC is separated from the liquid phase.

The Crystallizing Step can be carried out in the presence of a seed crystal. Typically, the seed crystal, when used, can be added to the cooled (*e.g*., 0°C or lower) admixture comprising trans-(-)-Δ⁹-THC, trans-(+)-Δ⁹-THC and the non-polar organic solvent. In one embodiment, the seed crystal is crystalline trans-(±)-Δ⁹-THC.

The progress of the Crystallizing Step can be monitored visually or using conventional analytical techniques, such as *e.g*., thin-layer chromatography ("TLC"), high-performance liquid chromatography ("HPLC"), gas chromatography ("GC"), gas-liquid chromatography ("GLC"), infrared spectroscopy ("IR"), Raman spectroscopy ("Raman") or nuclear magnetic resonance spectroscopy ("NMR") such as ¹H or ¹³C NMR.

The Crystallizing Step can be carried out at reduced pressure, atmospheric pressure or elevated pressure. In a specific embodiment, the Crystallizing Step is carried out at atmospheric pressure.

As noted above, certain impurities can be removed from the trans-(-)-Δ⁹-THC, trans-(+)-Δ⁹-THC, and/or trans-(±)-Δ⁹-THC compositions prior to carrying out the Crystallizing Step. Non-limiting methods for removing impurities prior to carrying out the Crystallizing Step include column chromatography or extraction under basic conditions as described herein below.

In one embodiment, trans-(+)-Δ⁹-THC, trans-(-)-Δ⁹-THC, or trans-(+)-Δ⁹-THC can be contacted with base prior to carrying out the Crystallizing Step.

In another embodiment, trans-(+)-Δ⁹-THC, trans-(-)-Δ⁹-THC, or trans-(±)-Δ⁹-THC can be purified using the "Δ⁹-THC Purification Method," which comprises contacting the trans-(+)-Δ⁹-THC, trans-(-)-Δ⁹-THC or trans-(±)-Δ⁹-THC, respectively, with a first water-immiscible organic solvent, a water-miscible alcohol, water, and an alkali metal hydroxide (the "Caustic Contacting Step") to form a biphasic mixture comprising (i) a first organic phase and (ii) an alcoholic-caustic phase comprising the trans-(+)-Δ⁹-THC, trans-(-)-Δ⁹-THC or trans-(±)-Δ⁹-THC. Without being limited by theory, the present inventor believes that the Caustic Contacting Step serves to remove impurities from the trans-(+)-Δ⁹-THC-, trans-(-)-Δ⁹-THC- or trans-(+)-Δ⁹-THC -containing alcoholic-caustic phase into the first organic phase, which impurities would otherwise impede or prevent trans-(±)-Δ⁹-THC from crystallizing.

The amount of alkali metal hydroxide such as NaOH, KOH, LiOH or CsOH, preferably NaOH or KOH to be used in the Caustic Contacting Step typically ranges from about 1 to about 1000 molar equivalents, from about 10 to about 100 molar equivalents, or from about 25 to about 55 molar equivalents, per molar equivalent of the trans-(+)-Δ⁹-THC, trans-(-)-Δ⁹-THC or trans-(±)-Δ⁹-THC.

Non-limiting examples of water-miscible alcohols that can be used in the Caustic Contacting Step include methanol, ethanol, isopropanol, or any combination thereof. In a specific embodiment, the water-miscible alcohol is methanol.
The amount of water-miscible alcohol that can be used in the Caustic Contacting Step typically is from about 1 part to about 100 parts by weight, from about 1 part to about 25 parts by weight, or from about 5 parts to about 10 parts by weight, based on the weight of the alkali metal hydroxide.

Non-limiting examples of a first water-immiscible organic solvent useful in the Caustic Contacting Step include the non-polar organic solvents described above for the Crystallizing Step. In a specific embodiment, the first water-immiscible solvent is n-heptane.

The amount of the first water-immiscible organic solvent used in the Caustic Contacting Step typically can be from about 1 part to about 1000 parts by weight, from about 5 parts to about 100 parts by weight, or from about 5 parts to about 20 parts by weight, based on the weight of the Δ⁹-THC.

The Caustic Contacting Step can be carried out by methods known in the art such as, but not limited to, stirring, shaking, countercurrent cascade, ultrasound admixing, or pumping. The Caustic Contacting Step can also be carried out by methods useful for liquid-liquid extraction (*see, e.g.,* Lo et al., "Extraction," in 7 Kirk-Othmer Encyc. of Chem. Technol. 349-381 (4th ed. 1993), which is incorporated herein by reference). The Caustic Contacting Step typically can be carried out in a time period of from about 0.25 hours to about 50 hours, or from about 0.25 hours to about 10 hours, or from about 0.25 hours to about 2 hours.

The Caustic Contacting Step is typically carried out in a temperature range of from about 0°C to about 100 °C, or from about 20°C to about 50°C, or from about 20°C to about 30°C.

The Caustic Contacting Step can be carried out at reduced pressure, or at atmospheric pressure (*i.e*., about 1 atmosphere), or at elevated pressure. In a specific embodiment, the Caustic Contacting Step is carried out at atmospheric pressure. The progress of the Caustic Contacting Step can be monitored using conventional techniques, such as those described above for the Crystallizing Step.

The Δ⁹-THC Purification Method can further comprise a second step in which the alcoholic-caustic phase is contacted with an acid to provide an acid-treated alcoholic phase. Without being limited by theory, the present inventor believes that Δ⁹-THC is immiscible in the acidified alcoholic phase. Non-limiting examples of acids useful in this second step include citric acid, acetic acid, and the like. In a specific embodiment, the acid is citric acid.

Typically, the acid can be added in an amount sufficient to achieve a pH of from about 5 to about 9, a pH from about 6 to about 8, or a pH of from about 7 to about 8. The Δ⁹-THC Purification Method may further comprise contacting the acid-treated alcoholic phase with a second water-immiscible organic solvent to form: (i) a second organic phase comprising Δ⁹-THC; and (ii) an acid-treated alcoholic phase. Non-limiting examples of second water-immiscible organic solvents useful for contacting the acid-treated alcoholic phase to form a second organic phase comprising Δ⁹-THC include the non-polar organic solvents described above for the Crystallizing Step. In one embodiment, the second water-immiscible organic solvent is n-heptane. The amount of the second water-immiscible organic solvent used typically can be from about 1 part to about 1000 parts by weight, or from about 1 part to about 50 parts by weight, or from about 1 part to about 10 parts by weight, based on the weight of the Δ⁹-THC. Methods useful for contacting the acid-treated alcoholic phase with a second water-immiscible organic solvent include those described above for the Caustic Contacting Step.

The Δ⁹-THC Purification Method can further comprise separating the second organic phase from the acid-treated alcoholic phase. Methods useful for separating the second organic phase from the acid-treated alcoholic phase include those described above for separating the first organic phase from the alcoholic-caustic phase. After separation from the acid-treated alcoholic phase, the second organic phase can be dried, *e.g*., by azeotropic distillation and/or contacting the second organic phase with a drying agent (*e.g*., Na₂SO₄ or MgSO₄).

The Δ⁹-THC Purification Method can further comprise the step of concentrating the second organic phase to form a concentrated second organic phase comprising Δ⁹-THC, *e.g*. by distillation. The distillation can be carried out at elevated pressure, atmospheric pressure, or at reduced pressure. In one embodiment, the distillation is carried out at atmospheric pressure. In another embodiment, the distillation is carried out at reduced pressure.

The Δ⁹-TH Purification Method can further comprise contacting the concentrated second organic phase with a non-polar organic solvent to form a first organic composition comprising Δ⁹-THC. The amount and type of non-polar organic solvent can be any of those described above in the Crystallizing Step for the non-polar organic solvent.

In one embodiment, the Δ⁹-THC used in the Δ⁹-THC Purification Method comprises trans-(-)-Δ⁹-THC. In another embodiment, the Δ⁹-THC used in the Δ⁹-THC Purification Method comprises trans-(+)-Δ⁹-THC. In another embodiment, the trans-Δ⁹-THC used in the Δ⁹-THC Purification Method comprises both trans-(-)-Δ⁹-THC and trans-(+)-Δ⁹-THC. In certain embodiments, the trans-(-)-Δ⁹-THC is present in a range of from about 0.75 to about 1.25 molar equivalents per molar equivalent of trans-(+)-Δ⁹-THC.

The Δ⁹-THC Purification Method can further comprise adding trans-(-)-Δ⁹-THC or trans-(+)-Δ⁹-THC to the first organic composition in an amount sufficient to provide a second organic composition comprising trans-(-)-Δ⁹-THC and trans-(+)-Δ⁹-THC, wherein the trans-(-)-Δ⁹-THC is present preferably in an amount of from about 0.75 to about 1.25 molar equivalents per molar equivalent of trans-(+)-Δ⁹-THC, and allowing trans-(-)-Δ⁹-THC and trans-(+)-Δ⁹-THC to crystallize to provide crystalline trans-(+)-Δ⁹-THC as described above for the Crystallizing Step.

The Δ⁹-THC Purification Method can further comprise allowing the trans-(-)-Δ⁹-THC and trans-(+)-Δ⁹-THC to crystallize from the first organic composition to provide crystalline trans-(+)-Δ⁹-THC as described above for the Crystallizing Step, wherein (a) the first organic composition comprises trans-(-)-Δ⁹-THC and trans-(+)-Δ⁹-THC, and (b) the trans-(-)-Δ⁹-THC is present in first organic composition preferably in an amount from about 0.75 to about 1.25 molar equivalents per molar equivalent of trans-(+)-Δ⁹-THC.

In one embodiment, a method for making crystalline trans-(±)-Δ⁹-THC, comprises allowing trans-(-)-Δ⁹-THC and trans-(+)-Δ⁹-THC to crystallize from a first composition comprising trans-(-)-Δ⁹-THC, trans-(+)-Δ⁹-THC, and a non-polar organic solvent to provide crystalline trans-(+)-Δ⁹-THC, wherein the trans-(-)-Δ⁹-THC and the trans-(+)-Δ⁹-THC were obtained by: (a) forming a biphasic composition comprising (i) a first organic phase comprising a first water-immiscible organic solvent, and (ii) an alcoholic-caustic phase containing trans-(-)-Δ⁹-THC and trans-(+)-Δ⁹-THC; and (b) separating the trans-(-)-Δ⁹-THC and the trans-(+)-Δ⁹-THC from the alcoholic-caustic phase.

Methods for forming the biphasic composition, as well as the amounts and type of water-immiscible organic solvent, water-miscible alcohol, water, and alkali metal hydroxide can be selected from those described above for the Caustic Contacting Step. Similarly, methods for separating the trans-(-)-Δ⁹-THC and the trans-(+)-Δ⁹-THC from the alcoholic-caustic phase, and methods for forming the first composition comprising (i) the trans-(-)-Δ⁹-THC and the trans-(+)-Δ⁹-THC of step (b), and (ii) the non-polar organic solvent, can be selected from those methods described above for the Δ⁹-THC Purification Method.

Once obtained, crystalline trans-(±)-Δ⁹-THC formed in the Crystallizing Step can be separated from the liquid phase by methods known in the art. Methods for separating the crystalline trans-(±)-Δ⁹-THC from the liquid phase can include, *e.g*., filtration, centrifugation, decantation or a combination thereof. In a specific embodiment, crystalline trans-(±)-Δ⁹-THC is separated from the liquid phase by filtration.

Crystalline trans-(±)-Δ⁹-THC formed in the Crystallizing Step can optionally be washed with an organic wash solvent, and separated from the liquid phase as described above. When crystalline trans-(±)-A⁹-THC is washed, the temperature of the organic wash solvent can be varied. Typically, however, the washing, when done, will be carried out with an organic wash solvent at a temperature from about -78°C to about 50°C, from about -30°C to about 30°C, or from about -20°C to about 25°C.

Examples of useful organic wash solvents include any of the non-polar organic solvents described above. In a specific embodiment, the organic wash solvent, when used, is n-heptane.

The separated trans-(±)-Δ⁹-THC can optionally be dried. The drying can be carried out at atmospheric pressure, optionally with the aid of a sweep gas such as dry air, nitrogen, helium, argon, or the like. Alternatively, the trans-(±)-Δ⁹THC can be dried at reduced pressure.

When the separated trans-(±)-Δ⁹-THC is dried, the drying temperature can be varied. Typically, the drying, when done, can be carried out at a temperature from about -25° to about 65°C, or from about 0° to about 60°C, or from about 25° to about 50°C. Typically, the trans-(±)-Δ⁹-THC obtained in the Crystallizing Step comprises at least 95% by weight, or at least 98% by weight, or at least 99%, or at least 99.5%, or at least 99.9% by weight of trans-(-)-Δ⁹-THC and trans-(+)-Δ⁹-THC, based on the total amount of cannabinoids.

### 5.6 The Resolving Step

The separated trans-(±)-Δ⁹-THC, which can be prepared according to the methods disclosed in the preceding section, can be resolved on a chiral stationary phase to provide purified trans-(-)-Δ⁹-THC and purified trans-(+)-Δ⁹-THC, where desired. Thus, according to the present invention, trans-(±)-Δ⁹-THC obtained from crystalline trans-(±)-Δ⁹-THC and an eluting solvent can be contacted with a chiral stationary phase to resolve the trans-(-)- and (+)-enantiomers (the "Resolving Step"). This can provide a composition comprising at least 98% by weight of trans-(-)-Δ⁹-THC or at least 98% by weight of trans-(+)-Δ⁹-THC based on the total amount of cannabinoids. In a further preferred embodiment of the invention the compositions comprise at least 99%, preferably at least 99.5% and more preferably 99.9% by weight of trans-(-)-Δ⁹-THC or of trans-(+)-Δ⁹-THC based on the total amount of cannabinoids. Without being limited by theory, the present inventor believes that resolving trans-(±)-Δ⁹-THC obtained from crystalline trans-(±)-Δ⁹-THC can provide a trans-(-)-Δ**⁹**-THC or trans-(+)-Δ⁹-THC composition having very low levels of, or no cannabinoid impurities found in trans-(-)-Δ⁹-THC or trans-(+)-Δ⁹-THC obtained by prior methods.

The composition comprising trans-(±)-Δ⁹-THC used in the Resolving Step can contain an amount of trans-(-)-Δ⁹-THC that is less than, equal to or greater than the amount of trans-(+)-Δ⁹-THC. For example, the composition comprising (±)-Δ⁹-THC may be obtained by admixing crystalline (±)-Δ⁹-THC with a trans-(-)-Δ⁹-THC composition and/or a trans-(+)-Δ⁹-THC prior to carrying out the Resolving Step. Typically, the composition comprising trans-(±)-Δ⁹-THC can contain about an equimolar amount of the trans-(-)-Δ⁹-THC and trans-(+)-Δ⁹-THC.

Any known or later-developed chiral stationary phase or methodology useful to resolve trans-(-)-Δ⁹-THC and trans-(+)-Δ⁹-THC can be used. For example, a method for resolving trans-(-)-Δ⁹-THC and trans-(+)-Δ⁹-THC enantiomers on a chiral stationary phase is described in S. L. Levin et al., J. Chromatogr. A 654:53-64 (1993)). Typically, the chiral stationary phase can contain a chiral group or derivative immobilized on a support such as, *e.g*., a polymer or inorganic oxide. A non-limiting example of a useful polymer support is polystyrene in bead form. Non-limiting examples of useful inorganic oxide supports include silica, magnesium silicate, magnesia, alumina and molecular sieves. In one embodiment, the inorganic oxide support is silica.

The immobilized chiral derivative comprises at least one chiral center. Non-limiting examples of useful chiral derivatives include tris(arylcarbamate) derivatives of saccharides such as, *e.g*., amylose, cellulose, chitosin, xylan, curdlan, dextran, and inulan. In one embodiment, the saccharide is amylose. In one embodiment, the tris(arylcarbamate) is tris(3,5-dimethylphenylcarbamate); tris(4-chlorophenylcarbamate); tris(4-methylcarbamate); tris(4-methylbenzoate); or tris[(S)-phenylethylcarbamate]. In another embodiment, the tris(arylcarbamate) is tris(3,5-dimethylphenylcarbamate). In another embodiment, the chiral stationary phase is amylose tris(3,5-dimethylcarbonate) immobilized on silica, available as Chiralpak^{®} AD^{™} from Daicel Chemical Industries, Tokyo, Japan.

Other non-limiting examples of useful chiral stationary phases include cellulose triacetate; cellulose tribenzoate; poly[(S)-N-acrylolyphenylalanine ethyl ester]; 3,5-dinitrobenzoylphenylglycine; crosslinked di-(3,5-dimethylbenzoyl)-L diallyltartramide; crosslinked di-(4-*tert*-butylbenzoyl)-L diallyltartramide; and tetrahydro-aminophenanthrene 3,5-dinitrobenzamide *(see* E.R. Francotte, J. Chromatogr. A 906:379-397 (2001)).

Typically, a concentrated solution of trans-(±)-Δ⁹-THC and an eluting solvent can be added to the top (or front) of a column containing a chiral stationary phase. The trans-(±)-Δ⁹-THC can then be eluted with the eluting solvent (*i.e*., the mobile phase) to provide eluent containing trans-(-)-Δ⁹-THC or trans-(+)-Δ⁹-THC.

The Resolving Step can be carried out using batch chromatography, continuous chromatography, or simulated moving bed chromatography (see, *e.g*., E.R. Francotte, J. Chromatogr. A 906:379-397 (2001)). In one embodiment, the Resolving Step is carried out using continuous chromatography.

The Resolving Step can be carried out at about 1 atmosphere of pressure, or at reduced pressure, or at elevated pressure. In one embodiment, the Resolving Step is carried out at about 1 atmosphere of pressure. In another embodiment, the Resolving Step is carried out at elevated pressure. In one embodiment, the Resolving Step is carried out using flash chromatography at from about 1.1 to about 10 atmospheres; from about 1.1 to about 5 atmospheres; or from about 1.1 to about 1.3 atmospheres. In another embodiment, the Resolving Step is carried out at using flash chromatography at from about 10 to about 175 atmospheres; from about 100 to about 175 atmospheres; from about 125 to about 175 atmospheres; or at about 150 atmospheres.

Non-limiting examples of eluting solvents useful in the Resolving Step include: (a) straight-chain or branched-chain (C₁-C₄)alkyls substituted with one or more -OH, -OR₁, -OC(O)R₁, -C(O)OR₁, -halo, or -CN; (b) straight-chain or branched-chain (C₄-C₁₀)aliphatic hydrocarbons; (c) (C₅-C₇)cycloaliphatic hydrocarbon optionally substituted with one or more -R₁; (d) (C₄-C₇)cyclic ethers optionally substituted with one or more -R₁; (e) aromatic hydrocarbons optionally substituted with one or more -R₁, -halo, -CH₂(halo), -CH(halo)₂, -C(halo)₃, or -O(C₁-C₆)alkyl; and (f) any mixture thereof; wherein R₁ is a (C₁-C₄)alkyl.

Non-limiting examples of straight-chain and branched-chain (C₁-C₄)alkyls substituted with one or more -OH, -OR₁, -OC(O)R₁, -C(O)OR₁, -halo, or -CN include methanol, ethanol, n-propanol, isopropanol, n-butanol, isobutanol, tert-butanol, chloromethane, methylene chloride, chloroform, carbon tetrachloride, diethyl ether, di-isopropyl ether, tert-butyl methyl ether, acetonitrile, methyl formate, ethyl formate, methyl acetate, ethyl acetate, isopropyl acetate, butyl acetate, and mixtures thereof.

Non-limiting examples of straight-chain and branched-chain (C₄-C₁₀)aliphatic hydrocarbons include butane, pentane, hexane, heptane, isooctane, nonane, decane, and mixtures thereof.

Non-limiting examples of (C₅-C₇)cycloaliphatic hydrocarbons optionally substituted with one or more -R₁ include cyclopentane, cyclohexane, methylcyclohexane, cycloheptane, and mixtures thereof.

Non-limiting examples of (C₄-C₇)cyclic ethers optionally substituted with one or more -R₁ include tetrahydrofuran, methyltetrahydrofuran, 1,4-dioxane, 1,3-dioxolane, and mixtures thereof.

Non-limiting examples of aromatic hydrocarbons optionally substituted with one or more -R₁, -halo, -CH₂(halo), -CH(halo)₂, -C(halo)₃ -O(C₁-C₆)alkyl include toluene, xylene, chlorobenzene, benzotrifluoride, and mixtures thereof.

In one embodiment, the eluting solvent can comprise a mixture of an aliphatic hydrocarbon and an alcohol. In one embodiment, the eluting solvent can comprise a mixture of n-heptane and iso-propanol. In a specific embodiment, the organic solvent comprises a 95:5 (v/v) mixture of n-heptane:2-propanol.

Eluents that contain trans-(-)-Δ⁹-THC and that are substantially free of other cannabinoids can be combined. In one embodiment, the eluents can comprise at least 98% by weight, at least 99% by weight, at least 99.5% by weight, or at least 99.9% by weight oftrans-(-)-Δ⁹-THC, based on the total amount of trans-(-)-Δ⁹-THC and trans-(+)-Δ⁹-THC, in the combined eluents.

Similarly, eluents that contain trans-(+)-Δ⁹-THC and that are substantially free of other cannabinoids can be combined. In one embodiment, the eluents can comprise at least 98% by weight, at least 99% by weight, at least 99.5% by weight, or at least 99.9% by weight oftrans-(+)-Δ⁹-THC, based on the total amount of trans-(+)-Δ⁹-THC and trans-(-)-Δ⁹-THC in the combined eluents.

Optionally, the eluents, which comprise a first solvent and trans-(-)-Δ⁹-THC or trans-(+)-Δ⁹-THC, can be separated from the volatiles to provide each enantiomer as an oil. Methods for separating the trans-(-)-Δ⁹-THC or trans-(+)-Δ⁹-THC from volatile components include, *e.g*., distillation at atmospheric pressure or reduced pressure. For example, the trans-(-)-Δ⁹-THC or trans-(+)-Δ⁹-THC can, if desired, be distilled by fractional distillation to provide a trans-(-)-Δ⁹-THC or trans-(+)-Δ⁹-THC distillate *(see* U.S. Patent No. 4,381,399 to Olsen et al.).

As noted above, trans-(+)-Δ⁹-THC, together with trans-(-)-Δ⁹-THC, can be useful for making crystalline trans-(±)-Δ⁹-THC, where the trans-(-)-Δ⁹-THC and/or trans-(+)-Δ⁹-THC compositions can be made by methods described above.

### 5.7 Formulation of Crystalline trans-(±)-Δ⁹-THC

As noted above, pure trans-(-)-Δ⁹-tetrahydrocannabinol is a thick, viscous material that is difficult to formulate. Moreover, since trans-(-)-Δ⁹-tetrahydrocannabinol is sensitive to oxygen and light, compositions comprising trans-(-)-Δ⁹-tetrahydrocannabinol that have been disclosed in the art are inherently unstable; they generally must be stored at low temperature, protected from light and air, and they tend to have a relatively short shelf-life. These properties of trans-(-)-Δ⁹-tetrahydrocannabinol have essentially precluded the formulation of practical controlled-release materials that would enable sustained release of trans-(-)-Δ⁹-tetrahydrocannabinol. For the same reasons, it has not been possible to provide effective formulations that would be suitable for administration of trans-(-)-Δ⁹-tetrahydrocannabinol by inhalation.

Due to the sensitivity and/or instability of the trans-(-)-Δ⁹ THC in the presence of light, heat, oxygen and combinations thereof, precautions are necessary to prevent or minimize the loss of material. In some cases failure to follow this precautions, may lead to decomposition of the material and thus of the resulting formulation.

In contrast the compositions according to the invention, comprising crystalline trans-(±)-Δ⁹ THC, preferably at least 95%, more preferably 98%, even more preferably 99%, even more preferably 99.5% and most preferably 99.9% by weight of trans-(±)-Δ⁹ THC, do not show any sensitivity against light, heat and therefore do not require special handling. The compositions according to the present invention comprising trans-(±)-Δ⁹ THC are stable under ambient conditions for weeks, preferably for months and even more preferably for 1 year and most preferably for 1-3 years without any substantial decomposition of the composition. In addition, within the mentioned time period of weeks, preferably months and even more preferably 1 year and most preferably 1-3 years no loss of titer is observed. Further, in contrast to the trans-(-)-Δ⁹ THC compositions, the compositions according to the present invention do not require any special storage conditions.

Without being bound to any specific theory, it is assumed that the dosage forms according to the present invention comprising the trans-(±)-Δ⁹ THC in the above-mentioned purity, exhibit a longer shelf-life.

In contrast, the crystalline trans-(±)-Δ⁹-THC of the present invention is a highly-purified, crystalline, solid material that will be more stable than the pure trans-(-)-Δ⁹-THC enantiomer. Accordingly, the crystalline trans-(±)-Δ⁹-THC of the present invention is amenable to formulation according to methods disclosed in the art for use with solid active pharmaceutical ingredients, including those disclosed in the art cited in Section 2, above. Described below are illustrative, non-limiting, examples of formulations that can be prepared using the crystalline trans-(±)-Δ⁹-THC of the present invention.

In one embodiment, the crystalline trans-(±)-Δ⁹-THC of the present invention can be granulated and/or micronized to provide free-flowing powders, microparticles and nanoparticles useful for the formulation of pharmaceutical compositions that can be administered to a patient in need of such treatment. Microparticulates comprising crystalline trans-(±)-Δ⁹-THC are suitable for inclusion in solid dosage forms such as tablets, capsules, dry powder inhalers, and the like. As used herein, the term "particle" is used broadly to refer to granules, particles, and spheres that have sizes on the order of microns or on the order of nanometers. Accordingly, unless context dictates otherwise, the terms "particle," "microparticle," and "nanoparticle" are used interchangeably. The particles comprising crystalline trans-(±)-Δ⁹-THC can be formed in a size range consistent with the intended properties such as, e.g. the release rate of the active agent from the pharmaceutical dosage form being prepared. Crystalline trans-(±)-Δ⁹-THC of the present invention can be micronized to produce particles in a size range of about 0.1 to about 10 microns.

For example, crystalline trans-(±)-Δ⁹-THC of the present invention can be micronized in a suitable mill, e.g. a jet mill, to produce particles in a size range of about 10 microns. In one approach, crystalline trans-(±)-Δ⁹-THC can be micronized separately from other pharmaceutically-acceptable carriers or excipients included in the formulation. In another approach, one or more of the pharmaceutically-acceptable carriers or excipients included in the formulation can be combined with crystalline trans-(±)-Δ⁹-THC prior to micronization. Such pharmaceutically-acceptable carriers or excipients are known in the art. These include desiccants, diluents, glidants, binders, colorants, preservatives, lubricants, disintegration agents, filling agents, surfactants, buffers and stabilizers *(see e.g.* Remington's, The Science and Practice of Pharmacy (2000); Lieberman, H. A. and Lachman, L., Eds., Pharmaceutical Dosage Forms, Marcel Decker, New York, N.Y., 1980; and Liebeman et al., Pharmaceutical Dosage Forms (Volumes 1-3, 1990)). Micronization of crystalline trans-(±)-Δ⁹-THC, optionally in the presence of one or more pharmaceutically-acceptable carriers or excipients can be advantageous in that the particles so produced comprise a more uniform distribution and content of the active pharmaceutical agent within those particles. It is believed that such particles can provide a more consistent release profile and improved bioavailablity for the finished dosage form into which they are incorporated. For example, such particulate material, e.g. granules, microparticles or nanoparticles comprising the crystalline trans-(±)-Δ⁹-THC of the present invention can be compressed to form tablets or can be distributed to capsules for administration.

Where appropriate, the micronized powder comprising the crystalline trans-(±)-Δ⁹-THC can be further processed to improve the flow properties of the material according to the route of administration, *e.g.* when using a dry powder inhaler. Specific approaches for the formation of microparticles and nanoparticles comprising an active pharmaceutical ingredient are known in the art and include spray drying, milling, fluid energy grinding, microfluidization *(see e.g.* U.S. Patent No. 6,555,139 B2), lyophilization, and melt-extrusion *(see e.g.* U.S. Patent No. 6,706,281 B1).

Microfluidization, for example, can be used to produce particles comprising crystalline trans-(±)-Δ⁹-THC, which particles can exhibit controlled dissolution rates and more consistent drug release properties. Compositions comprising crystalline trans-(±)-Δ⁹-THC can be processed in a microfluidizer in which shear forces reduce the particle size. Moreover, the product can be recycled into the microfluidizer to obtain smaller particles (*see* U.S. Patent No. 6,555,139 B2). In certain embodiments, such particles can have a substantially uniform size distribution that generally falls within a size range of from about 1 micron to about 30 microns, from about 1 micron to about 20 microns, from about 1 micron to about 10 microns, or from about 1 micron to about 5 microns.

Other methods for the production of small particles falling within a defined size range can be based upon the use of supercritical fluids. For example crystalline trans-(±)-Δ⁹-THC can first be solubilized in supercritical CO₂ and then sprayed through a nozzle into a low-pressure gaseous medium. Expansion of the solution as it passes through the nozzle causes a reduction in CO₂ density, leading to recrystallization of the solid in the form of fine particles. In an alternative approach, crystalline trans-(±)-Δ⁹-THC can be dissolved in a solvent (such as ethanol or hexane) to provide a solution that is then introduced into the supercritical fluid using a nozzle. Upon dissolution of the solvent in the supercritical fluid, crystalline trans-(±)-Δ⁹-THC would be expected to precipitate out in the form of very small particles, e.g. nano-particles *(see e.g.* U.S. Patent No. 6,620,351 B2).

In one embodiment, the crystalline trans-(±)-Δ⁹-THC of the present invention can be formulated for oral administration. In one aspect of this embodiment, the present invention provides an orally administrable, immediate-release formulation of crystalline trans-(±)-Δ⁹-THC. In another aspect of this embodiment, the present invention provides an orally-administrable, controlled-release formulation of crystalline trans-(±)-Δ⁹-THC suitable for once-a-day or twice-a-day administration. In one embodiment, the orally-administrable, controlled-release crystalline trans-(±)-Δ⁹-THC formulation provides an early onset of therapeutic effect and, after rising to a maximum concentration during the dosage interval, provides a relatively flat serum plasma profile. That is, the plasma level of the cannabinoid active pharmaceutical ingredient provides a C*₂₄*/C*ₘₐₓ* ratio of about 0.55 to about 1.0, and provides effective relief to the patient. In certain embodiments, the dosage form provides a C*₂₄* /C*ₘₐₓ* ratio of from about 0.55 to about 1.0; or from about 0.55 to about 0.85; or from about 0.55 to 0.75; or from about 0.60 to about 0.70.

In certain embodiments, the controlled-release oral dosage form of the present invention can comprise a matrix which includes a sustained-release material and crystalline trans-(±)-Δ⁹-THC. In certain embodiments, the matrix can be compressed into a tablet and may, optionally, be overcoated with a coating that, in addition to the sustained release material of the matrix, may control release of the crystalline trans-(±)-Δ⁹-THC from the formulation, such that blood levels of API are maintained within a therapeutic range over an extended period of time. That therapeutic range is, preferably, below that required to induce psychotropic effects.

In certain embodiments, the controlled-release oral dosage form of the present invention can be an osmotic dosage form which comprises a single layer or bilayer core comprising crystalline trans-(±)-Δ⁹-THC; an expandable polymer; a semipermeable membrane surrounding the core; and a passageway disposed in the semipermeable membrane for sustained release of the crystalline trans-(±)-Δ⁹-THC, such that blood levels of API can be maintained within a therapeutic range over an extended period of time when administered to a patient. The controlled-release dosage forms of the present invention may be "cannabinoid-sparing." For example, it is possible that the controlled-release oral dosage form may be dosed at a substantially lower daily dosage in comparison to conventional immediate-release products, with no difference in therapeutic efficacy. At comparable daily dosages, greater efficacy may result with the use of the controlled-release oral dosage form of the present invention in comparison to conventional immediate-release products.

Controlled-release formulations of crystalline trans-(±)-Δ⁹-THC can be provided by adapting any of a wide variety of controlled-release formulations known in the art in view of the instant disclosure. For example, the materials and methods disclosed in U.S. Patent No. 4,861,598, U.S. Patent No. 4,970,075, U.S. Patent No. 5,958,452, and U.S. Patent No. 5,965,161 (each of which is hereby incorporated by reference) can be adapted to prepare dosage forms useful according to the present invention.

Dosage forms of the present invention may further include one or more additional (or second) active pharmaceutical ingredients that may or may not act synergistically with the cannabinoid API of the present invention. If present, the additional API can be included in controlled-release form or in immediate-release form. The additional API can be an opioid agonist, a non-opioid analgesic, a non-steroidal antiinflammatory agent, an antimigraine agent, a Cox-II inhibitor, a β-adrenergic blocker, an anticonvulsant, an antidepressant, an anticancer agent, an agent for treating addictive disorder, an agent for treating Parkinson's disease and parkinsonism, an agent for treating anxiety, an agent for treating epilepsy, an agent for treating a seizure, an agent for treating a stroke, an agent for treating a pruritic condition, an agent for treating psychosis, an agent for treating ALS, an agent for treating a cognitive disorder, an agent for treating a migraine, an agent for treating vomiting, an agent for treating dyskinesia, or an agent for treating depression, or a mixture thereof.

In one non-limiting embodiment, the additional API is an opioid compound. Examples of useful opioid agonists include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof.

In certain embodiments, the opioid agonist is selected from codeine, hydromorphone, hydrocodone, oxycodone, dihydrocodeine, dihydromorphine, morphine, tramadol, oxymorphone, pharmaceutically acceptable salts thereof, and mixtures thereof.

Examples of useful non-opioid analgesics include non-steroidal anti-inflammatory agents, such as aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, and pharmaceutically acceptable salts thereof, and mixtures thereof. Examples of other suitable non-opioid analgesics include the following, non limiting, chemical classes of analgesic, antipyretic, nonsteroidal antiinflammatory drugs: salicylic acid derivatives, including aspirin, sodium salicylate, choline magnesium trisalicylate, salsalate, diflunisal, salicylsalicylic acid, sulfasalazine, and olsalazin; para aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone. For a more detailed description of the NSAIDs, see Paul A. Insel, Analgesic Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti Inflammatory Drugs in Remington: The Science and Practice of Pharmacy Vol II 1196-1221 (A.R. Gennaro ed. 19th ed. 1995) which are hereby incorporated by reference in their entireties. Suitable Cox-II inhibitors and 5-lipoxygenase inhibitors, as well as combinations thereof, are described in U.S. Patent No. 6,136,839, which is hereby incorporated by reference in its entirety. Examples of useful Cox II inhibitors include, but are not limited to, rofecoxib and celecoxib.

In another non-limiting aspect of this embodiment, co-administration of crystalline trans-(±)-Δ⁹-THC of the invention and the additional API enhances the antinociceptive potency of either the crystalline trans-(±)-Δ⁹-THC of the invention or of the additional API. Accordingly, equivalent analgesia may be obtained by using a lower dose of either or both components, when administered in combination.

In a particular embodiment, a dosage form or formulation of the present invention comprises the crystalline trans-(±)-Δ⁹-THC of the present invention and the additional API. In one aspect of this embodiment, the crystalline trans-(±)-Δ⁹-THC and the additional API are combined in a formulation that is adapted, *e.g.,* for parenteral, transdermal, or transmucosal administration, and which may be a controlled-release formulation. In a further aspect, the formulation is contained disposed within a patch adapted for transdermal delivery of the crystalline trans-(±)-Δ⁹-THC and the additional API. In a still further aspect of this embodiment, the formulation comprises an aqueous solution prepared with the crystalline trains-(±)-Δ⁹-THC of the present invention and the additional API. Formulations and methods, including controlled-release formulations and methods, suitable for administration of a combination of the crystalline trans-(±)-Δ⁹-THC of the present invention and the additional API are described herein.

In certain embodiments, the cannabinoid API of the present invention can be combined with a selective antagonist of the CB1 receptor in order to provide substantially CB2-specific pharmacological and/or therapeutic effects to a patient to whom the combination is administered. Similarly, in other embodiments, the cannabinoid API of the present invention can be combined with a selective antagonist of the CB2 receptor in order to provide substantially CB1-specific pharmacological and/or therapeutic effects to a patient to whom the combination is administered. Illustrative, non-limiting, examples of selective antagonists of cannabinoid receptors include the CB1 receptor antagonist SR 141716 A (*see e.g.* Shire et al. (1996) J. Biol. Chem. 271(12): 6941-46), and the CB2 receptor antagonist SR 144528 *(see e.g.* Shire et al. (1998) J. Pharmacol. Exp. Ther. 284(2): 644-50).

A controlled-release dosage form comprising crystalline trans-(±)-Δ⁹-THC can comprise a controlled-release material incorporated into a matrix along with crystalline trans-(±)-Δ⁹-THC. Alternatively or additionally, the controlled release material can be applied as a coating over a substrate core comprising the API (wherein the term "substrate" encompasses beads, pellets, particles, tablets, tablet cores, and the like). The controlled-release material may be hydrophobic or hydrophilic as appropriate.

An oral dosage form according to the present invention may be provided as, for example, as granules, microparticles, nanoparticles or other multiparticulate formulations known in the art. An amount of multiparticulates effective to provide the desired dose of crystalline trans-(±)-Δ⁹-THC over time can be disposed within a capsule, or may be incorporated in any other suitable oral solid form such as, *e.g*., by compression into a tablet. An oral dosage form according to the present invention may be prepared as a tablet core coated with a controlled-release coating, or as a tablet comprising a matrix of crystalline trans-(±)-Δ⁹-THC and controlled-release material, and optionally other pharmaceutically-desirable ingredients (*e.g*., diluents, binders, colorants, lubricants, etc.). A controlled-release dosage form of the present invention may alternatively be prepared as a bead formulation or as an osmotic dosage formulation.

In certain embodiments of the present invention, the controlled-release formulation can be achieved by use of a matrix (e.g. a matrix tablet) that includes a controlled-release aspect. The matrix may be a hydrophilic or a hydrophobic controlled-release material. The matrix may also include a binder. In such an embodiment, the binder can contribute to the controlled-release aspect of the matrix. The matrix may further comprise one or more diluents, lubricants, granulating aids, colorants, flavorants, glidants, or mixtures thereof, that are conventional in the pharmaceutical art.

Optionally, the controlled-release matrix, multiparticulates, or tablet can be coated, or the gelatin capsule comprising the API-containing particles can be further coated with a controlled-release coating. Such coatings preferably include a sufficient amount of a hydrophobic or hydrophilic controlled-release material to obtain a weight gain level from about 2 to about 25 percent, although the overcoat may be greater depending upon the desired release rate.

Controlled-release formulations of the present invention preferably release the API slowly upon ingestion and exposure to gastric fluids and then to intestinal fluids. The controlled-release profile of a formulation of the present invention can be altered using standard methodologies known in the art.

As noted above, controlled-release dosage forms according to the present invention may be prepared as osmotic dosage formulations. Such an osmotic dosage form can include a bilayer core comprising a drug layer comprising crystalline trans-(±)-Δ⁹-THC and a delivery, or push, layer, wherein the bilayer core is surrounded by a semipermeable wall and optionally having at least one passageway disposed therein. In certain embodiments, the bilayer core can comprise a crystalline trans-(±)-Δ⁹-THC-containing layer and a push layer. In certain embodiments, the drug layer may comprise at least one polymer hydrogel. In certain embodiments of the present invention, the delivery or push layer can comprise an osmopolymer, which drives the crystalline trans-(±)-ΔA⁹-THC from the osmotic dosage form. The push layer may also include one or more osmotically effective compounds that are referred to as osmagents or osmotically-effective solutes. Such compounds imbibe an environmental fluid, for example, from the gastrointestinal tract, into the dosage form and contribute to the delivery kinetics of the displacement layer.

Dosage forms of the present invention may optionally be coated with one or more coatings suitable for the regulation of release, or for the protection of, the formulation. In one embodiment, a coating can be provided to permit either pH-dependent or pH-independent release. In embodiments of the present invention where the coating comprises an aqueous dispersion of a hydrophobic controlled-release material, inclusion of an effective amount of a plasticizer in the aqueous dispersion of hydrophobic material will further improve the physical properties of the controlled-release coating.

Non-limiting examples of suitable controlled-release materials, binders, diluents, lubricants, binders, granulating aids, colorants, flavorants, glidants, controlled-release coating materials, coated bead controlled-release formulations, controlled-release osmotic dosages, osmopolymers, osmotically active compounds, and plasticizers are provided in U.S. Patent No. 6,733,783 B1 (which is hereby incorporated by reference in its entirety). Additionally, specific examples of pharmaceutically-acceptable carriers and excipients that may be used to formulate dosage forms of the present invention are described in the Handbook of Pharmaceutical Excipients, American Pharmaceutical Association (1986), incorporated by reference herein.

Although pharmacologically-active compounds are most commonly administered by the oral route, oral administration of the cannabinoid API of the present invention could be contraindicated in certain instances such as, e.g., for patients already suffering from nausea and/or emesis. In addition, the onset of pharmacological activity is expected to be less rapid with an orally-administered compound due to first-pass metabolism in the liver. Consequently, in another embodiment, the crystalline trans-(±)-Δ⁹-THC of the present invention can be administered by inhalation, using *e.g.* preferably a mechanical device such as a powder inhaler, a unit-dose inhaler, a metered-dose inhaler, a nebulizer, or a pump spray. In one aspect of this embodiment, crystalline trans-(±)-Δ⁹-THC can be dissolved in a pharmaceutically-acceptable solvent (e.g. ethanol) and administered to a patient using an inhalation device such as that described in U.S. Patent No. 5,497,944. In certain embodiments, the crystalline trans-(±)-Δ⁹-THC is admixed with the pharmaceutically-acceptable solvent at the time the API of the present invention is administered to the patient.

In another aspect of this embodiment, the crystalline trans-(±)-Δ⁹-THC of the present invention can be formulated as a powder for administration by inhalation. Inhalation delivery systems that may be useful for pulmonary administration of the cannabinoid API of the present invention as a powder include, but are not limited to, those of U.S. Patent No. 6,814,072, which describes a metered-dose inhaler that would be capable of delivering a pre-determined amount of a powdered pharmaceutical formulation comprising crystalline trans-(±)-Δ⁹-THC in view of the present disclosure; U.S. Patent No. 6,642,275 B2, which describes a number of such devices that are suitable for administration of powdered pharmaceutical formulations; and U.S. Patent Application Publication No. 2004/0069798, which is directed toward a remote-controlled dispensing device comprising a locking mechanism designed for the administration of controlled substances.

In another embodiment, granules, particles, microparticles or nanoparticles comprising the API of the present invention can be used to prepare a suspension, emulsion, or gel useful for transmucosal administration. Such pharmaceutical formulations can be advantageous in that crystalline trans-(±)-Δ⁹-THC administered by this route can avoid first-pass liver metabolism. In a specific aspect of this embodiment, crystalline trans-(±)-Δ⁹-THC-containing particles can be combined with an appropriate material, e.g. a hydrogel, that can form an emulsion capable of adhering to a mucosal surface. In specific, non-limiting aspects of this embodiment, the pharmaceutical composition can be formed as a solid gel and shaped e.g. as a pastille, a compressed tablet, a lozenge, a capsule, or incorporated into a gel spray that, upon contact with the mucosa, can form an emulsion that will adhere to the tissue *(see e.g.* U.S. Patent No. 6,642,275 B2 for additional pharmaceutical formulations that can be adapted for transmucosal administration of crystalline trans-(±)-Δ⁹-THC). In other non-limiting aspects of this embodiment, such as e.g. those in which the final pharmaceutical formulation is prepared at the time it is to be administered to the patient, crystalline trans-(±)-Δ⁹-THC-containing pharmaceutical formulations useful for transmucosal administration of the active agent may further comprise a solvent (e.g. a C₁ to C₄ alcohol such as ethanol), and a co-solvent which acts as a solubility enhancer (e.g. propylene glycol or glycerol).

In yet another embodiment, crystalline trans-(±)-Δ⁹-THC of the present invention can be formulated to provide a composition useful for transdermal administration. A "transdermal dosage form" of the present invention encompasses any device that when contacted with a patient's skin can deliver an effective amount of the API through the skin of the patient. In a specific, non-limiting aspect of this embodiment, the transdermal dosage form can be a diffusion-driven transdermal system (e.g. in the form of a patch) that comprises a drug-in-adhesive matrix system. Other transdermal dosage forms can include, but are not limited to, topical gels, lotions, ointments, transmucosal systems and devices, and iontophoretic (electrical-diffusion) delivery systems (*see e.g.,* U.S. Patent No. 4,626,539 to Aungst et al.; U.S. Patent No. 4,806,341 to Chien et al.; U.S. Patent No. 5,069,909; and U.S. Patent Application Publication No. 2004/0126323).

Compositions comprising crystalline trans-(±)-Δ⁹-THC as the API that may be formulated for use in transdermal administration may further comprise one or more permeation enhancers. Permeation enhancers are intended to facilitate transfer of the API across the skin and into the circulatory system of the patient. Non-limiting examples of such permeation enhancers are disclosed in U.S. Patent No. 4,783,450, U.S. Patent No: 3,989,816, U.S. Patent No. 4,316,893, U.S. Patent No. 4,405,616, U.S. Patent No. 4,557,934, and U.S. Patent No. 4,568,343, each of which is hereby incorporated by reference. Other permeation enhancers that may be useful in this embodiment are disclosed in: Percutaneous Penetration Enhancers, eds. Smith et al. (CRC Press, 1995).

Without having tried, it is assumed that formulations disclosed in US 6,713,048, US 6,509005, US 6,995,187, US 6,943,266, US 6,900,236, US 6,939,977, US 6,132,762, US 6,903,137, US 6,864,291, US 6,355,650, US 6,162,829, US 5,932,557, and US 5,338,753, which are enclosed herewith in their entirety, as well as others described in the art, are suitable formulations for the compositions according to the present invention.

### 5.8 Therapeutic/Prophylactic Administration of Compositions Comprising trans-(±)-Δ⁹-THC

The pharmaceutical compositions of the present invention comprising crystalline trans-(±)-Δ⁹-THC are useful for treating or preventing the same diseases, ailments, disorders or symptoms (i.e., the "Conditions") for which trans-(-)-Δ⁹-THC is known to be useful, or for any Condition for which trans-(-)-Δ⁹-THC may later be found to be useful for treatment or prevention. For example, a pharmaceutical composition of the present invention can be used for treating or preventing emesis, loss of weight, loss of appetite, multiple sclerosis, Tourette's syndrome, Parkinson's disease, or palsies such as cerebral palsy.

In one embodiment, a pharmaceutical composition of the present invention can be used to treat or prevent pain.

In another embodiment, a pharmaceutical composition of the present invention can be used to treat or prevent emesis, e.g., as the result of cancer chemotherapy.

In another embodiment, a pharmaceutical composition of the present invention can be used to treat or prevent loss of appetite.

In another embodiment, a pharmaceutical composition of the present invention can be used to treat or prevent weight loss, e.g., as the result of symptomatic HIV infection including acquired immunodeficiency syndrome (AIDS) or AIDS related complex (ARC).

In another embodiment, a pharmaceutical composition of the present invention can be used to treat or prevent a Condition selected from the group consisting of glaucoma, neuralgia, somatic pain, chronic pain, neuropathic pain, labor pain, inflammation, muscle spasticity (such as that associated with spinal cord injury or multiple sclerosis), movement disorders (such as that associated with dystonia, Parkinson's disease, Huntington's disease, or Tourette's syndrome), migraine headache, epilepsy, and Alzheimer's disease.

In another embodiment, a pharmaceutical composition of the present invention can be used to treat or prevent a Condition associated with neurological trauma or stroke.

In another embodiment, a composition of the present invention may further demonstrate beneficial activity at one or more N-methyl-D-aspartate (NMDA) receptor subtypes. Accordingly, a composition of the present invention may be useful to treat or prevent one or more NMDA-associated indications when administered at appropriate therapeutically effective levels. More specifically, a composition of the present invention may be useful to treat or prevent neuronal loss, a neurodegenerative disease, or may be useful as an anticonvulsant or for inducing anesthesia, or for treating epilepsy or psychosis. Neurodegenerative diseases which may be treated with a composition of the present invention may include those selected from the group consisting of Alzheimer's disease, amyotrophic lateral sclerosis, Huntington's disease, Parkinson's disease and Down's syndrome. A composition of the present invention may also find particular utility in the treatment or prevention of neuronal loss associated with multiple strokes which give rise to dementia. After a patient has been diagnosed as suffering from a stroke, a composition of the present invention may be administered to ameliorate the immediate ischemia and prevent further neuronal damage that may occur from recurrent strokes. In addition, a compound of the present invention may find particular utility in treating or preventing one or more adverse neurological consequences of surgery, such as coronary bypass surgery or carotid endarterectomy surgery.

In a further embodiment, a pharmaceutical composition of the present invention can be used to treat or prevent atherosclerosis or a Condition associated with atherosclerosis.

The present invention provides methods for treating or preventing any of the aforementioned Conditions, comprising administering to a patient in need thereof an effective amount of a pharmaceutical composition of the present invention. In certain embodiments, the crystalline trans-(±)-Δ⁹-THC present in the pharmaceutical composition comprises at least 95.0% by weight, at least 98.0% by weight, at least 99% by weight, at least 99.5%, or at least 99.9% by weight of crystalline trans-(±)-Δ⁹-THC based on the total amount of cannabinoids in the composition. In certain aspects of these embodiments, the crystalline trans-(±)-Δ⁹-THC composition comprises less then 0.05% of Δ⁹-THC carboxylic acid.

The present invention also provides a method for administering trans-(±)-Δ⁹-tetrahydrocannabinol to a patient in need thereof, which comprises admixing an effective amount of crystalline trans-(±)-Δ⁹-tetrahydrocannabinol and a pharmaceutically-acceptable carrier to provide a composition, and administering the composition to the patient. In one aspect of this embodiment, the composition is in the form of a solution, emulsion, gel, or suspension. In another aspect of this embodiment, the pharmaceutically-acceptable carrier is a solvent and the composition is a solution. In a further aspect of this embodiment, the step of admixing is carried out by the patient or by the attending medical practitioner. In certain embodiments, the administering step is carried out immediately upon admixing the crystalline trans-(±)-Δ⁹-tetrahydrocannabinol and a pharmaceutically-acceptable carrier to provide the composition.

The term "pharmaceutically acceptable" as it refers to a carrier or excipient, means that the carrier or excipient has been approved by a regulatory agency of the Federal or a state government or listed in the U.S. Pharmacopeia or other generally recognized pharmacopeia for use in animals, and more particularly in humans.

The term "carrier" (used interchangeably with "excipient") refers to one or more components selected from diluents, vehicles, binders, fillers, compression aids, disintegrants, lubricants, glidants, sweetners, coloring agents, flavoring agents, preservatives, suspension agents, dispersing agents, film formers, and coatings with which the crystalline trans-(±)-Δ⁹-THC is to be combined and administered to a subject. Such pharmaceutically-acceptable carriers can be solid or dry components. Alternatively, such pharmaceutically-acceptable carriers can be liquids, such as water or oils, including those of petroleum, animal, vegetable or synthetic origin, such as peanut oil, soybean oil, mineral oil, sesame oil, and the like in which the cannabinoid API of the present invention can be suspended. The pharmaceutical carrier can, e.g., be selected from saline, gum acacia, gelatin, starch paste, talc, keratin, colloidal silica, urea, and the like. In addition, auxiliary, stabilizing, thickening, lubricating and coloring agents may be used. The present compositions, if desired, can also contain minor amounts of wetting or emulsifying agents, pH buffering agents, anti-oxidant or other stabilizer, etc. When administered to a patient, the pharmaceutical composition is preferably sterile.

The present composition can take the form of a suspension, tablet, pill, pellet, suppository, or capsule, (e.g., a capsule containing a powder, microparticles, multiparticulates, or nanoparticles) form comprising the API of the present invention, or any other form suitable for use. Any of the present compositions can be prepared as a controlled-release formulation. In other specific, non-limiting embodiments of the present invention in which the composition in its final form can be prepared at the time it is to be administered to the patient, and preferably can take the form of a solution, emulsion, aerosol, spray, or liquid-filled capsule.

The API-containing composition of the present invention can be administered systemically or locally by any convenient route. Methods of administration include but are not limited to pulmonary, intradermal, intramuscular, intraperitoneal, intravenous, subcutaneous, oral, sublingual, intranasal, intrathecal, epidural, intracerebral, intravaginal, transdermal, topical (e.g. to the ears, nose, eyes, or skin), transmucosal, or rectal administration. One mode of administration is oral administration. Another mode of administration is pulmonary administration (e.g. by inhalation). Another mode of administration is transmucosal administration, e.g., by absorption through epithelial or mucocutaneous linings (e.g., oral mucosa, rectal and intestinal mucosa, etc.). Another mode of administration is by infusion or bolus injection. Various delivery systems are known, e.g., encapsulation in liposomes, microparticles, microcapsules, capsules, etc., and any of these can be used to administer the pharmaceutical compositions of the present invention. Other useful modes of administration can be left to the discretion of the practitioner.

When used for oral delivery, the cannabinoid API-containing composition of the present invention can be in the form, for example, of tablets, lozenges, aqueous or oily suspensions, granules, powders, emulsions, capsules, syrups, or elixirs. Orally-administered compositions can contain one or more optional agents such as, for example, sweetening agents (such as fructose, aspartame or saccharin); flavoring agents (such as peppermint, oil of wintergreen, or cherry); coloring agents; and preserving agents, to provide a pharmaceutically palatable preparation. Moreover, tablets or pills can be coated to delay disintegration and absorption in the gastrointestinal tract, thereby providing a sustained action over an extended period of time. Selectively-permeable membranes surrounding an osmotically active driving compound are also suitable for orally administered pharmaceutical compositions. In these latter platforms, fluid from the environment surrounding the capsule is imbibed by the driving compound, which swells to displace the agent or agent composition through an aperture. These delivery platforms can provide an essentially zero-order delivery profile as opposed to the spiked profiles of immediate release formulations. A time-delay material such as glycerol monostearate or glycerol stearate may also be used. Oral compositions can include standard carriers such as, e.g., mannitol, lactose, starch, magnesium stearate, sodium saccharine, cellulose, or magnesium carbonate. Such carriers are preferably of pharmaceutical grade.

When adapted for parenteral delivery, the API-containing composition of the present invention can be formulated in accordance with routine procedures for administration to human patients. Preferably, a pharmaceutical composition for parenteral administration is formulated as a solution or suspension in sterile isotonic aqueous buffer, optionally with a solubilizing agent. In a specific, non-limiting aspect of this embodiment, preparation of the final form pharmaceutical composition for parenteral administration is carried out at the time it is to be administered. Compositions for parenteral administration can optionally include a local anesthetic such as lignocaine to ease pain at the site of the injection. Generally, the ingredients are supplied either separately or mixed together in unit dosage form such as, for example, as a dry lyophilized powder or as a water-free concentrate in a hermetically-sealed container, such as an ampoule or sachette indicating the quantity of the cannabinoid active ingredient of the present invention. Where the pharmaceutical composition is to be administered by infusion, it can be dispensed with an infusion bottle containing sterile pharmaceutical grade water or saline, optionally with a solublizing agent. Where the pharmaceutical composition is administered by injection, an ampoule of sterile water for injection or saline can be provided so that the ingredients can be mixed prior to administration.

In one embodiment, the API-containing composition of the present invention is formed as a tablet.

In another embodiment, the API-containing composition of the present invention is encapsulated. In one embodiment, the encapsulated API-containing composition further comprises sesame oil (*see*, *e.g.,* U.S. Patent No. 6,703,418 B2).

The amount of the API-containing composition that is effective in the treatment or prevention of a Condition can be determined by standard clinical techniques. In addition, *in vitro* or *in vivo* assays can be employed to help identify optimal dosage amounts. The precise dose to be employed will generally depend on the route of administration and the seriousness of the Condition, and can generally be decided according to the judgment of a practitioner and/or each patient's circumstances, particularly in view of published clinical trials. When an immediate-release formulation of the API-containing composition is administered orally, the effective dosage amount ranges from about 0.01 mg/kg of body weight to about 0.8 mg/kg of body weight about every 4 hours, although it is typically preferably about 0.2 mg/kg of body weight or less about every 4 hours. Where the API-containing composition is to be administered e.g. only once every 8 hours, every 12 hours, or every 24 hours, the effective dosage ranges can be proportionately greater than those disclosed for administration every 4 hours. In one embodiment, the effective dosage amount can be from about 0.01 mg/kg of body weight to about 0.8 mg/kg of body weight, preferably from about 0.02 mg/kg of body weight to about 0.2 mg/kg of body weight, or more preferably from about 0.02 mg/kg of body weight to about 0.150 mg/kg of body weight.

In other embodiments the dosage form, which can be an oral dosage form, can comprise an amount of crystalline trans-(±)-Δ⁹-THC from about 1 mg to about 200 mg, or preferably from about 1 mg to about 100 mg, or more preferably from about 1 mg to about 80 mg, or even more preferably from about 5 mg to about 20 mg. In other embodiments, the oral dosage form can comprise about 2 mg, about 5 mg, about 10 mg, about 20 mg, about 40 mg, about 80 mg, about 100 mg, or about 200 mg of the API of the present invention.

In one embodiment, an effective dosage amount is administered about every 24 hours until the Condition is satisfactorily abated. In other embodiments, an effective dosage amount is administered about every 12 hours, or about every 8 hours, or about every 6 hours, or about every 4 hours, until the Condition is satisfactorily abated.

In certain embodiments, it may be desirable to introduce the pharmaceutical composition directly into the central nervous system by a suitable route, such as by intraventricular or intrathecal administration. Intraventricular administration can be facilitated, e.g., by an intraventricular catheter attached to a reservoir, such as an Ommaya reservoir.

Pulmonary administration can be employed, e.g., by use of an inhaler or nebulizer in which the API of the present invention is formulated with an aerosolizing agent, or with a fluorocarbon or synthetic pulmonary surfactant.

In certain embodiments, a pharmaceutical composition of the present invention can be formulated as a suppository, with traditional binders and carriers such as triglycerides.

In another embodiment, the API composition of the present invention can be delivered in a vesicle, such as a liposome (*see* Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.).

In yet another embodiment, the API-containing composition can be delivered in a controlled-release system. In one embodiment, a pump can be used (*see* Langer, *supra*; Sefton, CRC Crit. Ref Biomed. Eng. 14:201 (1987); Buchwald et al., Surgery 88:507 (1980); Saudek et al., N. Engl. J. Med. 321:574 (1989)). In another embodiment, the appropriate polymeric materials can be used (*see* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23:61 (1983); *see also* Levy et al., Science 228:190 (1985); During et al., Ann. Neurol. 25:351 (1989); Howard et al., J. Neurosurg. 71:105 (1989)). In another embodiment, a controlled-release system comprising the API-containing composition can be placed in proximity to the tissue target, thus requiring only a fraction of the systemic dose (*see, e.g.,* Goodson, in Medical Applications of Controlled Release, supra, vol. 2, pp. 115-138 (1984)). Other controlled-release systems, such as those discussed in the review by Langer (Science 249:1527-1533 (1990)), can be used.

The present invention also provides pharmaceutical packs or kits comprising one or more containers filled with a crystalline trans-(±)-Δ⁹-THC-containing composition of the present invention. Optionally associated with such container(s) can be a notice in the form prescribed by a governmental agency regulating the manufacture, use or sale of pharmaceuticals or biological products, which notice reflects approval by the agency for the manufacture, use or sale for human administration of the API of that particular formulation.

The following examples are set forth to assist in understanding the invention and do not limit the invention described and claimed herein. Such variations of the invention, including the substitution of all equivalents now known or later developed, which would be within the purview of those skilled in the art, and changes in formulations or minor changes in experimental design, fall within the scope of the present invention.

### 6. Examples

Unless otherwise stated, all reactions can be carried out under an argon or nitrogen atmosphere.

Unless otherwise stated, the phrase "cold water," "cold hexane," or "cold heptane" means water, hexane, or heptane, respectively, at a temperature of from about 0° to about 5°C.

*Reagents and Solvents:* Unless otherwise stated, all reagents and solvents can be purchased from Aldrich Chemical Company and can be used without further purification.

*High Performance Liquid chromatography:* High performance liquid chromatography (HPLC) can be carried out under the following conditions, and the purity of the samples eluents can be calculated from the resultant area percentages. Standard HPLC can be carried out using a 3 µm C₁₈-stationary phase column (150 x 4.6 mm); a mobile phase of the following composition: THF (71 %), MeOH (24%) and water (5%) for 25 min, gradient to THF (71 %), MeOH (5%) and water (24%) in 10 min, and THF (71 %), MeOH (24%) and water (5%) for 10 min; a flow rate of 1 mL/min; and a UV detector at 228 nm.

Chiral HPLC method 1 can be carried out using a 20 µm Chiralpak AD 250 x 4.6 mm column; a mobile phase of heptane:isopropanol (95:5 (v:v)); a flow rate of 1 mL/min; and a UV detector at 228 nm. The concentration of the sample can be about 1 mg per 1 mL of heptane.

Chiral HPLC method 2 can be carried out using a 5 µm Chiralpak AD-H 250 x 4.6 mm (Diacel) column; a mobile phase of hexane:ethanol (95:5 (v:v)) for CBD and hexane:isopropanol (90:10 (v:v)) for Δ⁹-THC; a flow rate of 1 mL/min; and a UV detector at 228 nm. The concentration of the sample can be about 1 mg per 1 mL of hexane.

*Gas chromatography:* Gas chromatography (GC) can be carried out under the following conditions, and the purity of the eluents can be calculated from the resultant area percentages.

*Standard GC* can be carried using a HP-5 capillary column (length - 30 m, ID - 0.25 mm); a stationary phase of 5% diphenyl/95% dimethyl-polysiloxane (0.25 µm film); an injection temperature of 230°C; a detector/temperature (FID) of 270°C; and an oven temperature program using a hold at 100°C for 3 min, increasing to 240°C at 10°C per minute, holding at 240°C for 10 min, increasing to 270°C per min, and holding at 270°C for 10 min. The concentration of the GC sample can be about 1 mg per 1 mL of EtOH.

*Chiral GC* can be carried out in a manner similar to that described above for standard GC, except that an Alpha-DEX-120, 30 m x 0.25 mm column is used; the injection temperature is 250°C; and the oven temperature is 90°C (isothermal).

*Powder x-ray diffraction patterns:* Powder x-ray diffraction analysis was carried out by known methods using a PANALYTICAL (Philips) X'Pert Pro MPD x-ray powder diffraction system (CuK_{α} radiation, PW3050/60 goniometer, PW3011/20 proportional detector). The Bragg-Brentano scheme can be used for beam focusing.

*Nuclear Magnetic Resonance Spectroscopy*: Nuclear magnetic resonance (NMR) spectra can be recorded on a Bruker AM-200 (¹H at 200 MHz, ¹³C at 50 MHz) or a Bruker AM-400 (¹H at 400 MHz) instruments using CDCl₃ (unless otherwise stated) as a solvent. Chemical shifts can be expressed in δ (ppm) relative to internal TMS.

*Melting points*: Melting point determinations can be carried out in open capillary tubes using a Buchi B-545 capillary melting point apparatus or with a Mettler-Toledo FP-81 Melting point accessory with FP-900 processor. The melting points are uncorrected.

### 6.1 Example 1: Preparation of (-)-Δ⁸-THC

Crude (-)-Δ⁸-THC (**2a**) can be prepared in a manner similar to that described below for the preparation of crude (±)-Δ⁸-THC, except that (+)-*p*-mentha-2,8-dien-1-ol is used instead of (±)-*p*-mentha-2,8-dien-1-ol.

### 6.2 Example 2: Preparation of (+)-A⁸-THC

Crude (+)-Δ⁸-THC (**2b**) can be prepared in a manner similar to that described below for the preparation of crude (±)-Δ⁸-THC, except that (-)-*p*-mentha-2,8-dien-1-ol is used instead of (±)-*p*-mentha-2,8-dien-1-ol.

### 6.3 Example 3: Two-part Preparation of trans-(-)-Δ⁹-THC

*Synthesis of ()-CBD (**3a**)*: A solution of (+)-p-mentha-2,8-dien-1-ol in dichloromethane is added drop-wise over 1 hour to a stirred mixture of olivetol, zinc chloride, water and dichloromethane at 40°C. The mixture is stirred for an additional 30 minutes at 40°C. The mixture is cooled to 25°C, poured into ice water, and the resultant biphasic mixture stirred for 20 minutes at 0°C. The resultant organic phase can be collected and washed twice with cold water. The organic phase can be collected and concentrated under reduced pressure to provide a first residue. Analysis (GC) of the first residue may contain more than 50% (-)-CBD, as well as *abn*-CBD, olivetol and dialkylated olivetol.

The first residue can be dissolved in n-heptane, and the resultant solution can be admixed with an approximately equal volume of 10% sodium hydroxide solution. The resultant organic phase can be collected, washed with water, and concentrated under reduced pressure to provide an oily-brown second residue. Analysis (GC) of the second residue is expected to contain more than 60% (-)-CBD, as well as a considerably lower amount of dialkylated olivetol.

The second residue can be fractionally distilled (171°-178°C; 0.1 mm Hg) to provide a distillate, which is expected to contain more than 70% (-)-CBD.

The distillate can then be dissolved in heptane at 57°C and filtered. The resultant filtrate is then cooled to 0° to 5°C and seeded with powdered crystalline (-)-CBD (3a). The seeded solution can then be stirred at 0° to 5°C for 5 hours and then at -15° to -20°C for 48 hours. The resultant mixture can be filtered, and the resultant solids washed with cold heptane. The solids are then dried under reduced pressure at 40°C to provide (-)-CBD (**3a)** of greater than 95% purity. The structure of the (-)-CBD (3a) so produced can be confirmed by ¹H NMR spectroscopy.

*Synthesis of trans-()-*Δ*⁹-THC **(1a):*** A solution of crystallized (-)-CBD (**3a**) in anhydrous dichloromethane can be added drop-wise over 1 hour to a stirred solution of BF₃·Et₂O in anhydrous dichloromethane at -10°C under an Ar atmosphere. The mixture can then be stirred for 2 hours at -10°C and poured into ice water. The resultant biphasic mixture is then further stirred for 20 minutes at 0°C. The resultant organic phase can be collected, washed sequentially with cold water, 7 % aqueous sodium bicarbonate, and water. The organic phase can then be dried with Na₂SO₄ and filtered. The resultant filtrate can be concentrated under reduced pressure at 40°C and is expected to provide trans-(-)-Δ⁹-THC **(1a)** as a yellow oil having a purity of about 80%.

### 6.4 Example 4: Preparation of trans-(-)-Δ⁹-THC

A mixture of olivetol, zinc chloride, and anhydrous dichloromethane is stirred at 40°C for 1 hour under an Ar atmosphere. A solution of (+)-*p*-mentha-2,8-dien-1-ol and dichloromethane is added drop-wise over 1 hour to the stirred olivetol-containing mixture, and the resultant mixture is then stirred for an additional 40 minutes at 40°C. The mixture can then be cooled to -10°C, and a solution of BF₃Et₂O in anhydrous dichloromethane is then added dropwise over one hour. The resulting mixture can then be stirred for 1.5 hours at -10°C. Cold water is then added, and the resulting organic phase can then be collected and washed with cold water, 7% aqueous sodium bicarbonate, and water. The organic phase can then be dried with Na₂SO₄ and filtered. The resulting filtrate can then be concentrated under reduced pressure to provide crude trans-(-)-Δ⁹-THC **(1a)** as a brown oil.

The crude trans-(-)-Δ⁹-THC oil can be dissolved in heptane and the resulting mixture can then be thoroughly washed with 10% NaOH and water, dried over Na₂SO4, and then filtered. The resulting filtrate can then be concentrated under reduced pressure to provide a first crude residue that contains trans-(-)-Δ⁹-THC **(1a).** The crude residue is also expected to contain Δ⁸-THC (**2a**), and Δ⁸-iso-THC.

This first crude residue can be dissolved in heptane to provide a solution that can then be extracted three times with 9% NaOH in 80% methanol. The combined basic methanolic extracts are then acidified to approximately pH 7 with 20% citric acid and then extracted three times with heptane. The combined organic fractions can then be washed with water, dried over Na₂SO₄, and then filtered. The resulting filtrate can then be concentrated under reduced pressure to provide a crude residue containing about 40 wt. % (HPLC) of trans-(-)-Δ⁹-THC.

### 6.5 Example 5: Preparation of trans-(+)-Δ⁹-THC

*Synthesis of crude (+)-CBD (**3b**)*: A mixture of olivetol, zinc chloride, water and dichloromethane is refluxed for 1 hour. A solution of (-)-p-mentha-2,8-dien-1-ol in dichloromethane is added drop-wise over 0.75 hour to the refluxing mixture, and the resulting reaction mixture is then mixed for 0.5 hours at reflux. The mixture can then be cooled to 25°C, ice water added, and the resultant biphasic mixture can then be stirred for 20 minutes at 0°C. The resultant organic phase is then collected, washed twice with water and then with 5% NaHCO₃. The organic phase is then dried over Na₂SO₄, filtered, and can then be concentrated under reduced pressure to provide a first crude (+)-CBD residue. The first crude (+)-CBD residue can then be purified by column chromatography on silica gel (eluent MTBE/hexane) to provide a second crude (+)-CBD residue.

*Synthesis of (+)-CBD-bis(3,5-dinitrobenzoate)(**4b**)*: A solution of 3,5-dinitrobenzoyl chloride in dichloromethane is added dropwise to a stirred mixture of the second crude (+)-CBD residue, 4, N,N-dimethylaminopyridine, pyridine, and dichloromethane at 0° to 5°C. The mixture is then allowed to warm to 25°C and stirred for 2 hour at 25°C. The mixture can then be poured into a mixture of 37% HCI, ice, and dichloromethane. The resulting organic phase can then be collected, sequentially washed with brine, and 5% NaHCO₃, dried over Na₂SO₄, and then filtered. The resulting filtrate can then be concentrated under reduced pressure to provide crude (+)-CBD-bis(3,5-dinitrobenzoate) (**4b**). A solution of the crude (+)-CBD-bis(3,5-dinitrobenzoate) in a 10:1 (vol:vol) mixture of isopropanol and ethylacetate is stirred overnight at 25°C and then filtered. The resulting precipitate can then be washed three times with a 10:1 (vol:vol) mixture of isopropanol and ethylacetate, and dried under reduced pressure to provide crystalline (+)-CBD-bis(3,5-dinitrobenzoate) (**4b**).

*Synthesis of (+)-CBD (**3b**)*: A mixture of the crystalline (+)-CBD-bis(3,5-dinitrobenzoate) (**4b**), butylamine, and toluene, is stirred at room temperature for 12 hours and then concentrated under reduced pressure. The resulting residue can then be purified by column chromatography on silica gel (eluent hexane:MTBE (70:1 (v:v)) to provide (+)-CBD as an oil, which can then be dissolved in hexane and stored overnight at -15°C. The resultant mixture can then be filtered, and the resulting solids can then be dried under reduced pressure to provide (+)-CBD (3b) as white crystals, which can have a purity of about 98% (by GC). *Synthesis of trans-(+)-*Δ*⁹-THC:* A solution of BF₃·Et₂O in anhydrous dichloromethane is added dropwise with stirring over 1 hour to a solution of crystalline (+)-CBD (**3a**) in anhydrous dichloromethane at -5°C. The resulting mixture is stirred for 1.5 hours at -5°C. The mixture is then added to a mixture of ice and 7% NaHCO₃. The resulting organic phase can then be collected and the aqueous phase can be extracted twice with dichloromethane. The combined organic phases are washed with water, dried with Na₂SO₄, and then filtered. The resulting filtrate can then be concentrated under reduced pressure at 40°C. The resulting residue can then be purified by column chromatography on silica gel (stationary phase) using MTBE:hexane (1:100 to 3:100 (v:v)) as eluent to provide crude trans-(+)-Δ⁹-THC **(1b),** which is expected to have a purity of about 90% and to appear as a yellow oil.

### 6.6 Example 6: Preparation of trans-(+)-Δ⁹-THC

A mixture of olivetol, zinc chloride, and anhydrous dichloromethane is stirred at 40°C for 1 hour. A solution of (-)-*p*-mentha-2,8-dien-1-ol in anhydrous dichloromethane is added drop-wise over 1 hour at 40°C to the stirred olivetol-containing mixture, and the resultant mixture is then stirred for an additional 40 minutes at 40°C. The mixture can then be cooled to -10°C, and a solution of BF₃·Et₂O in anhydrous dichloromethane can then be added drop-wise over 1 hour at -10°C. The mixture is then stirred for 30 minutes at -10°C. Cold water is then added, and the resulting biphasic mixture is stirred for an additional 20 minutes at 0°C. The resulting organic phases are collected, washed sequentially with cold water, 5% aqueous sodium bicarbonate, and water. The organic phase can then be concentrated under reduced pressure at 40°C, and the resulting residue can then be dissolved in n-heptane at 25°C. The resulting solution is then sequentially washed with 10% aqueous KOH, and water, then dried with MgSO₄, and filtered. The resulting filtrate can then be concentrated under reduced pressure at 40°C. The resulting residue can then be fractionally distilled at reduced pressure (0.1 mbar) to provide trans-(+)-Δ⁹-THC **(1b).**

### 6.7 Example 7: Preparation of trans-(±)-Δ⁹-THC

A solution of BF₃·Et₂O in anhydrous dichloromethane is added dropwise with stirring over 1 hour to a solution of (±)-CBD in anhydrous dichloromethane at -5°C. The (+)-CBD used in this step can be prepared according to the method disclosed in Example 3, above, except that (±)-p-mentha-2,8-dien-1-ol is used as a reagent rather than (+)-p-mentha-2,8-dien-1-ol. The resultant mixture is then stirred for 1.5 hours at -5°C. The mixture can then be added to 7% NaHCO₃. The resultant organic phase is then collected and the aqueous phase can be extracted with dichloromethane. The organic phase is then washed with brine and can be dried with Na₂SO₄ and filtered. The resulting filtrate can then be concentrated under reduced pressure. The resulting residue can then be purified by column chromatography on silica gel (stationary phase) and MTBE:hexane (1:100 to 2:100 (v:v)) as eluent to provide crude trans-(±)-Δ⁹-THC, which is expected to have the appearance of a yellow oil. The oily trans-(±)-Δ⁹-THC prepared in this manner can then be dissolved in hexane, and the resulting mixture is then maintained at -15 °C for 24 hours. The resulting mixture is then filtered, washed with cold hexane and then dried under reduced pressure to provide trans-(±)-Δ⁹-THC, which is expected to have the appearance of slightly rose-colored crystals.

### 6.8 Example 8: Preparation of trans-(±)-Δ⁹-THC

*Preparation of (-)-(1R, 2R, S5)-2-phenylthio-8-p-menthen-1-o*/*:* A mixture of (-)-limonene oxide (e.g. comprising about 1:1 cis:trans diastereomeric mixture; available from Aldrich Chemical, St. Louis, Missouri), thiophenol (e.g. available from Fluka Chemical, Buchs, Switzerland), potassium carbonate, N,N-dimethylformamide, and toluene are stirred at 117°C for 19 hours under an Ar atmosphere. The mixture can then be cooled to 25°C and water added. The resulting organic phase can then be collected, and the water layer can be extracted with toluene. The combined organic phases can then be washed sequentially with water and a 15% solution of brine. The organic phase can then be dried over Na₂SO₄ and filtered, and the resulting filtrate can then be concentrated under pressure at 65°C. The resulting product, which is expected to appear as a brown oil can then be fractionally distilled under reduced pressure to provide (-)-cis-limonene oxide, which can have a purity of about 90% or greater.

*Preparation of (1R,2R,4S)-1-Hydroxy-8-p-menthen-2-phenylsulfoxide*: (-)-(1R,2R,4S)-2-phenylthio-8-*p*-menthen-1-ol is dissolved in methyl alcohol with stirring at 25°C under an Ar atmosphere. The resulting solution can then be cooled to -10° to -5°C. A solution of OXONE^{®} (potassium peroxymonosulfate, available from Aldrich Chemical) in water is then added dropwise to the methyl alcohol solution over 2 hours at -10° to -5°C, and the resulting mixture is then stirred for an additional 30 min at -10° to -5°C. The mixture can then be warmed to 20° to 25°C, and water can then be added, and the resulting biphasic mixture can then be extracted with dichloromethane. The combined organic phases can then be dried over sodium sulfate and filtered, and the resulting filtrate can then be concentrated under reduced pressure at 60° C to provide a residue. The residue is then purified by chromatography on a silica gel column (eluent: n-heptane/ethyl acetate 9:1 then 8:2). The fractions that contain mainly (1R,2R,4S)-1-hydroxy-8-*p*-menthen-2-phenyl sulfoxide are then combined and concentrated under vacuum for 10 hours at 40° to 50°C to provide (1R,2R,4S)-1-hydroxy-8-*p*-menthen-2-phenyl sulfoxide as a mixture of two diastereomers. The product of this reaction can be stored frozen.

*Preparation of (-)-cis-p-Mentha-2,8-dien-1-ol:* A mixture of (1R,2R,4S)-1-hydroxy-8-*p*-menthen-2-phenylsulfoxide and piperidine in dimethylsulfoxide is heated to 163°C under a flowing Ar atmosphere, and the resultant mixture is then stirred at 163°C for 3 hours. The mixture can then be cooled to 20° to 25°C, treated with water, and then extracted with diethyl ether. The organic phases can be combined and washed sequentially with 1N HCl, a 7% solution of sodium hydrogen carbonate, and brine, and then dried over sodium sulfate. The organic phase can then be concentrated under reduced pressure. The resultant residue can then be purified by silica gel column chromatography (eluent: n-heptane followed by n-heptane:ethyl acetate (1:9 (v:v)). Fractions containing mainly (-)-*cis*-*p*-mentha-2,8-dien-1-ol are combined and concentrated under reduced pressure at 40° to 50°C over 10 hours to provide (-)-*cis*-*p*-mentha-2,8-dien-1-ol.

*Preparation of trans-(±)-*Δ*⁹-THC:* (+)-p-mentha-2,8-dien-1-ol is prepared as described above, except that (+)-limonene oxide (*e.g.* comprising about a 1:1 cis/trans diastereomeric mixture) is used instead of (-)-limonene oxide. A mixture of olivetol, zinc chloride, and anhydrous dichloromethane is stirred at 40°C for 1 hour. A solution of (-)-*p*-mentha-2,8-dien-1-ol, (+)-*p*-mentha-2,8-dien-1-ol, and anhydrous dichloromethane are added drop-wise over 1 hour at 40° to the stirred olivetol-containing mixture, and the resulting mixture is then stirred for an additional 40 minutes at 40°C. The mixture can then be cooled to -10°C, and a solution of BF₃·Et₂O in anhydrous dichloromethane is then added drop-wise over 1 hour at -10°C. The mixture can then be stirred for 30 minutes at -10°C, and then cold water can be added. The resulting biphasic mixture is then stirred for an additional 20 minutes at 0°C. The resulting organic phase can then be collected and washed with cold water, 8% aqueous sodium bicarbonate, and water. The organic phase can then be concentrated under reduced pressure at 40°C. The resulting residue can then be dissolved in n-heptane at 25°C and washed with 10% aqueous KOH for 40 min at 25°C, and then washed with water. The organic phase can then be concentrated under reduced pressure at 50°C to provide crude (±)-Δ⁹-THC, which is expected to appear as a brown oil.

Crude (±)-Δ⁹-THC oil prepared in this manner can then be dissolved in a minimal amount of heptane and then purified by chromatography in a single pass using a Merck-Knauer PP K-1800 preparative chromatograph with one cylinder (e.g. 50 mm x 210 mm of LUNA CM 10 µm; loading capacities 600 mg; eluent: n-heptane). Fractions containing trans-(±)-Δ⁹-THC are combined and concentrated under reduced pressure at 40°C to provide trans-(±)-Δ⁹-THC (1), which can have a purity of over 90%.

### 6.9 Example 9: Preparation of trans-(±)-Δ⁹-THC

A mixture of olivetol, zinc chloride, and anhydrous dichloromethane is stirred at 40°C for 1 hour. A solution of (±)-*p*-mentha-2,8-dien-1-ol in anhydrous dichloromethane is then added drop-wise over 1 hour at 40° to the stirred olivetol-containing mixture, and the resultant mixture is then stirred for an additional 0.50 hours at 40°C. The mixture can then be cooled to -10°C, and a solution of BF₃·Et₂O in anhydrous dichloromethane is then added drop-wise to the mixture over 1 hour at -10°C. The mixture is then stirred for 0.50 hours at -10°C, and cold water can then be added with stirring to form a biphasic mixture. The organic phase is then collected and washed with cold water, 5% aqueous sodium bicarbonate, and water. The organic phase can then be dried over Na₂SO₄ and filtered, and the resulting filtrate can then be concentrated under reduced pressure to provide a first crude trans-(±)-Δ⁹-THC residue.

The first crude trans-(±)-Δ⁹-THC residue can then be dissolved in heptane, and the resulting solution can then be washed with 10% NaOH, and water. The organic solution can then be dried by azeotropic distillation and concentrated under reduced pressure to provide a second crude trans-(±)-Δ⁹-THC residue.

### 6.10 Example 10: Preparation of trans-(±)-Δ⁹-THC from a Mixture of Crude trans-(-)-Δ⁹-THC and trans-(+)-Δ⁹-THC

Trans-(-)-Δ⁹-THC can be prepared as described in Example 9 for preparing trans-(±)-Δ⁹-THC residue, except that (+)-p-mentha-2,8-dien-1-ol is used instead of (±)-p-mentha-2,8-dien-1-ol. Crude trans-(-)-Δ⁹-THC prepared in this manner can be about 40% by weight of trans-(-)-Δ⁹-THC, as determined by HPLC.

Trans-(+)-Δ⁹-THC can be prepared as described in Example 9 for preparing trans-(±)-Δ⁹-THC residue, except that (-)-p-mentha-2,8-dien-1-ol is used instead of (±)-p-mentha-2,8-dien-1-ol. Crude trans-(+)-Δ⁹-THC prepared in this manner can be about 35% by weight of trans-(+)-Δ⁹-THC, by HPLC.

The crude trans-(-)-Δ⁹-THC and crude trans-(+)-Δ⁹-THC can be dissolved together in heptane at 25°C. The resultant solution is then admixed with a solution of 9% aqueous NaOH:methanol (20:80 (v:v)). The methanolic phases are then combined and treated with 10% citric acid at 0°C to about 5°C until the pH is about 7. Heptane is then added, and the resulting organic phase is washed with water. The organic phase can then be dried over Na₂SO₄ and filtered, and the resulting filtrate can then be concentrated under reduced pressure to provide crude trans-(±)**-**Δ⁹-THC. The crude trans-(±)-Δ⁹-THC prepared in this manner can have a purity (HPLC) of about 45%, and is expected to be a brown oil.

The crude trans-(±)-Δ⁹-THC can then be dissolved in heptane, and the resulting solution is then cooled to 0°C and seeded with crystalline (±)-Δ⁹-THC. The resulting mixture can then be further cooled to -15°C for 12 hours and filtered. The resulting solids can then be washed with cold heptane and dried under reduced pressure to provide trans-(±)-Δ⁹-THC. The trans-(±)-Δ⁹-THC can have a purity of over 95% and is expected to appear as a white crystalline solid. Moreover, crystalline trans-(±)-Δ⁹-THC prepared in this manner is expected to retain that white appearance for at least three days at 25°C. Stability of crystalline trans-(±)-Δ⁹-THC can be monitored, as a function of storage conditions, using HPLC analytical methods known in the art that are capable of separating and detecting cannabinoid impurities. In this manner, it can be demonstrated that the crystalline trans-(±)-Δ⁹-THC API of the present invention is more stable to air, temperature, and light as compared to the pure trans-(-)-Δ⁹-THC enantiomer.

### 6.11 Example 11: Preparation of (±)-Δ⁹-THC from (±)-Δ⁸THC

*Preparation of (±)-*Δ*⁸-THC:* A solution of methanesulfonic acid in dichloromethane is added to a solution of olivetol and (±)-*p*-mentha-2,8-dien-1-ol in dichloromethane. The resultant mixture can be refluxed for 4 hours with removal of water using a Dean-Stark separator. The mixture is then cooled to 25°C and treated with aqueous NaHCO₃. The resultant organic phase can then be collected and concentrated under reduced pressure. The resultant residue is dissolved in heptane and washed with 10% NaOH, and the resultant organic phases can be concentrated under reduced pressure to provide crude (±)-Δ⁸-THC, which can have a purity of greater than 65%.

*Preparation of (±)-9-chloro-trans-hexahydrocannabinol:* A mixture of the crude (±)-Δ⁸-THC, zinc chloride, and anhydrous dichloromethane is stirred for 0.5 hours at 25°C under an Ar atmosphere. The mixture is then cooled to 0°C, and gaseous hydrogen chloride is bubbled through the mixture for 1.5 hours. The mixture can then be poured into an ice bath, and the resultant biphasic mixture stirred for 1 hour at 0 to 5°C. The organic phase can then be collected and washed sequentially with cold water, 8% sodium bicarbonate solution, and water. The organic phase is then dried over anhydrous Na₂SO₄, and filtered. The resultant filtrate is then concentrated under reduced pressure at 30°C. The resultant residue can then be dissolved in n-heptane, cooled to 0°C, and seeded with (±)-9-chloro-trans-hexahydrocannabinol. The resultant mixture is then stirred at 0°C for 5 hours, cooled to -15°C, and stirred at -15°C for 60 hours. The mixture can then be filtered and the resultant solids are washed with cold n-heptane. The solids can then be dried under reduced pressure at 50°C to provide (±)-9-chloro-trans-hexahydrocannabinol. The purity of the (±)-9-chloro-trans-hexahydrocannabinol prepared in this manner can exceed 99%, as analyzed by HPLC.

*Preparation of (±)-*Δ*⁹-THC:* A mixture of potassium-tert-amylate, the (±)-9-chloro-trans-hexahydrocannabinol prepared as above, and anhydrous toluene is stirred for 75 minutes at 65°C. The mixture is then cooled to 25°C and poured into ice water. The resulting organic phase can then be collected and washed sequentially with cold water, 7% sodium bicarbonate, and water. The organic phase can then be dried over anhydrous Na₂SO₄ and concentrated under reduced pressure. The resulting residue can then be dissolved in n-heptane, cooled to 0°C, and then seeded with (±)-Δ⁹-THC. The resulting mixture can then be stirred at 0°C for 5 hours, cooled to -15°C, and then stirred at -15°C for 60 hours. The mixture can then be filtered and the resulting solids washed with cold n-heptane. The solids can then be dried under reduced pressure at 50°C to provide (±)-Δ⁹-THC, which can have a purity (HPLC) of over 95%.

### 6.12 Example 12: Purification of (±)-Δ⁹-THC

*Preparation of (±)-*Δ*⁹-THC m-nitrobenzenesulfonate:* A mixture of crude (±)-Δ⁹-THC (e.g., the second crude residue prepared according to the method of Example 9, above) can be combined with 3-nitrobenzenesulfonyl chloride, triethylamine, and dichloromethane, and then stirred at 25°C for 1 hour. The resultant admixture can then be treated with cold water, after which the resultant organic phase can be collected and washed sequentially with 10% HCl, water, 5% NaHCO₃ and water. The organic phase is then dried over Na₂SO₄ and filtered. The resultant filtrate can then be concentrated under reduced pressure to provide a first crude (±)-Δ⁹-THC *m*-nitrobenzenesulfonate residue, which can have a purity of about 40 wt.% (by HPLC).

This first crude (±)-Δ⁹-THC *m*-nitrobenzenesulfonate residue can then be dissolved in isopropanol at 50°C. The resultant solution is then cooled to room temperature, seeded with powdered crystalline (±)-Δ⁹-THC *m*-nitrobenzenesulfonate, cooled to 0°C, and then stirred for 12 hour at 0°C. The resultant mixture is then filtered, and the resultant solids can be washed with cold heptane, and dried under reduced pressure to provide a second crude (±)-Δ⁹-THC *m*-nitrobenzenesulfonate residue as a yellow solid. The second crude (±)-Δ⁹-THC *m*-nitrobenzenesulfonate residue can, according to HPLC, have a purity of over 75 %.

The second crude (±)-Δ⁹-THC *m*-nitrobenzenesulfonate can then be dissolved in dichloromethane. The resultant solution can be distilled while isopropanol is continuously added dropwise to the mixture. The distillation is stopped when the temperature of vapors in the head of the column reaches 82.4 °C. The contents of the distillation pot are then cooled to 0°C to 5°C, and the resultant suspension stirred for 12 hours at 0°C to about 5°C. The suspension can then be filtered, and the resultant solids washed with cold heptane and then dried under reduced pressure to provide crystalline (±)-Δ⁹-THC *m*-nitrobenzenesulfonate, which can have a , HPLC-determined purity of 99.0%

*Preparation of (±)-*Δ*⁹-THC*: A mixture of the crystalline (±)-Δ⁹-THC m-nitrobenzenesulfonate prepared as above, 50% NaOH, and methanol is stirred at 50°C for about 1-2 hours and then cooled to room temperature. The cooled mixture can then be mixed with cold water followed by addition of 10% HCl until the pH is about 7. The resultant mixture can then be extracted with heptane, and the organic extract washed sequentially with 7% NaHCO₃ and water. The organic phase is then dried over Na₂SO₄ and filtered. The resultant filtrate can then be concentrated under pressure to provide crude (±)-Δ⁹-THC. Analysis (HPLC) of the crude product indicates that a purity of over 92% by weight of (±)-Δ⁹-THC can be obtained.

The crude (±)-Δ⁹-THC can then be dissolved in heptane at 40°C. The resultant solution is then cooled to 0°C, seeded with powdered crystalline (±)-Δ⁹-THC, and stirred for 12 hours at -15°C. The resultant mixture can then be filtered and the resultant solids washed with cold heptane. The solids are then dried under reduced pressure to provide (±)-Δ⁹-THC as off-white crystals. This crystalline (±)-Δ⁹-THC is stable at 25°C in the presence of air and laboratory lighting. Moreover, HPLC analysis of the product indicates that a purity of 99.0% can be obtained.

### 6.13 Example 13: Preparation of Crystalline (±)-Δ⁹-THC from trans-(-)-Δ⁹-THC and trans-(+)-Δ⁹-THC

A solution of trans-(-)-Δ⁹-THC **(1a)** and trans-(+)-Δ⁹-THC **(1b)** in heptane is cooled to 0°C, seeded with trans-(±)-Δ⁹-THC, and stirred for 5 hours at 0°C. The resultant mixture is cooled to -15°C and stirred for an additional 48 hours at -15°C. The mixture is then filtered, and the resultant solids are washed with cold n-heptane. The solids recovered are then dried under reduced pressure at 35°C to provide crude (+)-Δ⁹-THC, which can have a purity of over 93%.

This crude (±)-Δ⁹-THC can then be dissolved in heptane at 50°C, and the mixture cooled to 0°C with stirring. The resultant mixture can then be stirred at 0°C for 2 hours, cooled to -15°C, and stirred for an additional 48 hours at -15°C. The mixture can then be filtered and the resultant crystalline solids washed with cold n-heptane. The solids obtained are then dried under reduced pressure at 35°C to provide crystalline (+)-Δ⁹-THC that can have a purity of over 97 %.

### 6.14 Example 14: Preparation of Crystalline (±)-Δ⁹-THC

Trans-(+)-Δ⁹-THC, obtained for example by enantioselective chromatography of crystalline (±)-Δ⁹-THC as described in Example 16 below, and trans-(-)-Δ⁹-THC which can be obtained according to the method of Example 4 above, are dissolved in heptane. The resultant solution can be cooled to 0°C and seeded with crystalline (+)-Δ⁹-THC. The resultant mixture is stirred for 5 hours at 0°C, and then for 72 hour at -15°C. The resultant mixture is filtered and the solids washed with cold heptane. The solids are then dried under reduced pressure at 35°C to provide crystalline (±)-Δ⁹-THC, which can have a purity of greater than 98%.

### 6.15 Example 15: Preparation of Crystalline (±)-Δ⁹-THC

Crude trans(-)-Δ⁹-THC and crude trans-(+)-Δ⁹-THC can be prepared by processes described in Examples 4 and 6, above, respectively. Crude trans-(-)-Δ⁹-THC and crude trans-(+)-Δ⁹-THC in heptane can be admixed with a methanolic caustic solution containing 50% caustic, water, and methanol for 20 minutes at 25°C. The resultant purple methanolic caustic (lower) phase can be collected, and the organic phase admixed again with a methanolic caustic solution containing 50% caustic, water, and methanol for 20 minutes at 25°C. The resultant methanolic caustic phase can be collected, and a 10% solution of citric acid in water can be added to the combined methanolic caustic phases to lower the pH to about 7. The yellow admixture that results can then be extracted with heptane. The resultant organic phase can be collected and washed with water, dried over Na₂SO₄, and filtered. The resultant filtrate can be dried by azeotropic distillation and concentrated under reduced pressure. The red oil that is produced can be dissolved in heptane, cooled to 0°C, and seeded with crystalline (+)-Δ⁹-THC. The resultant admixture can be cooled to -15°C and stirred at -15°C for 12 hours. The resultant mixture can be suction-filtered, and the solids washed with cold heptane. The resultant yellow solids are allowed to dry under suction to provide crude (+)-Δ⁹-THC.

The crude (+)-Δ⁹-THC can be dissolved in heptane at 50°C, and the resultant solution cooled to -10°C for 2-3 hours. The resultant mixture can be suction-filtered and the solids washed 3 times with cold heptane. The solids are then allowed to dry under suction to provide (±)-Δ⁹-THC as white crystals, that can have a purity greater than 95%.

### 6.16 Example 16: Resolution of trans-(-)-Δ⁹-THC and trans-(±)-Δ⁹-THC from (±)-Δ⁹-THC

(±)-Δ⁹-THC prepared according to any of the above procedures can be separated by flash chromatography on a Merck column using Chiralpak^{®} AD^{™} 20 µm chiral (Daicel, Tokyo, Japan) as the stationary phase (loading capacity 500 mg per injection, UV at 228 nm) and n-heptane:2-propanol (95:5 (v:v)) as the mobile phase. Fractions containing only the trans-(-)-Δ⁹-THC isomer can be combined and the volatiles removed using a rotary evaporator at 35° to 40°C to provide trans-(-)-Δ⁹-THC **(1a).** In this manner, up to 99.9% pure trans-(-)-Δ⁹-THC can be isolated.

### 6.17 Example 17: Resolution of trans-(-)-Δ⁹-THC and trans- (+)-Δ⁹-THC from (±)-Δ⁹-THC

Crystalline (+)-Δ⁹-THC, prepared according to any of the above procedures, can be dissolved in heptane:2-propanol (95:5 (v:v)) mixture. The resultant solution is then injected into a 2 inch stainless steel "Load and Lock" column (Varian) packed with Chiralpak^{®} AD chiral derivatized silica (Chiral Technologies, Inc. Exton, PA). Elution can be carried out under isocratic conditions with a solution of heptane:isopropanol (95:5 (v:v)) at a temperature of about 25°C and at a flow rate of 250 mL of eluent/min. Detection of compounds in the eluent can be carried out by UV absorption at 235 nm.

Trans-(+)- Δ⁹-THC will elute first, and the combined trans-(+)- Δ⁹-THC eluents can be concentrated under reduced pressure to provide trans-(+)-Δ⁹-THC **(1b)** as a reddish-yellow oil.

Trans-(-)-Δ⁹-THC will elute after the trans-(+)-Δ⁹-THC, and the combined trans-(-)- Δ⁹-THC eluents can be concentrated under reduced pressure to provide trans-(-)-Δ⁹-THC **(1a)** as a thick viscous reddish-yellow oil. Trans-(-)-Δ⁹- THC product prepared in this manner can have a purity of over 99%.

### 6.18 Example 18: Resolution of trans-(-)-Δ⁹-THC and trans-(+)-Δ⁹-THC from (±)-Δ⁹-THC

Crystalline (±)-Δ⁹-THC, e.g. prepared according to any of the above procedures, can be dissolved in a 95:5 heptane:IPA (v:v) mixture to provide a 10 wt.% solution. A portion of the 10% solution is injected into a 220 x 50 mm stainless steel column (Merck) packed with Chiralpak^{®} AD 20 mm chiral derivatized silica (Daicel, Tokyo, Japan). Elution can be carried out under isocratic conditions with a solution of heptane:2-propanol (95:5 (v:v)) solvent at about 25°C and at a flow rate of 200 mL of eluent/min. Detection of products in the eluent can be carried out by UV absorption at 228 nm.

The fractions containing (+)-Δ⁹-THC can be combined and concentrated under reduced pressure to provide (+)-Δ⁹-THC as reddish-yellow oil having a purity of about 97.0%.

The fractions containing trans-(-)-Δ⁹-THC can be combined and concentrated under reduced pressure to provide trans-(-)-Δ⁹-THC **(1a)** as a thick viscous reddish-yellow oil having a purity of 99.9%. This product is stored in a freezer and protected from light and oxygen.

### 6.19 Example 19: Preparation and Characterization of Crystalline trans-(±)-Δ⁹-THC

### 6.19.1 Preparation of (-)-Δ⁹-Tetrahydrocannabinol from n-Cannabidiol

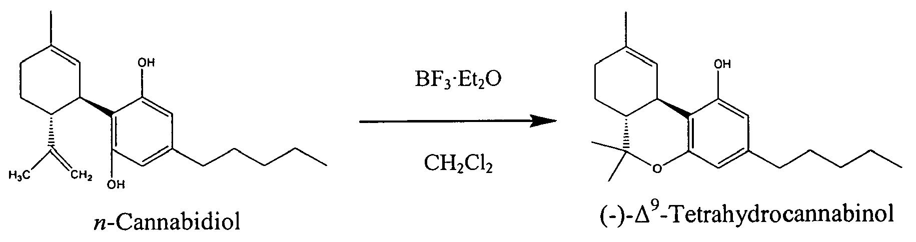

A 250-mL reactor was charged with dichloromethane (240 g) and boron trifluoride diethyletherate (8.4 g) and filled with argon. The resulting solution was cooled to -10°C and the solution of n-cannabidiol (15.0 g) in dichloromethane (60 g) was added dropwise to the mixture over a one-hour period at -10 °C. The reaction mixture was stirred for an additional two hours at the same temperature (-10°C). A sample taken after 1.5 hours was analyzed (gas chromatography) and found to contain (-)-Δ⁹-tetrahydrocannabinol ((-)-Δ⁹-THC) (80.8%), n-cannabidiol (CBD) (4.46%) and Δ⁸-iso-THC (12.3%).

The reaction mixture was poured into ice-water (100 g) and the mixture was stirred for 20 minutes at 0 °C. The dichloromethane layer was washed successively with cold water (50 g), dilute sodium hydrogen carbonate solution (50 g) and water (50 g). The dichloromethane solution was dried over anhydrous sodium sulfate (15 g), and solvent was evaporated under reduced pressure at 40 °C (i.e. temperature of the water bath) to provide 14.9 g of a yellow oil (yield 99%) containing 81.8% of (-)-Δ⁹-THC, according to HPLC analysis.

### 6.19.2 Preparation of (+)-Δ⁹-THC

(+)-Δ⁹-THC was prepared by chromatographic separation of racemic Δ⁹-TH on a preparative chromatographic instrument (Merck-Knauer PP K-1800) (Knauer; Berlin, Germany). The racemic Δ⁹-THC was separated by flash chromatography through a Merck column (210 x 50 mm) (Merck; Darmstadt, Germany) packed with Chiralpak^{®} AD™ (Diacel: Tokyo, Japan) 20 µm chiral stationary phase (loading capacity: 500 mg per injection) with UV detection at 228 nm. Elution was carried out with n-heptane/2-propanol 95:5 (v/v), flow rate 200 mL/min at 20-25 °C. Fractions containing pure (+)-Δ⁹-THC were combined and evaporated to dryness on a rotary evaporator under reduced pressure at a temperature (water bath) of 35 to 40°C. Drying was terminated when the product reached a constant weight (weight loss is less than 0.2% in 5 to 6 hours under a vacuum of less than 1.0 mbar) to give the target compound (+)-Δ⁹-THC. The combined sample, which was used in the following step (preparation of crystalline (±)-Δ⁹-THC) had a purity (HPLC) of 94.3%.

### 6.19.3 Preparation of Crystalline (±)-Δ⁹-THC

A 100 mL reactor was charged with a solution of crude comprising (-)-Δ⁹-THC (3.36 g; 81.8%) and (+)-Δ⁹-THC (2.76 g; 94.3%) in n-heptane (6.5 g). The solution was seeded with racemic Δ⁹-THC (0.01 g), at 0 °C and stirring continued for 5 hours at 0 °C. The mixture was cooled to -15°C and stirred for 48 hours at the same temperature. Precipitated solids were collected by filtration, washed on the filter with cold n-heptane (6.0 g) and dried under reduced pressure at 35°C (water bath temperature) to a constant weight to provide 3.5 g of racemic Δ⁹-THC. After two re-crystallizations from n-heptane, 2.0 g of crystalline racemic Δ⁹-THC was obtained. This material was characterized by powder X-ray diffraction, HPLC, melting point determination, differential scanning calorimetry (DSC), thermal gravimetric analysis (TGA), and infrared spectroscopy (FTIR), as provided in the following six sections.

### 6.19.4 Powder X-Ray Diffraction (PXRD) of Crystalline (±)-Δ⁹-THC

The powder X-ray diffraction pattern of crystalline (±)-Δ⁹-THC was determined according to methods known in the art using PANALYTICAL (Phillips/PANalytical Inc.; Natick, MA), X'Pert Pro MPD x-ray powder system (CuKα radiation, PW3050/60 goniometer, PW3015/20 RTMS detector (X'Celerator). The analysis was performed with goniometer running in continuous mode set for 6.35 second count per 0.017 step over a two-theta range of 5° to 35°. The results are shown in FIG 1 and summarized in Table 1.

**TABLE 1**

| Position [° 2Theta] | Height (counts) | FWHM [°2Theta] | d-Spacing [Å] | Relative Intensity [%] |
|---|---|---|---|---|
| 6.6692 | 19498.80 | 0.1937 | 13.24282 | 100.00 |
| 7.5065 | 371.92 | 0.2442 | 11.76756 | 1.91 |
| 8.2160 | 6175.19 | 0.2022 | 10.75285 | 31.67 |
| 10.0639 | 452.67 | 0.1833 | 8.78223 | 2.32 |
| 12.0785 | 2719.63 | 0.2648 | 7.32158 | 13.95 |
| 12.5994 | 3096.56 | 0.1849 | 7.01999 | 15.88 |
| 13.4115 | 1526.84 | 0.1946 | 6.59672 | 7.83 |
| 15.7538 | 3687.75 | 0.2438 | 5.62076 | 18.91 |
| 16.7992 | 3184.87 | 0.2996 | 5.27328 | 16.33 |
| 16.9469 | 3842.66 | 0.1379 | 5.22765 | 19.71 |
| 18.1358 | 824.73 | 0.2900 | 4.88753 | 4.23 |
| 18.3638 | 838.84 | 0.1931 | 4.82737 | 4.30 |
| 18.9889 | 1149.05 | 0.3438 | 4.66985 | 5.89 |
| 19.4280 | 1481.36 | 0.2117 | 4.56526 | 7.60 |
| 20.3297 | 5867.26 | 0.2421 | 4.36478 | 30.09 |
| 21.3925 | 2243.64 | 0.2405 | 4.15027 | 11.51 |
| 22.6319 | 891.49 | 0.2973 | 3.92572 | 4.57 |
| 23.1056 | 549.17 | 0.2055 | 3.84628 | 2.82 |
| 23.7747 | 914.98 | 0.2504 | 3.73952 | 4.69 |
| 24.8661 | 800.07 | 0.3940 | 3.57782 | 4.10 |
| 25.6949 | 264.12 | 0.2130 | 3.46427 | 1.35 |
| 26.8305 | 79.60 | 0.2079 | 3.32016 | 0.41 |
| 27.4632 | 144.77 | 0.2812 | 3.24509 | 0.74 |
| 28.6526 | 365.56 | 0.3754 | 3.11303 | 1.87 |
| 31.5552 | 296.56 | 0.3828 | 2.83298 | 1.52 |
| 33.7001 | 78.62 | 0.0830 | 2.65741 | 0.40 |
| 34.1623 | 86.35 | 0.4055 | 2.62251 | 0.44 |

### 6.19.5 HPLC Analysis of Crystalline (±)-Δ⁹-THC

HPLC analysis was carried out using a LaChrom System 2 (Merck-Hitachi; Merck KGaA, Darmstadt, Germany/Hitachi Instruments, Inc., Separation Systems Group, San Jose, CA) and indicated a purity of 98.8% (FIG 2). The column used was a Hypersil BDS C18 3 micron; 150 x 4.6 mm column. Mobile phase (A: methanol, B: water, C: THF); 71%A/24%B/5%C for 25 minutes, gradient to 71%A/5%B/24%C in 10 minutes, 71%A/5%B/24%C for 10 minutes; flow rate: 1 mL/min; Detection: UV detector fixed wavelength (228 nm); Temperature: 25°C.

The results are presented in FIG 2.

### 6.19.6 Melting Point of Crystalline (±)-Δ⁹-THC

The melting point of crystalline (±)-Δ⁹-THC was measured using a Büchi, B-545 (Zurich, Switzerland) melting point instrument. The melting point was determined to be 63.3-64.0 °C.

### 6.19.7 Differential Scanning Calorimetry of Crystalline (±)-Δ⁹-THC

Differential scanning calorimetry of crystalline (±)-Δ⁹-THC was performed using a Mettler Toledo DSC822e instrument (Mettler Toledo; Columbus, Ohio). Approximately 7 mg of crystalline (±)-Δ⁹-THC was accurately weighed into a 40 microliter aluminum pan and crimp sealed with a perforated lid. The sample was heated at 10°C/minute over a range of 25 °C to 320 °C with a nitrogen gas purge.

The results are depicted in FIG 3.

### 6.19.8 Thermal Gravimetric Analysis of Crystalline (±)-Δ⁹-THC

Thermal gravimetric analysis was performed using a Mettler Toledo TGA/SDTA851 a instrument. Approximately 15 mg of crystalline (±)-Δ⁹-THC was accurately weighed into a ceramic pan. The sample was heated at 10 °C/minute over the range of 25 °C to 320 °C with a nitrogen gas purge.

The results are depicted in FIG 4.

### 6.19.9 Infra-Red Spectroscopy of Crystalline (±)-Δ⁹-THC

The infra-red spectra were acquired using a Nicloet Impact 410 FT-IR Spectrometer (Nicolet Instrument Corporation, Madison, WI) equipped with a Pike Technologies (Madison, WI), EasiDiff Diffuse Reflectance Accessory using a 5% dispersion of a sample of crystalline (±)-Δ⁹-THC of the invention in potassium bromide. The spectrum was recorded at 4 cm⁻¹ resolution using 64 background and 64 sample scans over the wave number range 400 cm⁻¹ to 4000 cm⁻¹. Major peaks were recorded at 3325, 2926, 2863, 1622, 1578, 1509, 1420, 1332, 1270, 1233, 1186, 1128, 1113, 1091, 1051, 1034, 1009, 992, 972, 921, 909, 876, 846, 807, 772, 727, 694, and 654 cm⁻¹.

The spectra obtained are depicted in FIG 5A and FIG 5B.

### 6.19.10 ¹H and ¹³C NMR Spectroscopy of Crystalline (±)-Δ⁹-THC

Crystalline trans-(±)-Δ⁹-THC prepared according to the methods disclosed herein has also been characterized by both ¹H NMR (FIG 6A-6D) and ¹³C NMR (FIG 7A-7D). The data in Tables 2 and 3 below are provided for comparison with the data of FIG 6A-6D and FIG 7A-7D, respectively.

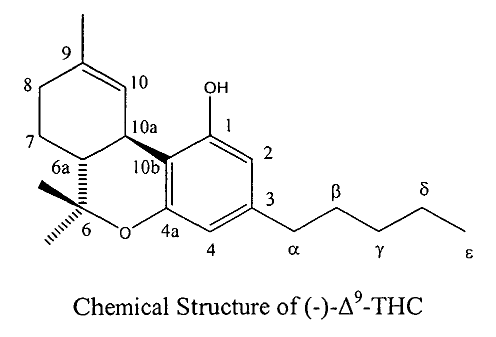

Table 2 provides a summary of a comparison of ¹H NMR Chemical Shifts Comparison of a (-)-Δ⁹-THC Reference Standard with literature values (Taylor et al. (1966) J. Am. Chem Soc. 88: 367)

**Table 2**

| **Proton** | **Chemical Shift Expt. Literature** | | **Multiplicity** | **Number of Protons** | ***J* (Hz) Expt. Literature** | |
|---|---|---|---|---|---|---|
| Aromatic | 6.13 | 6.12 | d | 1 | 1.2 | - |
| | 6.26 | 6.32 | d | 1 | 1.5 | - |
| Olefinic | 6.29 | 6.42 | d | 1 | - | - |
| 10aH | 3.20 | 3.14 | br d | 1 | - | - |
| Olefinic CH₃ | 1.69 | 1.65 | s | 3 | - | - |
| *gem* di-CH₃ | 1.09 | 1.08 | s | 3 | - | - |
| | 1.42 | 1.38 | s | 3 | - | - |
| Ω-CH₃ | 0.88 | 0.88 | t | 3 | - | - |

Table 3 provides a summary of a comparison of ¹³C NMR Chemical Shifts Comparison of a (-)-Δ⁹-THC Reference Standard with literature values (Archer et al. (1977) J. Org. Chem. 42: 490)

**Table 3**

| **Carbon** | **Chemical Shift (ppm)** | | **Carbon** | **Chemical Shift (ppm)** | |
|---|---|---|---|---|---|
| | **Expt.** | **Literature** | | **Expt.** | **Literature** |
| C(1) | 154.5 | 154.4 | C(10) | 123.5 | 123.7 |
| C(2) | 107.4 | 107.5 | C(10a) | 33.7 | 33.6 |
| C(3) | 142.6 | 142.5 | 6α-CH₃ | 19.4 | 19.2 |
| C(4) | 110.0 | 109.8 | 6β-CH₃ | 27.7 | 27.5 |
| C(4a) | 153.9 | 154.1 | 9-CH₃ | 23.5 | 23.3 |
| C(10b) | 109.0 | 108.9 | α-C | 35.6 | 35.4 |
| C(6) | 77.2 | 77.1 | β-C | 30.8 | 30.5 |
| C(6a) | 45.9 | 45.7 | γ-C | 31.6 | 31.4 |
| C(7) | 25.2 | 25.0 | δ-C | 22.7 | 22.5 |
| C(8) | 31.3 | 31.1 | ε-C | 14.2 | 15.0 |
| C(9) | 134.2 | 133.8 | | | |

The ¹H NMR spectra obtained upon analysis of crystalline trans-(±)-Δ⁹-THC prepared according to the methods disclosed herein are presented in FIG 6A-6D.

The ¹³C NMR spectra obtained upon analysis of crystalline trans-(±)-Δ⁹-THC prepared according to the methods disclosed herein are presented in FIG 7A-7D.

The present invention is not to be limited in scope by the specific embodiments disclosed in the Examples which are intended simply as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended items.

A number of references have been cited, the entire disclosures of which are hereby incorporated herein by reference.

Further Embodiments of this invention are the following:
1. A composition comprising crystalline trans-(±)-Δ⁹-tetrahydrocannabinol and a pharmaceutically-acceptable carrier.
2. A composition comprising trans-(±)-Δ⁹-tetrahydrocannabinol, wherein said composition is formulated with crystalline trans-(±)-Δ⁹-tetrahydrocannabinol and a pharmaceutically-acceptable carrier.
3. A dosage form comprising a therapeutically effective amount of crystalline trans-(±)-Δ⁹-tetrahydrocannabinol and a pharmaceutically-acceptable carrier.
4. A dosage form comprising a therapeutically effective amount of trans-(±)-Δ⁹-tetrahydrocannabinol, wherein said dosage form is formulated with crystalline trans-(±)-Δ⁹-tetrahydrocannabinol.
5. The composition or dosage form of any one of items 1-4, wherein the crystalline trans-(±)-Δ⁹-tetrahydrocannabinol comprises at least 95%, preferably at least 98%, more preferably at least 99%, even more preferably at least 99,5% and most preferably at least 99,9% by weight of the total amount of cannabinoids in said composition.
6. The composition or dosage form of any one of items 1-4, wherein the crystalline trans-(±)-Δ⁹-tetrahydrocannabinol consists essentially of trans-(-)-Δ⁹-tetrahydrocannabinol and trans-(+)-Δ⁹-tetrahydrocannabinol.
7. The composition or dosage form of any one of items 1-4, wherein the molar ratio of trans-(-)-Δ⁹-tetrahydrocannabinol to trans-(+)-Δ⁹-tetrahydrocannabinol is within a range of from about 0.8:1.2 to about 1.2:0.8, preferably within a range of from about 0.9:1.1 to about 1.1:0.9, more preferably from about 0.95:1.05 to about 1.05:0.95 and most preferably about 1:1.
8. The dosage form of item 3 or 4, wherein said amount of crystalline trans-(±)-Δ⁹-tetrahydrocannabinol is within a range of from about 0.1 mg to about 100 mg, preferably within a range of from about 0.5 mg to about 75 mg, more preferably from about 2 mg to about 50 mg, and most preferably from about 5 mg to about 25 mg.
9. The dosage form of item 3 or 4, wherein said amount of crystalline trans-(±)-Δ⁹-tetrahydrocannabinol is about 80 mg, about 40 mg, about 20 mg, about 10 mg or about 5 mg.
10. The composition or dosage form of any on of items 1-4, wherein the crystalline trans-(±)-Δ⁹-tetrahydrocannabinol is prepared by a process comprising:
   I) allowing trans-(-)-Δ⁹-tetrahydrocannabinol and
      trans-(+)-Δ⁹-tetrahydrocannabinol to crystallize from a first composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the first composition is obtained by:
      (a) forming a biphasic composition comprising (i) a first organic phase, and (ii) an alcoholic-caustic phase containing the trans-(-)-Δ⁹-tetrahydrocannabinol and the trans-(+)-Δ⁹-tetrahydrocannabinol;
      (b) separating the trans-(-)-Δ⁹-tetrahydrocannabinol and the trans-(+)-Δ⁹-tetrahydrocannabinol from the alcoholic-caustic phase; and
      (c) contacting the trans-(-)-Δ⁹-tetrahydrocannabinol and the trans-(+)-Δ⁹-tetrahydrocannabinol from step (b), with a non-polar organic solvent to form the first composition; or
   II) allowing trans-(-)-Δ⁹-tetrahydrocannabinol and trans-(+)-Δ⁹-tetrahydrocannabinol to crystallize from a first composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the first composition is obtained by:
      (a) forming a biphasic composition comprising (i) a first organic phase, and (ii) an alcoholic-caustic phase containing trans-(-)-Δ⁹-tetrahydrocannabinol;
      (b) separating the trans-(-)-Δ⁹-tetrahydrocannabinol from the alcoholic-caustic phase; and
      (c) contacting the trans-(-)-Δ⁹-tetrahydrocannabinol from step (b) with trans-(+)-Δ⁹-tetrahydrocannabinol and a non-polar organic solvent to form the first composition; or
   III) allowing trans-(-)-Δ⁹-tetrahydrocannabinol and trans-(+)-Δ⁹-tetrahydrocannabinol to crystallize from a first composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the first composition is obtained by:
      (a) forming a biphasic composition comprising (i) a first organic phase, and (ii) an alcoholic-caustic phase containing trans-(+)-Δ⁹-tetrahydrocannabinol;
      (b) separating the trans-(+)-Δ⁹-tetrahydrocannabinol from the alcoholic-caustic phase; and
      (c) contacting the trans-(+)-Δ⁹-tetrahydrocannabinol from step (b) with trans-(-)-Δ⁹-tetrahydrocannabinol and a non-polar organic solvent to form the first composition; or
   IV) allowing trans-(-)-Δ⁹-tetrahydrocannabinol and trans-(+)-Δ⁹-tetrahydrocannabinol to crystallize from a first organic composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the first organic composition is obtained by:
      (a) forming a first biphasic composition comprising (i) a first organic phase, and (ii) an alcoholic-caustic phase containing the trans-(-)-Δ⁹-tetrahydrocannabinol and the trans-(+)-Δ⁹-tetrahydrocannabinol;
      (b) separating the alcoholic-caustic phase from the first organic phase;
      (c) contacting the separated alcoholic-caustic phase with acid to provide an acid-treated alcoholic phase containing trans-(-)-Δ⁹-tetrahydrocannabinol and trans-(+)-Δ⁹-tetrahydrocannabinol;
      (d) forming a second biphasic composition comprising (i) the acid treated alcoholic phase of step (c) and (ii) a second organic phase;
      (e) separating the second organic phase of step (d) containing trans-(-)-Δ⁹-tetrahydrocannabinol and trans-(+)-Δ⁹-tetrahydrocannabinol; and
      (f) contacting the separated second organic phase of step (e) with a non-polar organic solvent to form the first organic composition; or
   V) allowing trans-(-)-Δ⁹-tetrahydrocannabinol and trans-(+)-Δ⁹-tetrahydrocannabinol to crystallize from a second organic composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the second organic composition is obtained by:
      (a) forming a first biphasic composition comprising (i) a first organic phase, and (ii) an alcoholic-caustic phase containing the trans-(-)-Δ⁹-tetrahydrocannabinol;
      (b) separating the alcoholic-caustic phase from the first organic phase;
      (c) contacting the separated alcoholic-caustic phase with acid to provide an acid-treated alcoholic phase containing trans-(-)-Δ⁹-tetrahydrocannabinol;
      (d) forming a second biphasic composition comprising the acid treated alcoholic phase of step (c) and a second organic phase;
      (e) separating the second organic phase of step (d) containing trans-(-)-Δ⁹-tetrahydrocannabinol; and
      (f) contacting the separated second organic phase of step (e) with trans-(+)-Δ⁹-tetrahydrocannabinol and a non-polar organic solvent to form the second organic composition; or
   VI) allowing trans-(-)-Δ⁹-tetrahydrocannabinol and trans-(+)-Δ⁹-tetrahydrocannabinol to crystallize from a second organic composition comprising trans-(-)-Δ⁹-tetrahydrocannabinol, trans-(+)-Δ⁹-tetrahydrocannabinol, and a non-polar organic solvent to provide crystalline trans-(±)-Δ⁹-tetrahydrocannabinol, wherein the second organic composition is obtained by:
      (a) forming a first biphasic composition comprising (i) a first organic phase, and (ii) an alcoholic-caustic phase containing the trans-(+)-Δ⁹-tetrahydrocannabinol;
      (b) separating the alcoholic-caustic phase from the first organic phase;
      (c) contacting the separated alcoholic-caustic phase with acid to provide an acid-treated alcoholic phase containing trans-(+)-Δ⁹-tetrahydrocannabinol;
      (d) forming a second biphasic composition comprising the acid treated alcoholic phase of step (c) and a second organic phase;
      (e) separating the second organic phase of step (d) containing trans-(+)-Δ⁹-tetrahydrocannabinol; and
      (f) contacting the separated second organic phase of step (e) with trans-(-)-Δ⁹-tetrahydrocannabinol and a non-polar organic solvent to form the second organic composition.
11. The dosage form of item 3 or 4, wherein the dosage form is a unit dosage form.
12. The dosage form of item 11, which is adapted for oral administration, parenteral administration, transmucosal administration, transdermal administration, or administration by inhalation.
13. A method for preparing a cannabinoid composition, the method comprising admixing crystalline trans-(±)-Δ⁹-tetrahydrocannabinol and a pharmaceutically-acceptable carrier.
14. A method for preparing a cannabinoid dosage form, the method comprising admixing an effective amount of crystalline trans-(±)-Δ⁹-tetrahydrocannabinol and a pharmaceutically-acceptable carrier.
15. An oral cannabinoid dosage form comprising a first composition and a second composition, wherein the first composition comprises a therapeutically-effective amount of crystalline trans-(±)-Δ⁹-tetrahydrocannabinol, and the second composition comprises an effective amount of an agent that is adverse to the pharmacological activity of the crystalline trans-(±)-Δ⁹-tetrahydrocannabinol, preferably said agent is selected from the group consisting of SR 141716 A, SR 144528, and combinations thereof.
16. The oral cannabinoid dosage form of item 15, wherein the second composition is coated with an inner acid-soluble layer and an outer base-soluble layer.
17. The oral cannabinoid dosage form of item 15, wherein said crystalline trans-(±)-Δ⁹-tetrahydrocannabinol comprises at least 95% preferably at least 98%, more preferably at least 99%, even more preferably at least 99.5% and most preferably at least 99.9% by weight of the total amount of cannabinoids in said first composition.
18. The oral cannabinoid dosage form of item 15, wherein the first composition and the second composition are each independently in a form selected from the group consisting of powders, granules, microparticles, multiparticulates, nanoparticles, beads, and mixtures thereof, contained within a capsule; or selected from the group consisting of powders, granules, microparticles, multiparticulates, nanoparticles, and mixtures thereof, dispersed in a pharmaceutically-acceptable matrix.
19. The oral cannabinoid dosage form of item 15, in the form of a two-layer tablet having a first layer comprising the first composition and a second layer comprising the second composition, wherein the tablet is coated with a coating that dissolves in the stomach.
20. An oral, controlled-release, cannabinoid dosage form suitable for 8-hour, 12-hour, or 24 hour dosing in a human patient comprising a pharmaceutically-acceptable matrix comprising a therapeutically-effective amount of crystalline trans-(±)-Δ⁹-tetrahydrocannabinol and a pharmaceutically-acceptable controlled-release material.
21. The oral, controlled-release, cannabinoid dosage form of item 20, wherein the controlled-release material is selected from the group consisting of hydrophobic polymers, hydrophilic polymers, gums, protein-derived materials, waxes, shellacs, oils, and mixtures thereof.
22. The oral, controlled-release, cannabinoid dosage form of item 20, said dosage form after administration to a human patient, providing a C₂₄/C*ₘₐₓ* ratio of from about 0.55 to about 1, preferably from about 0.55 to about 0.85; more preferably from about 0.55 to 0.75; most preferably from about 0.60 to about 0.70 and said dosage form providing a therapeutic effect for at least about 24 hours, wherein C*ₘₐₓ* is preferably a sub-psychotropic-threshold concentration.
23. The oral, controlled-release, cannabinoid dosage form of item 20, wherein said crystalline trans-(±)-Δ⁹-tetrahydrocannabinol comprises at least 95%, preferably at least 98%, more preferably at least 99%, even more preferably at least 99.5% and most preferably at least 99.9% by weight of the total amount of cannabinoids in said dosage form.
24. The dosage form of item 20, wherein said matrix comprises a plurality of multiparticulate matrices, wherein said multiparticulate matrices are preferably compressed into a tablet, disposed in a pharmaceutically-acceptable capsule or disposed in a pharmaceutically-acceptable solution, gel, suspension, or emulsion.
25. A process for the preparation of a solid, oral, controlled-release, cannabinoid dosage form comprising the step of incorporating a therapeutically-effective amount of crystalline trans-(±)-Δ⁹-tetrahydrocannabinol into a pharmaceutically-acceptable controlled-release material.
26. The process of item 25, wherein the controlled-release material comprises a matrix selected from the group consisting of hydrophobic polymers, hydrophilic polymers, gums, protein-derived materials, waxes, shellacs, oils, and mixtures thereof, forming a controlled-release matrix formulation, said dosage form after administration to a human patient, providing a C₂₄/C*ₘₐₓ* ratio of from about 0.55 to about 1, preferably from about 0.55 to about 0.85; more preferably from about 0.55 to about 0.75 most preferably from about 0.60 to about 0.70; and a therapeutic effect for at least about 24 hours.
27. The process of item 25, wherein said crystalline trans-(±)-Δ⁹-tetrahydrocannabinol comprises at least 95%, preferably at least 98%, more preferably at least 99%, even more preferably at least 99.5% and most preferably at least 99.9% by weight of the total amount of cannabinoids in said dosage form.
28. A cannabinoid composition for transmucosal or transdermal administration of trans-(±)-Δ⁹-tetrahydrocannabinol, wherein said composition is formulated with crystalline trans-(±)-Δ⁹-tetrahydrocannabinol and a pharmaceutically-acceptable carrier adapted for transmucosal or transdermal administration.
29. The cannabinoid composition of item 28, wherein said crystalline trans-(±)-Δ⁹-tetrahydrocannabinol comprises at least 95%, preferably at least 98%, more preferably at least 99%, even more preferably at least 99.5% and most preferably at least 99.9% by weight of the total amount of cannabinoids in said composition.
30. A use of an effective amount of the composition or the dosage form according to any one of items 1-4 in the preparation of a medicament for the treatment of pain, emesis, loss of appetite or weight loss for the treatment of a mammal in need.
31. A use of a therapeutically effective amount of crystalline trans-(±)-Δ⁹-tetrahydrocannabinol, optionally admixed with a pharmaceutically-acceptable carrier, in the production of a medicament, comprising the deposition of trans-(±)-Δ⁹-tetrahydrocannabinol into the lungs of a mammal in need thereof.
32. The use of item 31, wherein said crystalline trans-(±)-Δ⁹-tetrahydrocannabinol comprises at least 95%, preferably at least 98%, more preferably at least 99%, even more preferably at least 99.5% and most preferably at least 99.9% by weight of the total amount of cannabinoids in said composition.
33. The use of item 31, wherein said crystalline trans-(±)-Δ⁹-tetrahydrocannabinol, optionally admixed with a pharmaceutically-acceptable carrier, is in a form selected from the group consisting of powders, granules, microparticles, nanoparticles and mixtures thereof.
34. The use of item 31, wherein said crystalline trans-(±)-Δ⁹-tetrahydrocannabinol is delivered to the lungs of said mammal from a mechanical device suitable for pulmonary administration and capable of depositing said crystalline trans-(±)-Δ⁹-tetrahydrocannabinol of a mammal, preferably is selected from the group consisting of powder inhaler, a unit dose inhaler, a metered dose inhaler, a pump spray and a nebulizer.
35. A use of a composition comprising trans-(±)-Δ⁹-tetrahydrocannabinol, in the preparation of a medicament comprising admixing an effective amount of crystalline trans-(±)-Δ⁹-tetrahydrocannabinol and a pharmaceutically-acceptable carrier to provide a composition and administering the composition to the patient.
36. The use according to item 35 wherein the composition is in the form of a solution, emulsion, gel or suspension or in the form of a dry solid or dry powder.
37. The use according to item 35, wherein the pharmaceutically-acceptable carrier is a solvent and the composition is a solution.
38. The use according to item 35, wherein the admixing and the administering are carried out by the patient.
39. The use according to item 35, wherein the administering is carried out immediately after admixing the crystalline trans-(±)-Δ⁹-tetrahydrocannabinol and a pharmaceutically-acceptable carrier to provide the composition.
40. The composition according of item 1 or item 2, wherein the crystalline trans-(±)-Δ⁹-THC is characterized by powder X-ray diffraction data that is partially or fully equivalent to that of Table 1.
41. Composition or dosage form according to any of items 1 to 12, 15 to 24, 28 to 29, and 40 for use in the treatment of pain, emesis, loss of appetite or weight loss.
42. Composition or dosage form according to item 41 wherein the crystalline trans-(±)-Δ⁹-THC, optionally admixed with a pharmaceutically-acceptable carrier, comprises the deposition of trans-(±)-Δ⁹-THC into the lungs of a mammal in need thereof.
43. Composition or dosage form according to item 42, wherein said crystalline trans-(±)-Δ⁹-THC comprises at least 95%, preferably at least 98%, more preferably at least 99%, even more preferably at least 99,5% and most preferably at least 99,9% by weight of the total amount of cannabinoids in said composition or dosage form.
44. Composition or dosage form according to item 42, wherein said crystalline trans--(±)-Δ⁹-THC, optionally admixed with a pharmaceutically-acceptable carrier, is in a form selected from the group consisting of powders, granules, microparticles, nanoparticles and mixtures thereof.
45. Composition or dosage form according to item 42, wherein said crystalline trans-(±)-Δ⁹-THC is delivered to the lungs of said mammal from a mechanical device suitable for pulmonary administration and capable of depositing said crystalline trans-(±)-Δ⁹-THC of a mammal, preferably selected from the group consisting of powder inhaler, a unit dose inhaler, a metered dose inhaler, a pump spray and a nebulizer.
46. Composition comprising trans-(±)-Δ⁹-THC for use in the treatment of pain, emesis, loss of appetite or weight loss comprising admixing an effective amount of crystalline trans-(±)-Δ⁹-THC and a pharmaceutically-acceptble carrier to provide a composition and administering the composition to the patient.
47. Composition according to item 46, wherein the composition is in the form of a solution, emulsion, gel or suspension or in the form of a dry solid or dry powder.
48. The use according to item 46, wherein the pharmaceutically-acceptable carrier is a solvent and the composition is a solution.
49. The composition according to item 46, wherein the admixing and administering are carried out by the patient.
50. The compound according to item 46, wherein the administering is carried out immediately after admixing the crystalline trans-(±)-Δ⁹-THC and a pharmaceutically-acceptable carrier to provide the composition.

## Claims

1. A composition comprising crystalline trans-(±)-Δ⁹-tetrahydrocannabinol and a pharmaceutically-acceptable carrier.

2. A composition comprising trans-(±)-Δ⁹-tetrahydrocannabinol, wherein said composition is formulated with crystalline trans-(±)-Δ⁹-tetrahydrocannabinol and a pharmaceutically-acceptable carrier.

3. A dosage form comprising a therapeutically effective amount of crystalline trans-(±)-Δ⁹-tetrahydrocannabinol and a pharmaceutically-acceptable carrier.

4. A dosage form comprising a therapeutically effective amount of trans-(±)-Δ⁹-tetrahydrocannabinol, wherein said dosage form is formulated with crystalline trans-(±)-Δ⁹-tetrahydrocannabinol.

5. The dosage form of claim 3 or 4, wherein the dosage form is a unit dosage form.

6. The dosage form of claim 5, which is adapted for oral administration, parenteral administration, transmucosal administration, transdermal administration, or administration by inhalation.

7. A method for preparing a cannabinoid composition, the method comprising admixing crystalline trans-(±)-Δ⁹-tetrahydrocannabinol and a pharmaceutically-acceptable carrier.

8. A method for preparing a cannabinoid dosage form, the method comprising admixing an effective amount of crystalline trans-(±)-Δ⁹-tetrahydrocannabinol and a pharmaceutically-acceptable carrier.

9. An oral cannabinoid dosage form comprising a first composition and a second composition, wherein the first composition comprises a therapeutically-effective amount of crystalline trans-(±)-Δ⁹-tetrahydrocannabinol, and the second composition comprises an effective amount of an agent that is adverse to the pharmacological activity of the crystalline trans-(±)-Δ⁹-tetrahydrocannabinol, preferably said agent is selected from the group consisting of SR 141716 A, SR 144528, and combinations thereof.

10. The oral cannabinoid dosage form of claim 9, in the form of a two-layer tablet having a first layer comprising the first composition and a second layer comprising the second composition, wherein the tablet is coated with a coating that dissolves in the stomach.

11. An oral, controlled-release, cannabinoid dosage form suitable for 8-hour, 12-hour, or 24 hour dosing in a human patient comprising a pharmaceutically-acceptable matrix comprising a therapeutically-effective amount of crystalline trans-(±)-Δ⁹-tetrahydrocannabinol and a pharmaceutically-acceptable controlled-release material.

12. A cannabinoid composition for transmucosal or transdermal administration of trans-(±)-Δ⁹-tetrahydrocannabinol, wherein said composition is formulated with crystalline trans-(±)-Δ⁹-tetrahydrocannabinol and a pharmaceutically-acceptable carrier adapted for transmucosal or transdermal administration.

13. A use of an effective amount of the composition or the dosage form according to any one of claims 1-4 in the preparation of a medicament for the treatment of pain, emesis, loss of appetite or weight loss for the treatment of a mammal in need.

14. A use of a therapeutically effective amount of crystalline trans-(±)-Δ⁹-tetrahydrocannabinol, optionally admixed with a pharmaceutically-acceptable carrier, in the production of a medicament, comprising the deposition of trans-(±)-Δ⁹-tetrahydrocannabinol into the lungs of a mammal in need thereof.

15. Composition or dosage form according to any of claims 1 to 6, 9 to 12, for use in the treatment of pain, emesis, loss of appetite or weight loss.

16. Composition comprising trans-(±)-Δ⁹-THC for use in the treatment of pain, emesis, loss of appetite or weight loss comprising admixing an effective amount of crystalline trans-(±)-Δ⁹-THC and a pharmaceutically-acceptble carrier to provide a composition and administering the composition to the patient.
